# EUROPÄISCHE PATENTSCHRIFT

(11) **EP 3 397 637 B1**
(45) Veröffentlichungstag und Bekanntmachung des Hinweises auf die Patenterteilung: **11.08.2021**
(21) Anmeldenummer: 16829099.7
(22) Anmeldetag: 30.12.2016
(51) Int. Cl.: C07D 407/10, C07D 409/14, C07D 493/04, C07D 495/04, H01L 51/42

(54) **VERBINDUNG FÜR FOTOAKTIVE ORGANISCHE ELEKTRONISCHE BAUELEMENTE UND FOTOAKTIVES ORGANISCHES ELEKTRONISCHES BAUELEMENT ENTHALTEND DIE VERBINDUNG**
COMPOUND FOR PHOTOACTIVE ORGANIC ELECTRONIC ELEMENT, AND PHOTOACTIVE ORGANIC ELECTRONIC ELEMENT COMPRISING THE COMPOUND
LIAISON POUR ELEMENTS DE CONSTRUCTION ELECTRONIQUES ORGANIQUES PHOTO-ACTIFS ET ELEMENT DE CONSTRUCTION ELECTRONIQUE ORGANIQUE PHOTO-ACTIF COMPRENANT LA LIAISON

(30) Priorität: 30.12.2015 DE 102015123005; 26.07.2016 EP 16181348
(43) Veröffentlichungstag der Anmeldung: 07.11.2018
(73) Patentinhaber: Heliatek GmbH, 01139 Dresden (DE)
(72) Erfinder: HILDEBRANDT, Dirk, 89077 Ulm (DE); GERDES, Olga, 89077 Ulm (DE); FITZNER, Roland, 89077 Ulm (DE); D'SOUZA, Daniel, 89077 Ulm (DE); MATTERSTEIG, Gunter, 89077 Ulm (DE); WEISS, Andre, 01139 Dresden (DE)
(86) Internationale Anmeldenummer: PCT/EP2016/082900
(87) Internationale Veröffentlichungsnummer: WO 2017/114937

(56) Entgegenhaltungen:
- WO-A1-2015/044377
- ROLAND FITZNER ET AL: "A-D-A-Type Oligothiophenes for Small Molecule Organic Solar Cells: Extending the [pi]-System by Introduction of Ring-Locked Double Bonds", ADVANCED FUNCTIONAL MATERIALS, WILEY-VCH, WEINHEIM, Bd. 25, Nr. 12, 25. März 2015 (2015-03-25) , Seiten 1845-1856, XP001595297, DOI: 10.1002/ADFM.201404210
- AMARESH MISHRA ET AL: "A-D-A-type S , N -Heteropentacenes: Next-Generation Molecular Donor Materials for Efficient Vacuum-Processed Organic Solar Cells", ADVANCED MATERIALS, Bd. 26, Nr. 42, 1. November 2014 (2014-11-01), Seiten 7217-7223, XP055310415, DE ISSN: 0935-9648, DOI: 10.1002/adma.201402448 in der Anmeldung erwähnt
- ROLAND FITZNER ET AL: "Dicyanovinyl?Substituted Oligothiophenes: Structure-Property Relationships and Application in Vacuum-Processed Small Molecule Organic Solar Cells", ADVANCED FUNCTIONAL MATERIALS, WILEY - V C H VERLAG GMBH & CO. KGAA, DE, Bd. 21, Nr. 5, 8. März 2011 (2011-03-08), Seiten 897-910, XP001560466, ISSN: 1616-301X, DOI: 10.1002/ADFM.201001639 [gefunden am 2011-01-27] in der Anmeldung erwähnt
- YASSIN A ET AL: "Evaluation of bis-dicyanovinyl short-chain conjugated systems as donor materials for organic solar cells", SOLAR ENERGY MATERIALS AND SOLAR CELLS, ELSEVIER SCIENCE PUBLISHERS, AMSTERDAM, NL, Bd. 95, Nr. 2, 1. Februar 2011 (2011-02-01), Seiten 462-468, XP027576773, ISSN: 0927-0248 [gefunden am 2010-12-27]

## Beschreibung

### Gebiet der Erfindung

Die Erfindung betrifft eine Verbindung, die sich beispielsweise zur Verwendung in fotoaktiven organischen elektronischen Bauelementen eignet, sowie ein fotoaktives organisches elektronisches Bauelement, das die Verbindung enthält. Zudem betrifft die Erfindung ein Verfahren zur Herstellung der genannten Verbindung.

### Stand der Technik

Fotoaktive organische elektronische Bauelemente ermöglichen es elektromagnetische Strahlung, etwa im Wellenlängenbereich des sichtbaren Lichts, unter Ausnutzung des photoelektrischen Effekts in elektrischen Strom umzuwandeln. Für die Umwandlung werden organische Halbleitermaterialien benötigt, die hinreichend gute Absorptionseigenschaften zeigen.

Fitzner, R. u.a. zeigt A-D-A-Absorbermaterialien mit DCC-Akzeptorblock (A-D-A-type: Oligithiophenes for Small Molecules Organic Solar Cells: Extending the π-System by Introduction of Ring-Locked Double Bonds, Advanced Functional Materials, Wiley-VCH, Weinheim, Bd. 25, Nr. 12, 25. März 2015, Seite 1845-1856).

WO2015044377A1 offenbart A-D-A-Moleküle mit anneliertem mittleren Donorblock und deren Verwendung in optoelektronischen Bauelementen.

Mishra, A. u.a. beschreibt A-D-A-Moleküle mit DCV-Akzeptorblock und mit Schwefel und Stickstoff substituiertem Donorblock und die Verwendung dieser Materialien in optoelektronischen Bauelementen (A-D-A-types S, N-heteropentacenes: Next Generation Molecular donor Materials for efficient Vacuum-processed Organic solar cells, Advanced Materials, Bd. 26, Nr. 42, 1. November 2014, Seiten 7217-7223).

Fitzner, R. u.a. zeigt A-D-A-Moleküle in Form von DCVnT (Dicyanovinyl-Substituted Oligothiophenes: Structure-Property Relationships and Application in Vacuum-Processed Small-Molecule Organic Solar cells, Advanced Functional Materials, Wiley-VCH, Weinheim, Bd. 21, Nr. 5, 8. März 2011, Seite 897-910).

Yassin, E. u.a. zeigt die Verwendung von drei Verbindungen mit einer Dicyanovinyleinheit in einer Solarzelle (Evaluation of bisdicyanovinyl short-chain conjugated systems as donor materials for organic solar cells, Solar Energy Materials and Solar Cells, Elsevier Science Publishers, Amsterdam, NL, Bd. 95, Nr. 2, 1. Februar 2011, Seiten 462-468).

### Technische Aufgabe

Es ist folglich eine Aufgabe der vorliegenden Erfindung, organische Verbindungen anzugeben, die gute Absorptionseigenschaften zeigen und sich beispielsweise für den Einsatz in fotoaktiven organischen elektronischen Bauelementen eignen.

Die vorliegende Erfindung betrifft neben der erfindungsgemäßen Verbindung zudem die Verwendung der erfindungsgemäßen Verbindung in einem fotoaktiven organischen elektronischen Bauelement.

Die vorliegende Erfindung betrifft außerdem ein fotoaktives organisches elektronisches Bauelement, umfassend die erfindungsgemäße Verbindung.

Die Erfindung betrifft zudem eine Verbindung - als Zwischenprodukt - für die Herstellung der erfindungsgemäßen Verbindung.

### Technische Lösung

Die Anmeldung offenbart Verbindungen der allgemeinen Formel

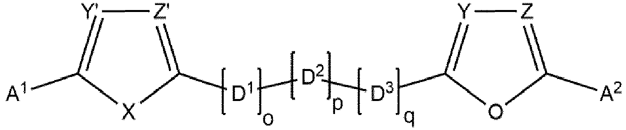

Y, Y', Z und Z' sind dabei jeweils unabhängig voneinander ausgewählt aus: N oder CR^{a}.

Unabhängig voneinander ausgewählt bedeutet hierbei auch, dass R^{a} für Y, Y', Z und Z' jeweils verschieden sein kann. Es ist aber beispielsweise auch möglich, dass R^{a} für zwei, mehrere oder alle von Y, Y', Z und Z' gleich ist.

In der erfindungsgemäßen Verbindung ist X ausgewählt aus: O, S, Se, Si(R^{b}R^{c}), P(R^{b}), P(O)R^{b}.

Die Reste R^{a} bis R^{c} sind jeweils unabhängig voneinander aus der Gruppe der folgenden Reste ausgewählt:
- H,
- Halogen,
- CN,
- NR^{d}R^{e}, wobei R^{d} und R^{e} jeweils unabhängig voneinander ausgewählt sind aus der Gruppe der Reste: H sowie zyklisches oder offenkettiges C₁-C₂₀-Alkyl, wobei einzelne C-Atome durch Heteroatome ersetzt sein können und wobei Wasserstoffatome des Alkylrests substituiert sein können,
- zyklisches oder offenkettiges C₁-C₂₀-Alkyl, wobei einzelne C-Atome durch Heteroatome ersetzt sein können,
- zyklisches oder offenkettiges C₁-C₂₀-O-Alkyl,
- zyklisches oder offenkettiges C₁-C₂₀-S-Alkyl
- zyklisches oder offenkettiges C₂-C₂₀-Alkenyl,
- zyklisches oder offenkettiges C₂-C₂₀-O-Alkenyl,
- zyklisches oder offenkettiges C₂-C₂₀-S-Alkenyl,
- zyklisches oder offenkettiges C₂-C₂₀-Alkinyl,
- Aryl
- Heteroaryl,
wobei Wasserstoffatome der Reste Alkyl, O-Alkyl, S-Alkyl, Alkenyl, O-Alkenyl, S-Alkenyl, Alkinyl, Aryl und Heteroaryl jeweils unabhängig voneinander substituiert sein können.

Die erste Aufgabe wird durch eine Verbindung gemäß Anspruch 1 gelöst.

Demnach betrifft die vorliegende Erfindung eine Verbindung der allgemeinen Formel (I): 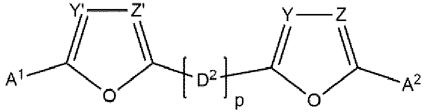
mit p ausgewählt aus: 1, 2, 3, 4 und 5,
mit Y, Y', Z und Z' jeweils unabhängig voneinander ausgewählt aus: N, CH, C-F, C-CH₃, C-CF₃, C-C₂H₅, C-C₃H₈, C-OCH₃, C-OC₂H₅, C-SCH₃, C-SC₂H₅; und
wobei die konjugierten Blöcke D² jeweils unabhängig voneinander ausgewählt sind aus der Gruppe der folgenden Struktureinheiten:

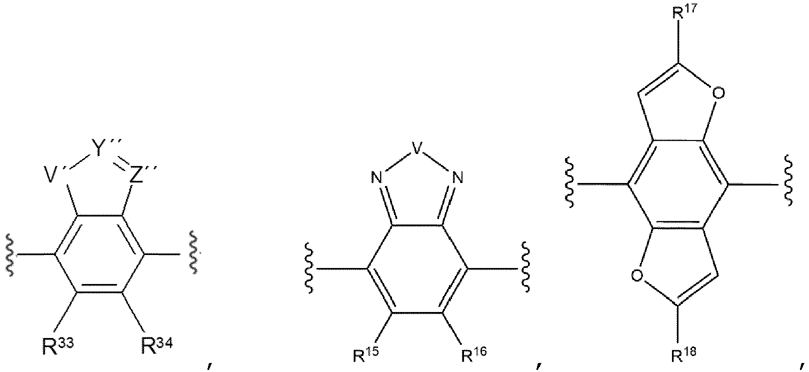

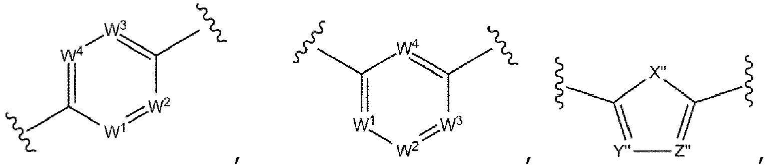

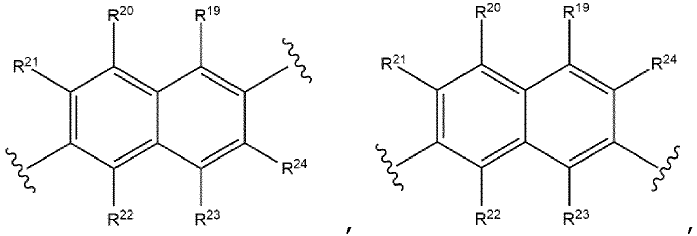

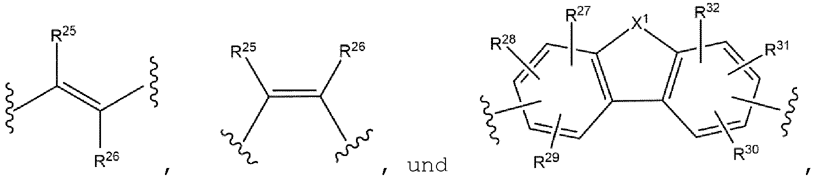

mit Y" ausgewählt aus: N oder CR^{g};
mit Z" ausgewählt aus: N oder CR^{h};
mit X" ausgewählt aus: O, S, Se, Si (RⁱR^{j}) , P(Rⁱ), P(O)Rⁱ;
mit V ausgewählt aus: O, S, Se oder NR^{k};
mit V' ausgewählt aus: O, S, Se oder NR^{k};
mit X¹ ausgewählt aus: NR¹, CR¹R^{m};
mit W¹ bis W⁴ unabhängig voneinander ausgewählt aus: N, CRⁿ;
wobei die Reste R^{g} bis Rⁿ und die Reste R¹⁵ bis R³⁴ jeweils unabhängig voneinander aus der Gruppe der Reste ausgewählt sind: H, Halogen, CN, NR^{d}R^{e}, wobei R^{d} und R^{e} jeweils unabhängig voneinander ausgewählt sind aus der Gruppe der Reste: H, sowie zyklisches oder offenkettiges C₁-C₂₀-Alkyl, wobei einzelne C-Atome durch Heteroatome ersetzt sein können und wobei Wasserstoffatome des Alkylrests substituiert sein können, zyklisches oder offenkettiges C₁-C₂₀-Alkyl, wobei einzelne C-Atome durch Heteroatome ersetzt sein können, zyklisches oder offenkettiges C₁-C₂₀-O-Alkyl, zyklisches oder offenkettiges C₁-C₂₀-S-Alkyl, zyklisches oder offenkettiges C₂-C₂₀-Alkenyl, zyklisches oder offenkettiges C₂-C₂₀-O-Alkenyl, zyklisches oder offenkettiges C₂-C₂₀-S-Alkenyl, zyklisches oder offenkettiges C₂-C₂₀-Alkinyl, Aryl, Heteroaryl,
wobei Wasserstoffatome der Reste Alkyl, O-Alkyl, S-Alkyl, Alkenyl, O-Alkenyl, S-Alkenyl, Alkinyl, Aryl und Heteroaryl jeweils unabhängig voneinander substituiert sein können; und wobei die Reste R^{g} und R^{h} miteinander in Form einer Ringstruktur verbunden sein können und wobei an die Ringstruktur ein Aryl-Rest kondensiert sein kann, wobei der gegebenenfalls an die Ringstruktur kondensierte Aryl-Rest substituiert sein kann, wobei A¹ ausgewählt ist aus der Gruppe der folgenden Reste: 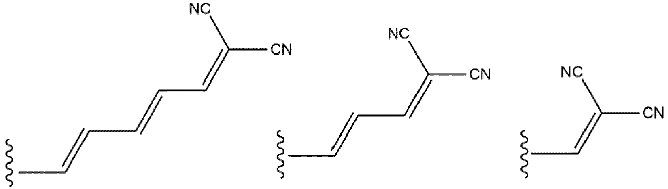 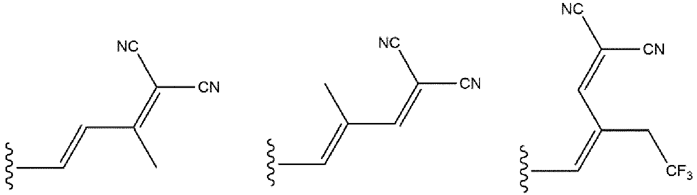 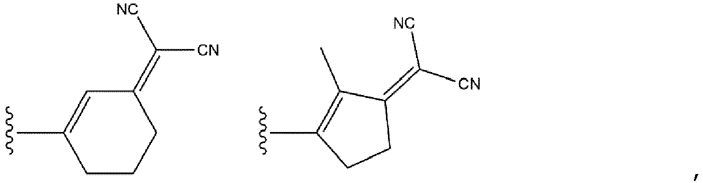 und
wobei A² ausgewählt ist aus der Gruppe der folgenden Reste:

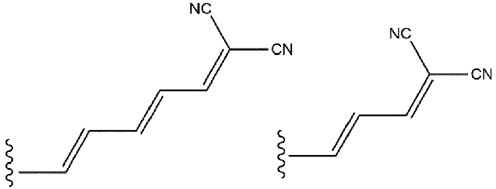

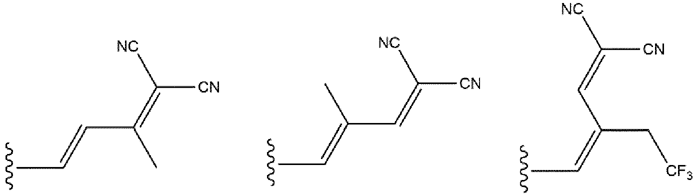

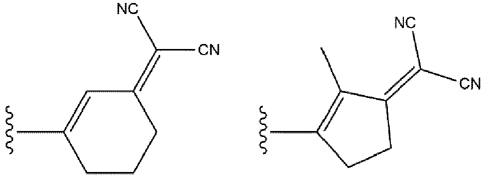

Unter einer Substitution ist hierbei und im Folgenden der Ersatz eines oder mehrerer Wasserstoffatome durch andere Reste zu verstehen. Beispielsweise können Wasserstoffatome durch Halogenatome wie etwa Fluoratome ersetzt sein. Beispielsweise können Wasserstoffatome aber auch durch zyklische oder offenkettige Alkylreste, wie etwa C₁-C₁₀-, bevorzugt C₁-C₆-Alkylreste, insbesondere Methyl, Ethyl, Propyl, Butyl, Pentyl, Hexyl und dergleichen substituiert sein, die jeweils linear oder verzweigt sein können und wobei die Alkylreste zumindest teilweise halogeniert, z.B. fluoriert, vorzugsweise perfluoriert, sein können.

Unter Halogen ist im Zusammenhang mit der erfindungsgemäßen Verbindung hier und im Folgenden jeweils F, Cl, Br und I zu verstehen, bevorzugt jeweils F.

-CN steht für eine Nitril-Gruppe.

NR^{d}R^{e} steht für eine Amin-Funktion, wobei es sich um ein primäres, sekundäres oder tertiäres Amin handeln kann. R^{d} und R^{e} schließen lineare wie verzweigte Reste ein. Für R^{d} und R^{e} kann es sich auch um C₁-C₂₀-Alkyl-Reste handeln, bei welchen Wasserstoffatome subsituiert sind. Zum Beispiel können Wasserstoffatome mit Fluoratomen ersetzt sein. Es kann sich bei R^{d} und R^{e} zudem auch um C₁-C₂₀-Alkyl-Reste handeln, wobei einzelne C-Atome durch Heteroatome ersetzt sein können. Damit ist insbesondere gemeint, dass nicht endständige, nicht benachbarte C-Atome durch Atome wie O, S, Se oder eine Gruppe NR ersetzt sein können, wobei R dabei H oder ein Alkyl ist. Es ist aber bevorzugt, dass keines der C-Atome des C₁-C₂₀-Alkyl Rests durch Heteroatome ersetzt ist. Bevorzugt sind R^{d} und R^{e} ausgewählt aus H und C₁-C₁₀-Alkyl-Resten, insbesondere aus H, Methyl, Ethyl, Propyl, Butyl und Pentyl.

Der für die Reste R^{a} bis R^{c} gebrauchte Term zyklisches oder offenkettiges C₁-C₂₀-Alkyl, schließt jeweils lineare und verzweigte C₁-C₂₀-Alkyl-Reste ein. Es kann sich auch um C₁-C₂₀-Alkyl-Reste handeln, wobei einzelne C-Atome durch Heteroatome ersetzt sein können. Damit ist insbesondere gemeint, dass nicht endständige, nicht benachbarte C-Atome durch Atome wie O, S, Se oder eine Gruppe NR ersetzt sein können, wobei R dabei H oder ein Alkyl ist. Es ist aber bevorzugt, dass keines der C-Atome des C₁-C₂₀-Alkyl-Rests durch Heteroatome ersetzt ist. Beispielsweise kann es sich um zyklische oder offenkettige C₁-C₁₀-Alkyl-Reste handeln. Insbesondere kann es sich um offenkettige C₁-C₅-Alkylreste handeln.

Auch für die Reste zyklisches oder offenkettiges C₁-C₂₀-O-Alkyl, zyklisches oder offenkettiges C₁-C₂₀-S-Alkyl, zyklisches oder offenkettiges C₂-C₂₀-Alkenyl, zyklisches oder offenkettiges C₂-C₂₀-O-Alkenyl, zyklisches oder offenkettiges C₂-C₂₀-S-Alkenyl und zyklisches oder offenkettiges C₂-C₂₀-Alkinyl gilt jeweils, dass die Reste linear oder verzweigt sein können.

Für das zyklische oder offenkettige C₁-C₂₀-O-Alkyl und für das zyklische oder offenkettige C₂-C₂₀-O-Alkenyl ist jeweils der Sauerstoff die Anbindungsstelle an die erfindungsgemäße Verbindung.

Analog ist für das zyklische oder offenkettige C₂-C₂₀-S-Alkyl sowie für das zyklische oder offenkettige C₂-C₂₀-S-Alkenyl jeweils der Schwefel die Anbindungsstelle an die erfindungsgemäße Verbindung.

Unter Aryl ist im Falle der vorliegenden Erfindung bevorzugt ein C₅-C₂₀-Aryl zu verstehen, etwa ein C₅-C₁₂-Aryl. Unter einem Heteroaryl ist im Falle der vorliegenden Erfindung ein Aryl zu verstehen, wobei jeweils mindestens ein C-Atom des Aryls mit einem Heteroatom ersetzt ist. Bei dem Heteroatom kann es sich beispielsweise um O, S, Se, Si, B, N oder P handeln.

In der erfindungsgemäßen Verbindung handelt es sich bei A¹ um einen elektronenziehenden Rest, der zumindest eine C=C-Doppelbindung aufweist. Außerdem handelt es sich bei A² um einen elektronenziehenden Rest, der zumindest zwei konjugierte C=C-Doppelbindungen aufweist. Beispielsweise weist A¹ eine, zwei oder drei C=C-Doppelbindungen auf, die miteinander konjugiert sein können. Beispielsweise weist A² zwei oder drei konjugierte C=C-Doppelbindungen auf. Für A¹ gilt hierbei, dass die zumindest eine C=C-Doppelbindung bevorzugt nicht Teil eines aromatischen Ringes ist. Für A² gilt ebenfalls, dass die zumindest zwei konjugierten C=C-Doppelbindungen bevorzugt nicht Teil eines aromatischen Ringes sind.

Die Gruppen A¹ und A² zeichnen sich also jeweils durch ihren elektronenziehenden Charakter aus und wirken insbesondere als Akzeptor-Gruppen.

In der erfindungsgemäßen Verbindung sind die konjugierten Blöcke D² jeweils unabhängig voneinander ausgewählt aus der Gruppe der folgenden Struktureinheiten:

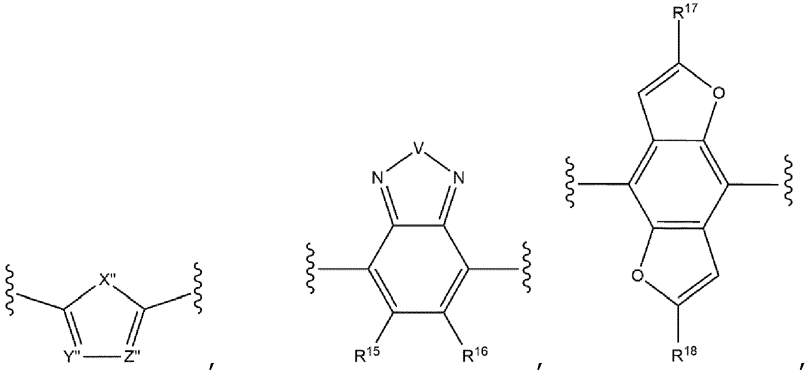

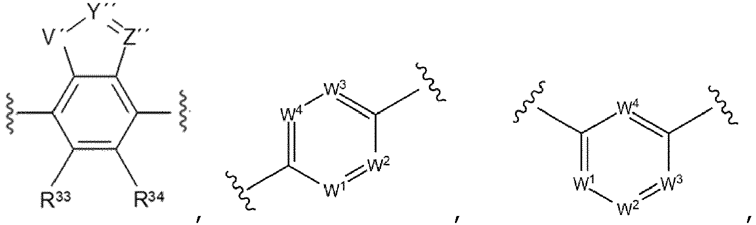

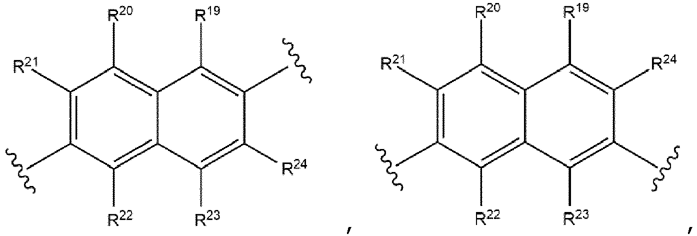

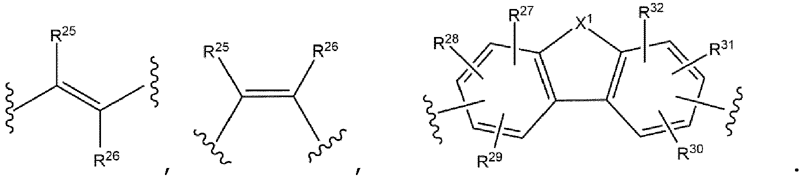

Unabhängig voneinander ausgewählt bedeutet hierbei, dass die einzelnen konjugierten Blöcke D² jeweils gleiche oder verschiedene von den angegebenen Struktureinheiten sein können. Außerdem ist es möglich, dass zwei oder mehr der konjugierten Blöcke D² zwar die gleiche Grundstruktur wie zum Beispiel 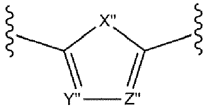 aufweisen können, wobei jedoch X", Y" und Z" für die verschiedenen konjugierten Blöcke nicht gleich sein müssen, sondern verschieden sein können. Die soeben genannten Beispiele dienen nur der Veranschaulichung und sind nicht limitierend auszulegen.

Der Koeffizient p nimmt die Werte 1, 2, 3, 4 und 5 an.

Für die genannten Struktureinheiten gilt dabei:
- Y" ist ausgewählt aus: N oder CR^{g};
- Z" ist ausgewählt aus: N oder CR^{h};
- X" ist ausgewählt aus: O, S, Se, Si(RⁱR^{j}), P(Rⁱ), P(O)Rⁱ;
- V ist ausgewählt aus: O, S, Se oder NR^{k};
- V' ist ausgewählt aus: O, S, Se oder NR^{k}; bevorzugt NR^{k},
- X1 ist ausgewählt aus: NR^{l}, CR^{l}R^{m};
- W¹ bis W⁴ sind unabhängig voneinander ausgewählt aus: N, CRⁿ;
wobei die Reste R^{g} bis Rⁿ und die Reste R¹⁵ bis R³⁴ jeweils unabhängig voneinander aus der Gruppe der Reste ausgewählt sind wie R^{a} bis R^{c}_{.} Außerdem können die Reste R^{g} und R^{h} miteinander in Form einer Ringstruktur verbunden sein. Zudem kann an die genannte Ringstruktur ein Aryl-Rest kondensiert sein. Dabei ist es möglich, dass der zuletzt genannte Aryl-Rest substituiert ist.

Bei der Ringstruktur kann es sich z.B. um einen fünf-, sechs- oder siebengliedrigen Ring handeln. Beispielsweise kann es sich um einen gesättigten Ring handeln. Beispielsweise kann der Ring Heteroatome wie etwa O oder S aufweisen. Es ist aber auch möglich, dass der Ring ein homocyclischer Ring ist. Beispielsweise kann der Ring substituiert sein. Zum Beispiel kann der Ring Fluoratome oder Alkyl-Gruppen als Substituenten aufweisen. Auch kann der Ring zumindest teilweise fluorierte Alkyl-Gruppen als Substituenten aufweisen.

Bei dem Aryl-Rest, der an die Ringstruktur kondensiert sein kann, kann es sich zum Beispiel um einen Benzol-Ring handeln, der zwei C-Atome mit der Ringstruktur gemeinsam hat.

Die konjugierten Blöcke D² bilden zusammen mit den beiden sie umgebenden Fünfringen, gemeinsam mit den beiden elektronenziehenden Gruppen A¹ und A² ein ausgeprägtes *π*-Elektronensystem.

### Vorteile der Verbindung

Ob eine organische Verbindung für die Verwendung in fotoaktiven organischen elektronischen Bauelementen geeignet ist, hängt unter anderem vom Absorptionsverhalten der Verbindung ab. Eine hohe Absorption ist wünschenswert. Insbesondere ist es von Vorteil, wenn die Verbindung in einem breiten Bereich des zur Verfügung stehenden Spektrums der elektromagnetischen Strahlung absorbiert, da somit Photonen verschiedenster Wellenlängen für die Erzeugung von elektrischem Strom genutzt werden können.

Gerade im Bereich von Wellenlängen kleiner 600 nm - insbesondere kleiner 500 nm - ist die Absorption vieler herkömmlicherweise in fotoaktiven organischen elektronischen Bauelementen eingesetzter organischer Verbindungen ungenügend. Dies führt dazu, dass die Photonen dieses Wellenlängenbereichs nicht in ausreichendem Maße genutzt werden können.

Demgegenüber haben die Erfinder der vorliegenden Erfindung festgestellt, dass die erfindungsgemäßen Verbindungen gemäß der allgemeinen Formel (I) ein erstaunlich gutes Absorptionsverhalten in einem vergleichsweise breiten Bereich des sichtbaren Lichts (ca. 400 bis 700 nm) zeigen. Insbesondere konnte eine überraschend hohe Absorption im kurzwelligen solaren Spektralbereich von ca. 400 bis 600 nm festgestellt werden. Vor allem für den Wellenlängenbereich unter 500 nm wurde ein deutlich besseres Absorptionsverhalten beobachtet, als dies bei vielen herkömmlicherweise in fotoaktiven organischen elektronischen Bauelementen eingesetzten Verbindungen der Fall ist.

Die Erfinder der vorliegenden Erfindung haben erkannt, dass Verbindungen der allgemeinen Struktur von Formel (I) Molekülorbitale aufweisen, deren energetische Lage die besonders guten Absorptionseigenschaften auch im hochenergetischen, also kurzwelligen Bereich des sichtbaren Lichts ermöglichen. Die Verbindungen sind somit hervorragend für eine Verwendung in fotoaktiven organischen elektronischen Bauelementen geeignet, beispielsweise für Bauelemente, welche Sonnenlicht in elektrischen Strom umwandeln wie etwa in Fotodetektoren, lichtempfindlichen Transistoren und vor allem in organischen Solarzellen.

Die Erfinder haben zudem herausgefunden, dass die erfindungsgemäße Verbindung mit einem Sauerstoffatom in dem Strukturelement: 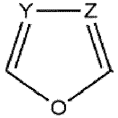 von Formel (I) in der Regel zu besseren optischen Eigenschaften führt, als dies für ähnliche Verbindungen jedoch ohne Sauerstoff der Fall ist. Es hat sich beispielsweise gezeigt, dass die Anwesenheit von O gegenüber S in diesem Strukturelement vorzuziehen ist. Beispielsweise ist Furan Thiophen vorzuziehen.

Die Erfinder haben außerdem festgestellt, dass der soeben beschriebene Effekt des genannten Strukturelements 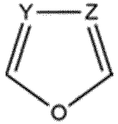 von Formel (I) insbesondere in einer Verbindung mit einer Gruppe A² auftritt, die zumindest zwei konjugierte C=C Doppelbindungen aufweist. Das gemeinsame Vorliegen des Strukturelements 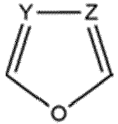 mit einer elektronenziehenden Gruppe A² mit zumindest zwei konjugierten C=C Doppelbindungen - gemäß der vorliegenden Erfindung - stellt ein Strukturmotiv dar, das sich überraschender Weise günstig auf die Absorptionseigenschaften der erfindungsgemäßen Verbindung auswirkt.

Zudem zeigen die erfindungsgemäßen Verbindungen in der Regel eine gute Verarbeitbarkeit und können mit herkömmlichen Verfahren in dünnen organischen Schichten allein oder gemeinsam mit weiteren Materialien abgeschieden werden. Sie verfügen zudem über eine hinreichende thermische, chemische und elektrochemische Stabilität, um den Anforderungen gerecht zu werden, die bei der Fertigung und im Betrieb von fotoaktiven organischen elektronischen Bauelementen üblicherweise an sie gestellt werden.

Schließlich sind die Verbindungen mit einem moderaten technischen Aufwand herstellbar und somit auch vergleichsweise preiswert.

Im Folgenden sollen eine Reihe von bevorzugten Ausführungsformen der vorliegenden Erfindung angegeben werden:
Gemäß einer bevorzugten Ausführungsform ist p gleich 1. Das bedeutet, dass genau einer der drei konjugierten Blöcke D² in der Verbindung vorliegt.

Die Erfinder der vorliegenden Erfindung haben erkannt, dass bereits Verbindungen mit nur einem der konjugierten Blöcke D² gemeinsam mit den beiden sie umgebenden Fünfringen und den Akzeptor-Gruppen A¹ und A² eine hervorragende Absorption im kurzwelligen Bereich des sichtbaren Spektrums zeigen. Gegenüber Verbindungen mit mehr als einem konjugierten Block sind sie zudem einfacher herstellbar, da weniger Syntheseschritte erforderlich sind. Insbesondere verfügen sie auch über ein geringeres Molekulargewicht als analoge Verbindungen mit mehr als einem konjugierten Block und sind aus diesem Grunde zumeist gut verdampfbar. Es konnte zum Beispiel gezeigt werden, dass viele dieser Verbindungen rückstandsfrei im Vakuum verdampft werden können. Aus diesem Grund sind diese Verbindungen in der Regel gut mittels Gasphasenabscheidung oder anderen Verfahren, die ein vorheriges Verdampfen oder Sublimieren der Verbindung erfordern abzuscheiden. Damit lassen sich die Verbindungen mit geringem Aufwand und kostengünstig auch in industriellem Maßstab zur Herstellung von fotoaktiven organischen elektrischen Bauelementen verwenden.

Die Erfinder der vorliegenden Erfindung haben erkannt, dass das Vorliegen von mehr als einem konjugierten Block D² eine Prozessierung in der Flüssigphase erleichtern kann. Das bedeutet, dass eine Abscheidung der Verbindung in organischen Schichten in der Flüssigphase einfacher durchgeführt werden kann, wenn mehr als nur ein konjugierter Block D² vorliegt.

Gemäß einer bevorzugten Ausführungsform der Erfindung weist die Verbindung zumindest einen konjugierten Block D² auf, bei dem es sich nicht um die Struktureinheit: 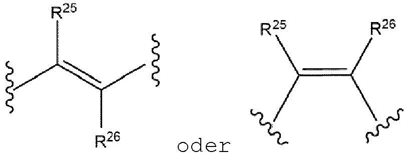 handelt. Dies ist günstig für die Absorptionseigenschaften.

Eine Ausführungsform der Erfindung betrifft die erfindungsgemäße Verbindung aufweisend ein Molekulargewicht zwischen ca. 300 und ca. 2000 g/mol, insbesondere ein Molekulargewicht größer oder gleich 330 g/mol und kleiner oder gleich 1200 g/mol.

Es werden Verbindungen offenbart, wobei A¹ ausgewählt ist aus der Gruppe der folgenden Reste: 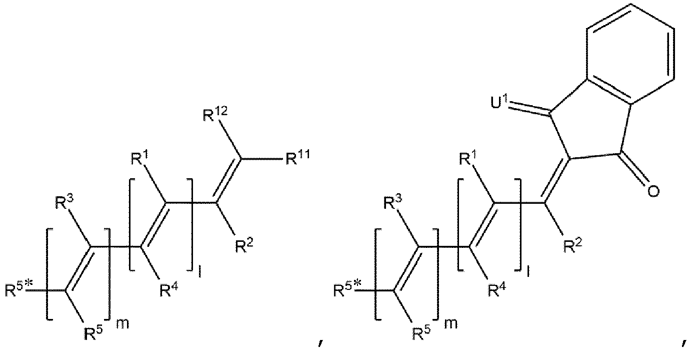 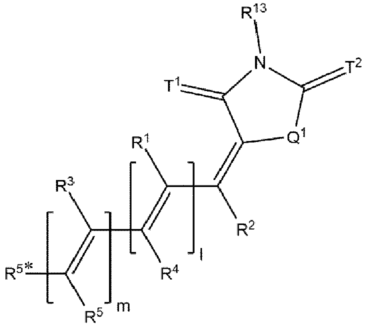 wobei bei jedem dieser drei Reste für A¹ jeweils für jede C=C-Doppelbindung jeweils unabhängig voneinander sowohl das E-Isomer wie auch das Z-Isomer vorliegen kann.

Für jede C=C-Doppelbindung kann also sowohl das E-Isomer
("E" = entgegen; d.h. trans-Konfiguration) wie auch das Z-Isomer
("Z" = zusammen; d.h. cis-Konfiguration) vorliegen.

Dies soll im Folgenden anhand des Beispiels des Restes 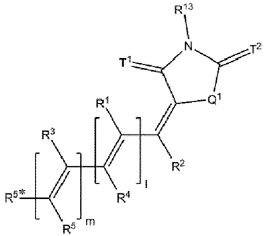 demonstriert werden. Unter Berücksichtigung aller E- und Z-Isomere für die drei C=C-Doppelbindungen ergeben sich für besagten Rest bis zu 8 Stereoisomere:

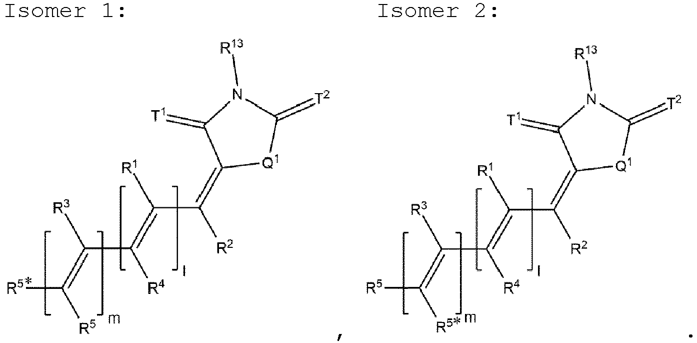

Bei Isomer 1 sind die Reste an der ersten Doppelbindung, R⁵* und R³ cis-ständig zueinander angeordnet, während R⁵* und R³ in Isomer 2 trans-ständig zueinander angeordnet sind (Isomer 2 kann aus Isomer 1 erhalten werden, indem man in Gedanken eine Rotation um 180° um die erste der drei C=C-Doppelbindungen vollzieht).

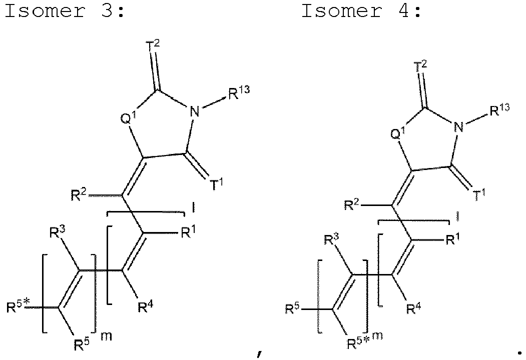

Bei Isomer 1 sind R¹ und R⁴ trans-ständig zueinander angeordnet, während sie bei Isomer 3 cis-ständig zueinander angeordnet sind (Isomer 3 kann aus Isomer 1 erhalten werden, indem man in Gedanken eine Rotation um 180° um die zweite der drei C=C-Doppelbindungen vollzieht). Bei Isomer 3 sind wie bei Isomer 1 an der ersten C=C-Doppelbindung R⁵* und R³ cis-ständig. Bei Isomer 4 sind R⁵* und R³ dagegen trans-ständig (Isomer 4 kann aus Isomer 3 erhalten werden, indem man in Gedanken eine Rotation um 180° um die erste der drei C=C-Doppelbindungen vollzieht).

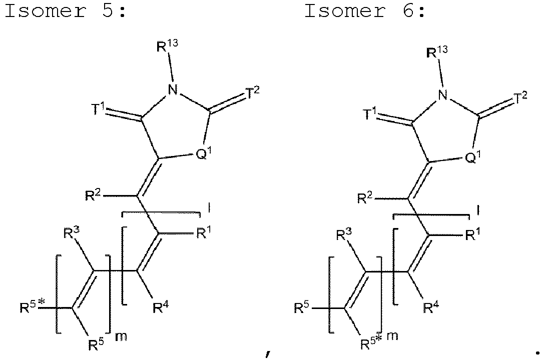

Bei Isomer 3 sind Q¹ und R² cis-ständig zueinander angeordnet, während sie in Isomer 5 trans-ständig zueinander angeordnet sind (Isomer 5 kann aus Isomer 3 erhalten werden, indem man in Gedanken eine Rotation um 180° um die dritte der drei C=C-Doppelbindungen vollzieht). Bei Isomer 5 sind wie bei Isomer 1 an der ersten C=C-Doppelbindung R⁵* und R³ cis-ständig. Bei Isomer 6 sind R⁵* und R³ dagegen trans-ständig (Isomer 6 kann aus Isomer 5 erhalten werden, indem man in Gedanken eine Rotation um 180° um die erste der drei C=C-Doppelbindungen vollzieht).

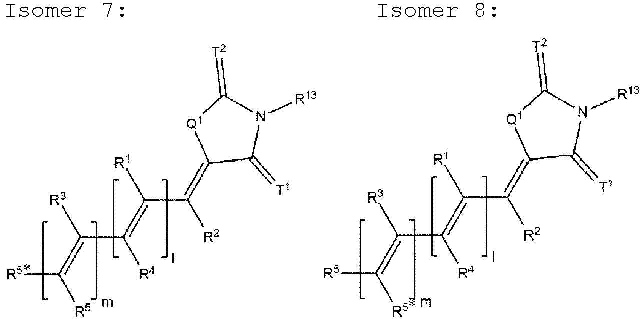

Bei Isomer 1 sind Q¹ und R² cis-ständig zueinander angeordnet, während sie in Isomer 7 trans-ständig zueinander angeordnet sind (Isomer 7 kann aus Isomer 1 erhalten werden indem man in Gedanken eine Rotation um 180°C um die dritte der drei C=C-Doppelbindungen vollzieht). Bei Isomer 7 sind wie bei Isomer 1 an der ersten C=C-Doppelbindung R⁵* und R³ cis-ständig. Bei Isomer 8 sind R⁵* und R³ dagegen trans-ständig (Isomer 8 kann aus Isomer 7 erhalten werden, indem man in Gedanken eine Rotation um 180° um die erste der drei C=C-Doppelbindungen vollzieht).

In analoger Weise können auch für die anderen beiden angegebenen Reste von A¹ sowie jeweils bis zu 8 Stereoisomere, wie sie sich aus der E-Z-Isomerie an den bis zu drei C=C-Doppelbindungen ergeben, vorliegen. Die Stereoisomere sind lediglich der Knappheit halber nicht ausdrücklich dargestellt, verfügen aber im Hinblick auf die elektronischen Eigenschaften über eine vergleichbare Wirkung. Bevorzugt sind jedoch die drei ausdrücklich für A¹ dargestellten Reste.

Hierbei sind die Koeffizienten m und l jeweils unabhängig voneinander 0 oder 1. Beispielsweise können beide Koeffizienten gleichzeitig 0 sein oder beide zugleich 1 sein. Bevorzugt ist genau einer der beiden Koeffizienten 1 und der andere der beiden Koeffizienten 0.

Es werden Verbindungen offenbart, wobei A² ausgewählt ist aus der Gruppe der folgenden Reste: 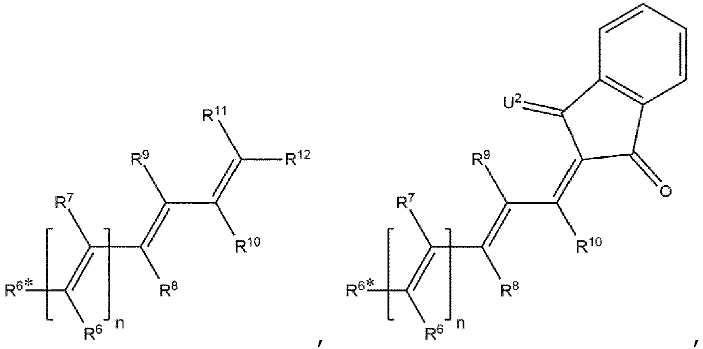 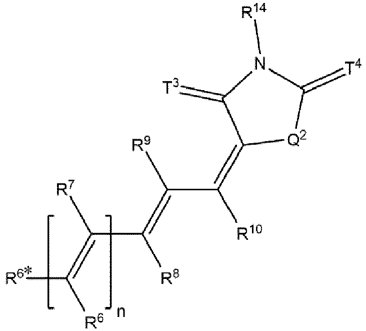 wobei bei jedem dieser drei Reste für A² jeweils für jede C=C-Doppelbindung jeweils unabhängig voneinander sowohl das E-Isomer wie auch das Z-Isomer vorliegen kann.

In analoger Weise wie zuvor schon für A¹ gezeigt, können auch für die drei dargestellten Reste von A¹ eine Stereoisomere, wie sie sich aus der E-Z-Isomerie an den bis zu drei C=C-Doppelbindungen ergibt, vorliegen. Die Stereoisomere sind lediglich der Knappheit halber nicht ausdrücklich dargestellt, verfügen aber im Hinblick auf die elektronischen Eigenschaften über eine vergleichbare Wirkung. Bevorzugt sind jedoch die drei ausdrücklich für A² dargestellten Reste.

Hierbei ist der Koeffizient n jeweils 0 oder 1. Bevorzugt ist n gleich Null.

Die Reste R¹ bis R¹⁰ sowie R⁵* und R⁶* sind für die Gruppen A¹ und A² jeweils unabhängig voneinander ausgewählt aus der Gruppe der folgenden Reste:
- H,
- Halogen,
- CN,
- zyklisches oder offenkettiges C₁-C₂₀-Alkyl,
- zyklisches oder offenkettiges C₂-C₂₀-Alkenyl,
- zyklisches oder offenkettiges C₁-C₂₀-O-Alkyl,
- Aryl
- Heteroaryl,
wobei Wasserstoffatome der Reste Alkyl, Alkenyl, O-Alkyl, Aryl und Heteroaryl jeweils unabhängig voneinander substituiert sein können und wobei jeweils unabhängig voneinander die folgenden Paare von Resten, R¹ und R³, R² und R⁴, R⁴ und R⁵, R³ und R⁵*, R⁷ und R⁹, R⁶ und R⁸, R⁸ und R¹⁰, R⁶* und R⁷, einen Ring miteinander bilden können.

Dies gilt jeweils für die drei oben dargestellten Reste für A¹ und für die drei dargestellten Reste für A². Für den Fachmann ist ersichtlich, dass eine entsprechende Verbrückung bzw. Ringbildung bei den nicht explizit dargestellten E- und Z-Isomeren gleichermaßen möglich ist. Mit einer entsprechenden Ringbildung ist dabei gemeint, dass jeweils Reste, die an einer C=C-Doppelbindung von A¹ cis-ständig zueinander angeordnet sind miteinander einen Ring bilden können. Außerdem kann ein Rest, der an eine Atomgruppe von A¹ angebunden ist, die an einer C=C-Doppelbindung von A¹ angebunden ist und sich zu einem weiteren Rest derselben Doppelbindung cis-ständig befindet, mit diesem weiteren Rest einen Ring bilden. Das Gleiche gilt jeweils für A². Mit anderen Worten ausgedrückt: Reste (R¹ bis R¹⁰) die sich auf der gleichen Seite des konjugierten Rückrates eines der Reste A¹ bzw. A² befinden und sich dabei relativ zueinander in 1,2-Position - an einer C=C-Doppelbindung - oder in einer 1,3-Position - an eine C=C-C-Struktureinheit befinden, d.h. räumlich benachbart sind, können miteinander einen Ring bilden.

Das bislang zu den Resten A¹ und A² ausgeführte gilt mit der Maßgabe, dass für A¹ genau einer der Reste R⁵*, R⁵, R⁴, R³, R² und R¹ eine Anknüpfung 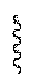 repräsentiert, mittels der A¹ an das Strukturelement 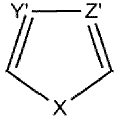 der Verbindung der allgemeinen Formel (I) gebunden ist und mit der weiteren Maßgabe, dass für A² genau einer der Reste R⁶*, R⁶, R⁷, R⁸, R⁹ und R¹⁰ eine Anknüpfung 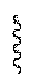 repräsentiert, mittels der A² an das Strukturelement 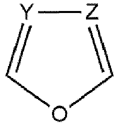 der Verbindung der allgemeinen Formel (I) gebunden ist.

Bevorzugt handelt es sich bei genau einem der Reste R⁵*, R⁵ und R⁴ um die Anknüpfung 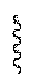 mit der A¹ an das Strukturelement 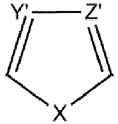 der Verbindung der allgemeinen Formel (I) gebunden ist. Insbesondere handelt es sich bei dem Rest R⁵* um die Anknüpfung 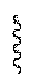 .

Bevorzugt handelt es sich bei genau einem der Reste R⁶*, R⁶ und R⁸ um die Anknüpfung 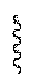 mit der A² an das Strukturelement 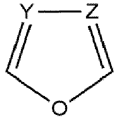 der Verbindung der allgemeinen Formel (I) gebunden ist. Insbesondere handelt es sich bei dem Rest R⁶* um die Anknüpfung 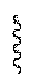 .

Wie angegeben können für R¹ bis R¹⁰ sowie R⁵* und R⁶* Wasserstoffatome der Reste Alkyl, Alkenyl, O-Alkyl, Aryl und Heteroaryl jeweils unabhängig voneinander substituiert sein. Beispielsweise können Wasserstoffatome durch Halogenatome wie etwa Fluoratome ersetzt sein.

In dem Fall der Bildung eines Rings durch Paare von Resten, kann der Ring ein substituierter oder nicht substituierter fünf, sechs oder sieben gliedriger Ring sein. Beispielsweise kann es sich um einen Cyclopenten-, Cyclohexen- oder Cyclohepten-Ring handeln.

Die Begriffe Halogen, CN, zyklisches oder offenkettiges C₁-C₂₀-Alkyl, zyklisches oder offenkettiges C₂-C₂₀-Alkenyl, zyklisches oder offenkettiges C1-C₂₀-O-Alkyl, Aryl und Heteroaryl sind jeweils ebenso zu verstehen, wie bereits im Zusammenhang mit den Resten R^{a} bis R^{c} erläutert.

Die Reste R¹¹ und R¹² sind für die Gruppen A¹ und A² jeweils unabhängig voneinander ausgewählt aus der Gruppe der Reste:
- H,
- CN,
- COOR^{f}, wobei R^{f} ausgewählt ist der Gruppe der Reste: H sowie zyklisches oder offenkettiges C₁-C₂₀-Alkyl, wobei Wasserstoffatome des Alkyls durch Fluoratome ersetzt sein können,
mit der Maßgabe, dass nicht R¹¹ und R¹² beide zugleich H sein können.

Beispielsweise kann genau einer der Reste R¹¹ oder R¹² CN sein und der andere der beiden Reste H. Insbesondere ist es auch möglich, dass R¹¹ und R¹² beide CN sind. Ebenso kann beispielsweise einer der Reste R¹¹ oder R¹² COOR^{f} sein und der andere der beiden Reste H oder CN. Es können auch beide Reste zugleich COOR^{f} sein.

Die Reste R¹³ und R¹⁴ sind für die Gruppen A¹ und A² jeweils unabhängig voneinander aus der gleichen Gruppe von Resten ausgewählt sind wie R^{d} und R^{e}.

U¹, U² sowie T¹ bis T⁴ sind für die Gruppen A¹ und A² jeweils unabhängig voneinander ausgewählt aus der Gruppe bestehend aus:
- O,
- S und
- C(CN)₂.

Beispielsweise könne U¹ und U² jeweils gleich sein. Beispielsweise können U¹ und U² jeweils beide O oder beide S sein. Beispielsweise können auch jeweils T¹ bis T⁴ jeweils gleich sein. Beispielsweise können T¹ bis T⁴ jeweils O sein. Beispielsweise können T¹ bis T⁴ auch jeweils S sein.

Q¹ und Q² sind für die Gruppen A¹ und A² jeweils unabhängig voneinander ausgewählt aus der Gruppe bestehend aus O und S.

Beispielsweise können Q¹ und Q² gleich sein. Beispielsweise können Q¹ und Q² jeweils beide O oder jeweils beide S sein.

Wenn A¹ und A², wie in dieser Ausführungsform beschrieben gewählt, sind, wirkt sich dies besonders günstig auf die Absorptionseigenschaften aus.

Es werden Verbindungen offenbart, wobei für A¹ der Rest R⁵* die Anknüpfung 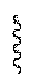 repräsentiert, mittels der A¹ an das Strukturelement 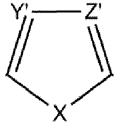 der Verbindung der allgemeinen Formel (I) gebunden ist, sodass A¹ ausgewählt ist aus der Gruppe der folgenden Reste: 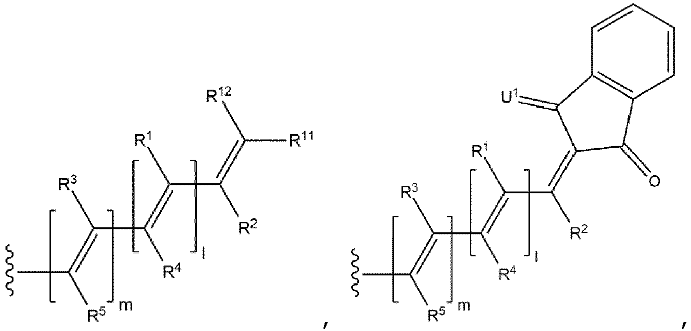 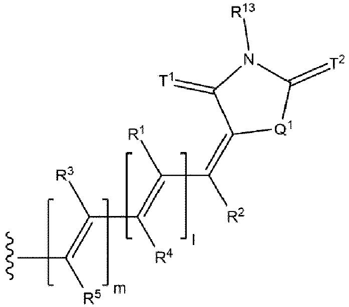 wobei bei jedem dieser drei Reste für A¹ jeweils für jede C=C-Doppelbindung jeweils unabhängig voneinander sowohl das E-Isomer wie auch das Z-Isomer vorliegen kann.

Zudem repräsentiert gemäß dieser Ausführungsform für A² der Rest R⁶* die Anknüpfung 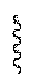 , mittels der A² an das Strukturelement 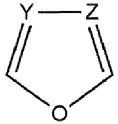 der Verbindung der allgemeinen Formel (I) gebunden ist, sodass A² ausgewählt ist aus der Gruppe der folgenden Reste: 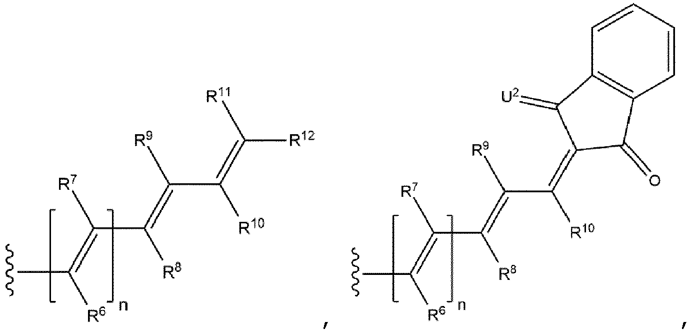 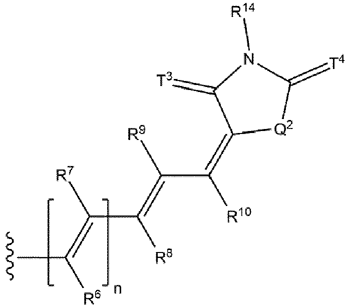 wobei bei jedem dieser drei Reste für A² jeweils für jede C=C-Doppelbindung jeweils unabhängig voneinander sowohl das E-Isomer wie auch das Z-Isomer vorliegen kann.

Gemäß einer bevorzugten Ausführungsform der Erfindung ist die Verbindung der Formel (I) symmetrisch, beispielsweise spiegelsymmetrisch. Insbesondere kann das Molekül eine Spiegelebene senkrecht zur Moleküllängsachse aufweisen. Dann lässt sich die Synthese der erfindungsgemäßen Verbindung vereinfachen.

Gemäß einer Ausführungsform der erfindungsgemäßen Verbindung sind die beiden elektronenziehenden Gruppen A¹ und A² jeweils identisch. Es hat sich gezeigt, dass ein gewisses Maß an Symmetrie bzw. nahezu symmetrische Strukturen oftmals besonders günstige Absorptionseigenschaften zeigen. Auch erleichtert dies in vielen Fällen die Synthese der Verbindung.

Es werden Verbindungen offenbart, die als A¹ 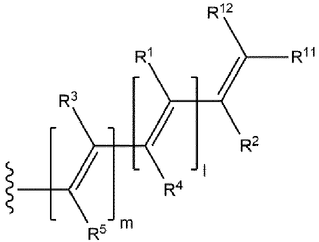 und als A² 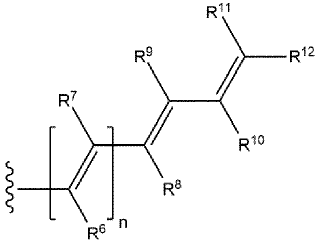 aufweisen.

Verbindungen mit diesen Seitenketten sind gut mittels Knoevenagelkondensation zugänglich. Ihre Herstellung ist technisch einfach umzusetzen. Zudem sind diese Gruppen gute Akzeptoren, insbesondere wenn zumindest einer der beiden Reste R¹¹ oder R¹² gleich CN ist.

Eine bevorzugte Weiterbildung der Erfindung betrifft die erfindungsgemäße Verbindung, wobei beide Reste R¹¹ und R¹² jeweils CN sind.

Durch die in diesem Fall insgesamt vier Nitril-Gruppen kann eine besonders gute Akzeptor-Wirkung der beiden Gruppen A¹ und A² erreicht werden. Die Akzeptor-Wirkung ist von Bedeutung für die Lage der Energieniveaus, welche die Absorptionseigenschaften im Wesentlichen bestimmen. Mit Verbindungen der beschriebenen Form konnte eine besonders gute Absorption auch im hochenergetischen Bereich des sichtbaren Lichts im Zusammenspiel mit den anderen Moleküleinheiten der erfindungsgemäßen Verbindung beobachtet werden.

Es werden Verbindungen offenbart, wobei die Reste R¹ bis R¹⁰ sowie R⁵* und R⁶* jeweils unabhängig voneinander ausgewählt sind aus der Gruppe der folgenden Reste:
- H,
- F,
- C₁-C₅-Alkyl, wobei Wasserstoffe des Alkyls jeweils unabhängig voneinander durch Fluoratome ersetzt sein können.

Auch hier gilt entsprechend die Maßgabe, dass für A¹ genau einer der Reste R⁵*, R⁵, R⁴, R³, R² und R¹ eine Anknüpfung 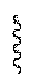 repräsentiert, mittels der A¹ an das Strukturelement 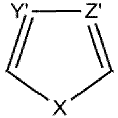 der Verbindung der allgemeinen Formel (I) gebunden ist. Zudem gilt entsprechend die Maßgabe, dass für A² genau einer der Reste R⁶*, R⁶, R⁷, R⁸, R⁹ und R¹⁰ eine Anknüpfung 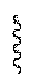 repräsentiert, mittels der A² an das Strukturelement 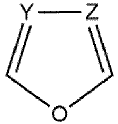 der Verbindung der allgemeinen Formel (I) gebunden ist.

Bevorzugt erfolgt die Anbindung von A¹ wiederum mittels R⁵*, R⁵ und R⁴, insbesondere R⁵*. Ebenfalls bevorzugt erfolgt die Anbindung von A² mittels R⁶*, R⁶ und R⁸, insbesondere R⁶*.

Auch in dieser Ausführungsform können im Einklang mit der allgemeinen Struktur die folgenden Paare von Resten, R¹ und R³, R² und R⁴, R⁴ und R⁵, R³ und R⁵*, R⁷ und R⁹, R⁶ und R⁸, R⁸ und R¹⁰, R⁶* und R⁷ einen Ring miteinander bilden. Bei dem Ring kann es sich insbesondere um einen fünf-, sechs- oder siebengliedrigen Ring handeln. Beispielsweise kann es sich um ein Cyclopenten, Cyclohexen oder Cyclohepten handeln.

Das C₁-C₅-Alkyl kann jeweils auch linear oder verzweigt sein. Der Alkyl-Rest kann auch teilweise oder vollständig fluoriert, bevorzugt perfluoriert, sein, kann aber auch frei von Fluoratomen sein.

Die erfindungsgemäße Verbindung bedarf keiner langkettigen Reste R¹ bis R¹⁰ sowie R⁵* und R⁶*. Vielmehr haben die Erfinder beobachtet, dass auch mit den hier beschriebenen Resten die gewünschte hohe Absorption erzielt werden kann. Dies kann von Vorteil sein, wenn die Verbindung zum Zwecke der Verarbeitung verdampfbar sein soll ohne sich thermisch zu zersetzten. Zudem sind Verbindungen mit den genannten Resten, welche eine vergleichsweise geringe Größe aufweisen mit höherer Atomeffizienz herstellbar und zumeist auch einfacher synthetisierbar als Verbindungen mit größeren Resten R¹ bis R¹⁰ sowie R⁵* und R⁶*.

Gemäß einer Ausführungsform weist die Verbindung Reste R¹ bis R¹⁰ sowie R⁵* und R⁶* auf, von denen zumindest einer ein Fluoratom oder ein fluoriertes Alkyl ist. Bevorzugt sind mindestens zwei oder noch mehr der Reste R¹ bis R¹⁰ Fluoratome oder fluorierte Alkyl-Reste. Durch die Einführung von elektronenziehendem Fluor kann Einfluss auf die Lage der Energieniveaus der Molekülorbitale genommen werden.

Gemäß einer anderen Weiterbildung sind mindestens vier der Reste R¹ bis R¹⁰ sowie R⁵* und R⁶* Wasserstoffatome, bevorzugt mindestens sechs. In diesem Fall sind die elektronenziehenden Gruppen A¹ und A² in der Regel besonders einfach herstellbar.

Die erfindungsgemäße Verbindung weist ein A¹ auf, das ausgewählt ist aus der Gruppe der folgenden Reste:

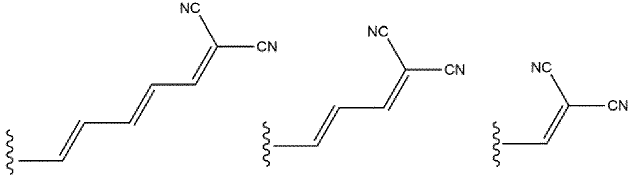

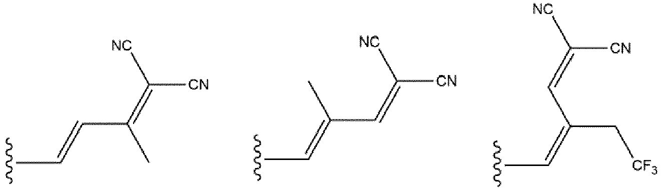

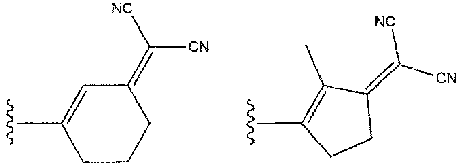

Diese Gruppen für A¹ sind hervorragende Akzeptoren, haben gute elektronenziehende Wirkung, sind ohne großen Aufwand herstellbar und haben sich für die Herstellung von Verbindungen mit besonders guten Absorptionseigenschaften bewährt.

Die erfindungsgemäße Verbindung weist ein A² auf, das ausgewählt ist aus der Gruppe der folgenden Reste:

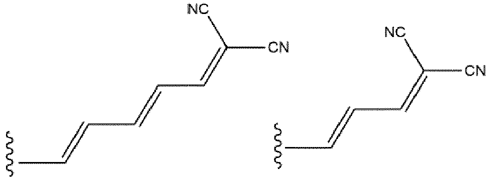

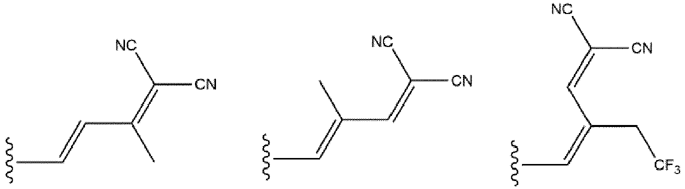

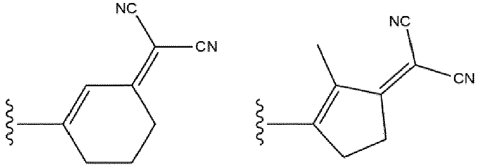

Diese Gruppen für A² sind hervorragende Akzeptoren, haben gute elektronenziehende Wirkung, sind ohne großen Aufwand herstellbar und haben sich für die Herstellung von Verbindungen mit besonders guten Absorptionseigenschaften bewährt.

Die Erfindung betrifft die erfindungsgemäße Verbindung, wobei X für Sauerstoff (O) steht. In diesem Fall weisen beide Fünfringe, welche die mittleren konjugierten Blöcke D² umgeben, jeweils ein Sauerstoffatom im Ring auf. Beispielsweise kann es sich bei den beiden Fünfringen um Furane handeln.

Dadurch, dass für X Sauerstoff gewählt wird, erhöht sich die Symmetrie der Verbindung. Die Symmetrie von Verbindungen spielt oftmals bei Elektronenübergängen eine wichtige Rolle. Die Erfinder der vorliegenden Erfindung haben erkannt, dass die Verbindungen mit X = O besonders gute Absorptionseigenschaften zeigen. Es kann sich bei dem Strukturelement umfassend X zum Beispiel um ein Furan handeln.

Beispielsweise hat sich gezeigt, dass die Verwendung von zwei Furanen für die äußeren Fünfringe zu einer höheren Absorption führt, als dies mit Thiophenen der Fall ist.

Die Erfindung betrifft die erfindungsgemäße Verbindung, mit X gleich Sauerstoff, mit m = n = 0, mit o = q = 0 und mit p ausgewählt aus: 1, 2, 3, 4 und 5, aufweisend die allgemeine Formel:

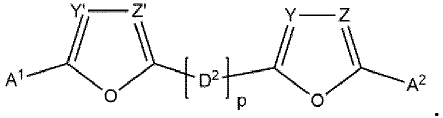

Verbindungen dieser Art sind mit geringem technischem Aufwand zugänglich und verfügen über besonders gute Absorptionseigenschaften. Sie eignen sich gut für die Verwendung z.B. in fotoaktiven organischen Bauelementen.

Eine besonders bevorzugte Ausführungsform der vorliegenden Erfindung betrifft die erfindungsgemäße Verbindung, wobei X gleich Sauerstoff und wobei die Koeffizienten m, n, o und q jeweils Null sind. Zudem ist p ausgewählt aus 1, 2, 3, 4 und 5. Eine derartige Verbindung weist die folgende allgemeine Formel auf:

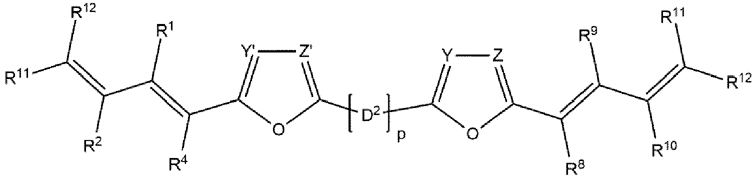

Eine Verbindung dieser Formel weist zwei in ihrer Grundstruktur ähnliche oder gleiche Akzeptoren A¹ und A² auf, sowie zwei Fünfringe, die jeweils ein Sauerstoffatom in der Ringstruktur aufweisen. Bevorzugt sind die beiden Fünfringe Furane, wobei die Furane auch substituiert sein können. Verbindungen der beschriebenen Formel sind nicht nur synthetisch gut zugänglich und gut für die Herstellung von Schichten für fotoaktive organische elektronische Bauteile geeignet, sondern zeigen auch besonders gute Absorptionseigenschaften vor allem für den Wellenlängenbereich zwischen 400 und 600 nm. Gerade für Wellenlängen unter 500 nm sind in der Regel deutlich höhere Absorptionen möglich als mit den meisten herkömmlichen Verbindungen. Beispielsweise ist mit diesen Verbindungen eine stärkere Absorption zu erzielen als mit vergleichbaren Verbindungen, bei welchen die D²-umgebenden Fünfringe Thiophene sind. Die verbesserte Absorption kann für die Erlangung höherer Sensitivitäten von Fotodetektoren oder von lichtempfindlichen Feldeffekttransistoren und insbesondere für die Erzielung einer höheren Effizienz von organischen Solarzellen genutzt werden. All diese Anwendungen erfordern organische Verbindungen, die wie die erfindungsgemäße Verbindung, Sonnenlicht unterschiedlicher Wellenlänge, absorbieren und so nutzbar machen kann.

Gemäß einer bevorzugten Ausführungsform der beiden soeben erwähnten Ausführungsformen der erfindungsgemäßen Verbindung ist p = 1. Dies ist zum Beispiel günstig falls die Verbindung verdampfbar sein soll. Auch sind für p = 1 weniger Reaktionsschritte erforderlich.

Es werden Verbindungen offenbart, wobei Y, Y', Z und Z' jeweils unabhängig voneinander ausgewählt sind aus: N oder CR^{a}, wobei R^{a} ausgewählt ist aus der Gruppe der folgenden Reste:
- H
- F,
- C₁-C₅-Alkyl,
- C₁-C₅-O-Alkyl,
- C₁-C₅-S-Alkyl,
- C₂-C₅-Alkenyl,
- C₂-C₅-O-Alkenyl,
- C₂-C₅-S-Alkenyl,
- C₂-C₅-Alkinyl,
- Phenyl,
wobei Wasserstoffatome der Reste Alkyl, O-Alkyl, S-Alkyl, Alkenyl, O-Alkenyl, S-Alkenyl, Alkinyl und Phenyl jeweils unabhängig voneinander substituiert sein können, insbesondere mit durch Fluoratome ersetzt sein können.

Die Reste Alkyl, O-Alkyl, S-Alkyl, Alkenyl, O-Alkenyl, S-Alkenyl, Alkinyl können dabei jeweils auch verzweigt oder linear sein.

R^{a} kann dabei für die verschiedenen Platzhalter Y, Y', Z und Z' jeweils verschieden gewählt sein.

Gemäß einer Ausführungsform der Erfindung ist zumindest einer der Positionen Y, Y', Z und Z' gleich N. Die Erfinder haben festgestellt, dass Anwesenheit von N an einer der Positionen Y, Y', Z und Z' eine gezielte Absenkung des höchstliegenden mit Elektronen besetzten Molekülorbitals (HOMO) führt. Dies erlaubt es, dass sich das Absorptionsspektrum der erfindungsgemäßen Verbindung hin zu kürzeren Wellenlängen verschieben lässt. Wenn mehrere der Positionen Y, Y', Z und Z' mit N besetzt werden, verstärkt sich der Effekt. Demnach kann es auch bevorzugt sein, dass genau 2, 3 oder gar 4 der Positionen Y, Y', Z und Z' mit N besetzt sind.

Am meisten bevorzugt sind Y, Y', Z, Z' jeweils CH. In diesem Fall sind die beiden Fünfringe, welche die konjugierten Blöcke D² umgeben, jeweils Furan-Ringe. Dies erhöht die Symmetrie des Moleküls, was einen möglichen Grund für die besonders günstigen Absorptionseigenschaften dieser Verbindungen bildet, ohne dabei durch die Theorie gebunden zu sein.

Eine andere Ausführungsform der Erfindung betrifft die erfindungsgemäße Verbindung, wobei die Verbindung zumindest einen konjugierten Block D² aufweist, der zumindest einen Substituenten aufweist, wobei es sich bei dem Substituenten um ein Fluoratom oder um ein Fluoralkyl handelt. Die Erfinder der vorliegenden Erfindung haben festgestellt, dass Fluorsubstituenten in der Regel pro Substituent zu einer gewissen energetischen Absenkung des HOMOs der erfindungsgemäßen Verbindung führen. Die Ausprägung des Effekts hängt dabei von der Größe des Orbitalkoeffizienten des HOMO auf dem jeweiligen Kohlenstoffatom, an welches der Fluorsubstituent bindet, der erfindungsgemäßen Verbindung ab. Dies führt in der Regel zu einer Verschiebung des Absorptionsspektrums hin zu kürzeren Wellenlängen. Schließlich haben die Erfinder auch festgestellt, dass FluorSubstituenten S-F-Wechselwirkungen zu Schwefelatomen von benachbarten S-haltigen Ringen ausbilden können, was eine höhere Koplanarität der erfindungsgemäßen Verbindung fördern kann. Dadurch werden häufig die Ladungstransporteigenschaften der Verbindung verbessert. Auch kann sich dies günstig auf die Absorption auswirken.

Eine andere Ausführungsform der Erfindung betrifft die erfindungsgemäße Verbindung, wobei die Verbindung zumindest einen konjugierten Block D² aufweist, der zumindest einen Alkylsubstituenten C₁-C₂₀-Alkyl, bevorzugt C₁-C₁₀-Alkyl, insbesondere C₁-C₅-Alkyl, aufweist. Die Erfinder der vorliegenden Erfindung haben festgestellt, dass Alkylsubstituenten eine gering ausgeprägte elektronenschiebende Wirkung haben. Sie können zu einer leichten Anhebung des HOMO der erfindungsgemäßen Verbindung führen, wobei die Ausprägung des Effekts von der Größe des Orbitalkoeffizienten des HOMO auf dem jeweiligen Kohlenstoffatom, an welches der Alkylsubstituent bindet, der erfindungsgemäßen Verbindung abhängt.

Eine andere Ausführungsform der Erfindung betrifft die erfindungsgemäße Verbindung, wobei die Verbindung zumindest einen - konjugierten Block D² aufweist, der zumindest einen O-Alkylsubstituenten C₁-C₂₀-O-Alkyl, bevorzugt C₁-C₁₀-O-Alkyl, insbesondere C₁-C₅-O-Alkyl aufweist. O-Alkylsubstituenten, also Alkoxy-Gruppen, zeichnen sich durch eine deutlich ausgeprägte elektronenschiebende Wirkung aus, die wesentlich stärker ist als die von Alkyl-Substituenten. O-Alkyle führen somit zu einer Anhebung des HOMOs. Durch die Einführung von O-Alkyl-Substituenten kann eine Verschiebung des Absorptionsspektrums zu längeren Wellenlängen ermöglicht werden. O-Alkyl-Gruppen sind sterisch zudem weniger anspruchsvoll als Alkylgruppen und können über S-O- oder O-H-Wechselwirkungen zu einer Versteifung der Molekülstruktur der erfindungsgemäßen Verbindung beitragen.

Eine andere Ausführungsform der Erfindung betrifft eine erfindungsgemäße Verbindung, wobei zumindest einer der konjugierten Blöcke D² die allgemeine Formel 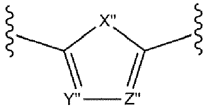 aufweist. In diesem Fall besitzt die Verbindung insgesamt mindestens drei aromatische Fünfring als Teil eines konjugierten ***π-***Elektronensystems. Diese Kombination hat sich in Experimenten als besonders günstig für die Absorptionseigenschaften erwiesen.

Gemäß einer Ausführungsform ist zumindest eine der Positionen Y" und Z" gleich N. Die Erfinder haben festgestellt, dass Anwesenheit von N an einer der Positionen Y" und Z" eine gezielte Absenkung des höchstliegenden mit Elektronen besetzten Molekülorbitals (HOMO) führt. Die Effekte sind hierbei vergleichbar mit den zuvor für N an einer der Positionen Y, Y', Z und Z' beschriebenen Effekten.

Besonders bevorzugt ist es gemäß einer anderen Ausführungsform, wenn zumindest einer der konjugierten Blöcke D² die allgemeine Formel 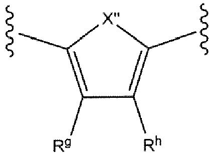 aufweist, wobei die Reste R^{g} und R^{h} unabhängig voneinander aus der Gruppe der folgenden Reste ausgewählt sind:
- H
- F,
- C₁-C₅-Alkyl, wobei einzelne C-Atome durch Heteroatome ersetzt sein können,
- C₁-C₅-O-Alkyl,
- C₁-C₅-S-Alkyl,
wobei Wasserstoffatome der Reste Alkyl, O-Alkyl und S-Alkyl, jeweils unabhängig voneinander mit Fluor substituiert sein können, wobei die Reste R^{g} und R^{h} miteinander in Form einer Ringstruktur verbunden sein können und
wobei an die Ringstruktur ein Aryl-Rest kondensiert sein kann. Der zuletzt genannte Aryl-Rest kann dabei auch substituiert, zum Beispiel fluoriert sein.

Die Reste Alkyl, O-Alkyl und S-Alkyl können dabei jeweils auch linear oder verzweigt sein.

Bei der Ringstruktur kann es sich beispielsweise um einen fünf-, sechs- oder siebengliedrigen Ring handeln, der neben C-Atomen auch O-oder S-Atome aufweisen kann. Der Ring kann also heterozyklisch oder homozyklisch sein. Bevorzugt ist der Ring gesättigt. Beispielsweise kann der Ring substituiert sein. Zum Beispiel kann der Ring Fluoratome oder Alkyl-Gruppen als Substituenten aufweisen. Auch kann der Ring zumindest teilweise fluorierte Alkyl-Gruppen als Substituenten aufweisen. Durch die Fluorierung kann eine elektronenziehende Wirkung erzielt werden.

Gemäß einer bevorzugten Ausführungsform der soeben genannten Ausführungsform sind R^{g} und R^{h} jeweils unabhängig voneinander ausgewählt aus der Gruppe der Reste, H, F, Methyl, Methoxy, Ethly, Ethyloxy, Propyl, Propyloxy, wobei bei Methyl, Methoxy, Ethyl, Ethyloxy, Propyl und Propyloxy jeweils Wasserstoffatome mit Fluor substituiert sein können.

Besonders bevorzugt sind erfindungsgemäße Verbindungen, wobei X" gleich S. Es sind also erfindungsgemäße Verbindungen bevorzugt, die zumindest für einen der konjugierten Blöcke D² einen Fünfring der allgemeinen Formel 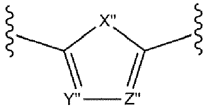 aufweisen, wobei X" gleich Schwefel ist.

Am meisten bevorzugt ist es, wenn der Ring ein substituierter oder nicht substituierter Thiophen-Ring ist, d.h. wenn neben Schwefel keine weiteren Heteroatome im Fünfring vorliegen.

Die Verwendung eines Schwefel-aufweisenden Fünfringes als einer der konjugierten Blöcke für D¹ bis D³ zur Erzielung von hohen Absorptionswerten bei kurzen Wellenlängen des sichtbaren Lichts hat sich experimentell als besonders günstig erwiesen.

Eine weitere besondere Ausführungsform der Erfindung betrifft die erfindungsgemäße Verbindung wobei ein konjugierter Block D², d.h. der Koeffizient p ist 1, bei dem es sich um den konjugierten Block 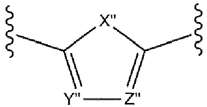 handelt, wobei X" gleich Schwefel ist. In diesem Fall handelt es sich bei der Verbindung um eine Verbindung der folgenden allgemeinen Formel:

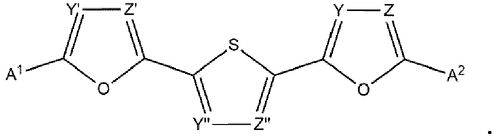

Eine Kombination von zwei äußeren Fünfringen jeweils mit einem Sauerstoff und einem mittleren Fünfring mit Schwefel in Verbindung mit den beiden Akzeptoren A¹ und A² hat sich als besonders geeignetes Strukturelement erwiesen um die günstigen Absorptionseigenschaften in Gestalt einer gut zugänglichen und somit kostengünstigen Verbindung zu erzielen.

Eine weitere Ausführungsform betrifft die erfindungsgemäße Verbindung, wobei zumindest einer der konjugierten Blöcke D² die allgemeine Formel 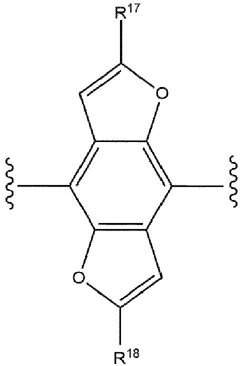 aufweist,
wobei R¹⁷ und R¹⁸ unabhängig voneinander ausgewählt sind aus der Gruppe der folgenden Reste:
- H,
- F,
- C₁-C₅-Alkyl,
- C₁-C₅-O-Alkyl,
- C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können hierbei jeweils auch linear oder verzweigt sein.

Die Erfinder der vorliegenden Erfindung haben beobachtet, dass dieses Strukturelement als einer der konjugierten Blöcke D² hervorragend geeignet ist, um Verbindungen mit den gewünschten hohen Absorptionseigenschaften zu erlangen.

Weiter bevorzugt sind R¹⁷ und R¹⁸ jeweils Wasserstoff, Methyl, Ethyl, Methoxy, Ethoxy. Am meisten bevorzugt sind R¹⁷ und R¹⁸ jeweils Wasserstoff.

Eine Weiterbildung betrifft die erfindungsgemäße Verbindung, wobei zumindest einer der konjugierten Blöcke D² die allgemeine Formel 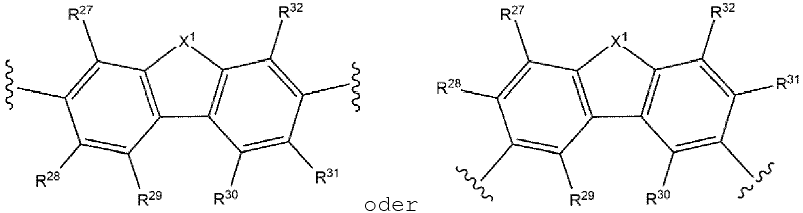 aufweist,
wobei X¹ ausgewählt ist aus: NR^{l}, CR^{l}R^{m},
wobei R^{l} und R^{m} jeweils unabhängig voneinander ausgewählt sind aus der Gruppe der Reste:
   - H,
   - offenkettiges C₁-C₅-Alkyl,
   - C₅-C₁₂-Aryl
   - C₅-C₁₂-Heteroaryl
wobei Wasserstoffatome des offenkettigen C₁-C₅-Alkyls, zumindest teilweise durch Fluoratome ersetzt sein können, und wobei Wasserstoffatome des C₅-C₁₂-Aryls und des C₅-C₁₂-Heteroaryls substituiert sein können, beispielsweise durch Fluoratome ersetzt sein können, und
wobei das offenkettige C₁-C₅-Alkyl linear oder verzweigt sein kann, und
wobei R²⁷ bis R³² unabhängig voneinander ausgewählt sind aus der Gruppe der folgenden Reste:
   - H,
   - F,
   - C₁-C₅-Alkyl,
   - C₁-C₅-O-Alkyl,
   - C₁-C₅-S-Alkyl,
wobei Wasserstoffatome der Reste-Alkyl, O-Alkyl und S-Alkyl jeweils unabhängig voneinander durch Fluoratome ersetzt sein können und wobei die Reste jeweils unabhängig voneinander linear oder verzweigt sein können.

Beispielsweise kann es sich bei dem C₅-C₁₂-Aryl für den Rest R^{l} und R^{m} um ein Phenyl handeln, der wiederum substituiert sein kann.

Eine weitere Ausführungsform betrifft die erfindungsgemäße Verbindung, wobei zumindest einer der konjugierten Blöcke D² die allgemeine Formel 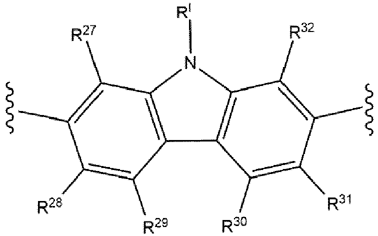 aufweist,
wobei R^{l} H oder ein offenkettiges C₁-C₅-Alkyl oder Phenyl ist, und
wobei R²⁷ bis R³² unabhängig voneinander ausgewählt sind aus der Gruppe der folgenden Reste:
   - H,
   - F,
   - C₁-C₅-Alkyl,
   - C₁-C₅-O-Alkyl,
   - C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können dabei jeweils auch unabhängig voneinander linear oder verzweigt sein.

Die Erfinder der vorliegenden Erfindung haben beobachtet, dass dieses Strukturelement als einer der konjugierten Blöcke D² hervorragend geeignet ist, um Verbindungen mit den gewünschten hohen Absorptionseigenschaften zu erlangen. Carbazol führt ebenso wie Fluoren tendenziell zu kurzwellig verschobener Absorption und zu einem abgesenktem HOMO im Vergleich zur Verwendung eines Thiophens oder Furans als konjugierten Block Carbazol ist dabei ein stärkerer Donor als Fluoren, sodass das HOMO von Carbazolderivaten zumeist höher liegt als das von analogen Fluorenen.

Bevorzugt handelt es sich bei R^{l} um H, Methyl, Ethyl, Propyl oder Butyl.

Weiter bevorzugt ist es, wenn die R²⁷ bis R³² jeweils ausgewählt sind aus der Gruppe umfassend: H, F, Methyl, Ethyl, Methoxy, Ethoxy. Beispielsweise können auch alle Reste R²⁷ bis R³² jeweils Wasserstoff sein.

Eine bevorzugte Weiterbildung betrifft die erfindungsgemäße Verbindung, wobei zumindest einer der konjugierten Blöcke D² die allgemeine Formel 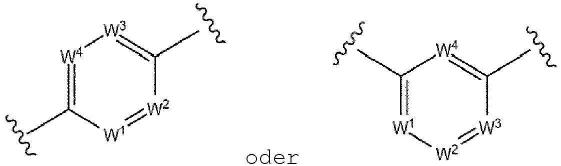 aufweist,
wobei W¹ bis W⁴ unabhängig voneinander ausgewählt sind aus: N und CRⁿ,
wobei Rⁿ ausgewählt ist aus der Gruppe der folgenden Reste:
   - H,
   - F,
   - C₁-C₅-Alkyl,
   - C₁-C₅-O-Alkyl,
   - C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können dabei jeweils auch unabhängig voneinander linear oder verzweigt sein.

Die Erfinder der vorliegenden Erfindung haben beobachtet, dass dieses Strukturelement als einer der konjugierten Blöcke D² hervorragend geeignet ist, um Verbindungen mit den gewünschten hohen Absorptionseigenschaften zu erlangen. Der Einbau führt in der Regel zu kurzwellig verschobener Absorption und abgesenktem HOMO im Vergleich zur Verwendung eines Thiophens oder Furans als konjugierten Block.

Weiter bevorzugt ist es, wenn Rⁿ für W¹ bis W⁴ jeweils unabhängig voneinander ausgewählt ist aus H, Methyl, Methoxy, Ethyl, Ethoxy, Propyl, Propyloxy.

Eine weitere Weiterbildung betrifft die erfindungsgemäße Verbindung, wobei zumindest einer der konjugierten Blöcke D² die allgemeine Formel 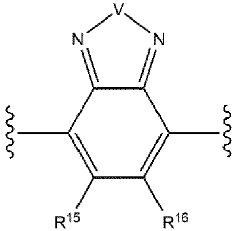 aufweist.

R¹⁵ und R¹⁶ sind dabei bevorzugt ausgewählt aus der Gruppe der folgenden Reste:
- H,
- F,
- C₁-C₅-Alkyl,
- C₁-C₅-O-Alkyl,
- C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können dabei jeweils auch unabhängig voneinander linear oder verzweigt sein.

Beispielsweise kann es sich bei R¹⁵ und R¹⁶ um H, F, Methyl, Ethyl, Methoxy und Ethoxy handeln.

Die Erfinder der vorliegenden Erfindung haben erkannt, dass dieses Strukturelement als einer der konjugierten Blöcke D² ebenfalls geeignet ist, um Verbindungen mit guten Absorptionseigenschaften zu erlangen. Der Einbau dieses Strukturelements führt in der Regel zu einer verbesserten Koplanarität zu den Nachbarstruktureinheiten. Eine Verschiebung des Absorptionsspektrums zu längeren Wellenlängen kann so gezielt herbeigeführt werden.

Gemäß einer bevorzugten Ausführungsform der soeben genannten Ausführungsform handelt es sich bei V um Schwefel oder NR^{k}, wobei R^{k} ausgewählt ist aus der Gruppe H und C₁-C₅-Alkyl.

Eine bevorzugte Weiterbildung der Erfindung betrifft die erfindungsgemäße Verbindung, wobei zumindest einer der konjugierten Blöcke D² die allgemeine Formel 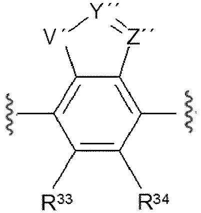 aufweist,
wobei R³³ bis R³⁴ jeweils unabhängig voneinander ausgewählt sind aus der Gruppe der folgenden Reste:
   - H,
   - F,
   - C₁-C₅-Alkyl,
   - C₁-C₅-O-Alkyl,
   - C₁-C₅-S-Alkyl,
wobei Wasserstoffatome der Reste Alkyl, O-Alkyl und S-Alkyl jeweils unabhängig voneinander durch Fluoratome ersetzt sein können, und
wobei V' ausgewählt aus: O, S, Se oder NR^{k};
wobei Y" ausgewählt aus: N oder CR^{g};
wobei Z" ausgewählt aus: N oder CR^{h};
wobei R^{k} ausgewählt ist aus der Gruppe der Reste:
   - H,
   - offenkettiges C₁-C₅-Alkyl,
   - C₅-C₁₂-Aryl
   - C₅-C₁₂-Heteroaryl
wobei Wasserstoffatome des offenkettigen C₁-C₅-Alkyls zumindest teilweise durch Fluoratome ersetzt sein können und wobei Wasserstoffatome des C₅-C₁₂-Aryls und des C₅-C₁₂-Heteroaryls zumindest teilweise substituiert sein können,
wobei R^{g} und R^{h} unabhängig ausgewählt sind wie R^{a}.

Die Erfinder der vorliegenden Erfindung konnten beobachten, dass Verbindungen dieser Art eine besonders hohe Absorption zeigen und sich überraschend gut für die Verwendung in fotoaktiven organischen Bauelementen eignen.

Gemäß einer bevorzugten Ausführungsform der soeben genannten Weiterbildung sind die Reste R³³ bis R³⁴ jeweils unabhängig voneinander ausgewählt aus der Gruppe: H, F, Methyl, Ethyl, Methoxy und Ethoxy, wobei Methyl, Ethyl, Methoxy und Ethoxy jeweils zumindest teilweise fluoriert sein können. Beispielsweise handelt es sich bei R³³ bis R³⁴ jeweils um Wasserstoff.

Gemäß einer bevorzugten Ausführungsform handelt es sich bei R^{k} um Wasserstoff.

Eine weitere Weiterbildung betrifft die erfindungsgemäße Verbindung, wobei zumindest einer der konjugierten Blöcke D² die allgemeine Formel 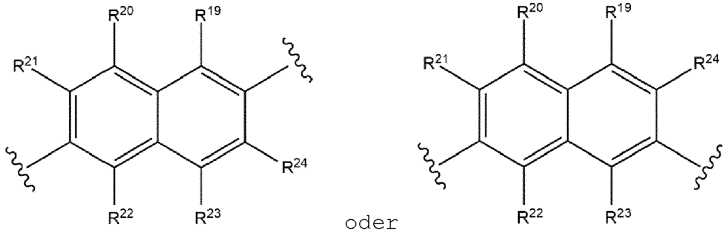 aufweist.

R¹⁹ bis R²⁴ sind dabei bevorzugt ausgewählt aus der Gruppe der folgenden Reste:
- H,
- F,
- C₁-C₅-Alkyl,
- C₁-C₅-O-Alkyl,
- C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können dabei jeweils auch unabhängig voneinander linear oder verzweigt sein.

Beispielsweise kann es sich bei R¹⁹bis R²⁴ um H, F, Methyl, Ethyl, Methoxy, Ethoxy, Propyl und Propyloxy handeln.

Die Erfinder der vorliegenden Erfindung haben erkannt, dass dieses Strukturelement als einer der konjugierten Blöcke D² ebenfalls geeignet ist, um Verbindungen mit guten Absorptionseigenschaften zu erlangen. Naphthalen führt in der Regel zu einer Absorption bei kürzeren Wellenlängen und zu einem abgesenkten HOMO im Vergleich zur Verwendung eines Thiophens oder Furans als konjugierten Block.

Eine weitere Ausführungsform der Erfindung betrifft die erfindungsgemäße Verbindung, wobei zumindest einer der konjugierten Blöcke D² die allgemeine Formel 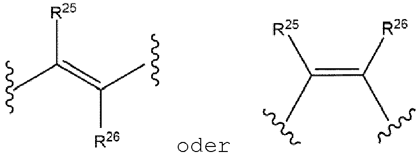 aufweist.

R²⁵ und R²⁶ können jeweils unabhängig voneinander bevorzugt ausgewählt sein aus der Gruppe der folgenden Reste:
- H,
- F,
- C₁-C₅-Alkyl,
- C₁-C₅-O-Alkyl,
- C₁-C₅-S-Alkyl,
wobei Wasserstoffatome der Reste Alkyl, O-Alkyl und S-Alkyl jeweils unabhängig voneinander durch Fluoratome ersetzt sein können. Die Reste Alkyl, O-Alkyl und S-Alkyl können dabei jeweils auch unabhängig voneinander linear oder verzweigt sein.

Beispielsweise kann es sich bei R²⁵ und R²⁶ um H, F, Methyl, Ethyl, Methoxy und Ethoxy handeln. Besonders bevorzugt sind H, F und Methyl.

Gemäß einer anderen Ausführungsform handelt es sich nur dann bei zumindest einem der konjugierten Blöcke D² um eine Struktureinheit der allgemeinen Formel 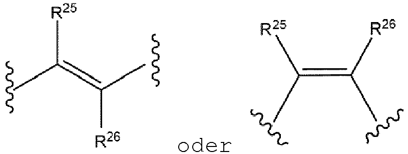 sofern zumindest ein weiterer Donor D² vorliegt, bei dem es sich nicht um einen konjugierten Block der Struktureinheit 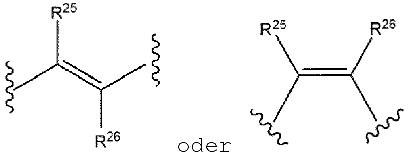 handelt.

Eine besonders bevorzugte Ausführungsform der vorliegenden Erfindung betrifft die erfindungsgemäße Verbindung, wobei die konjugierten Blöcke D² unabhängig voneinander ausgewählt sind aus der Gruppe der folgenden Struktureinheiten:

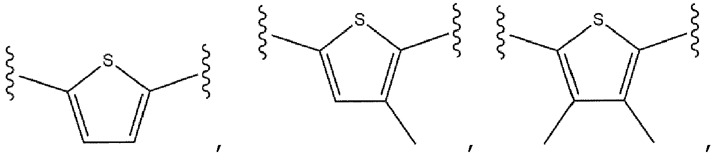

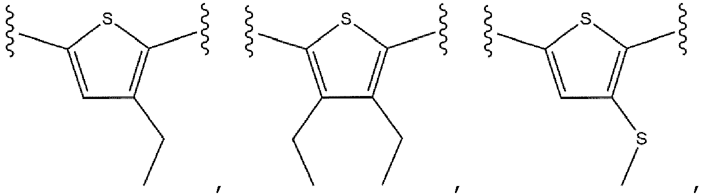

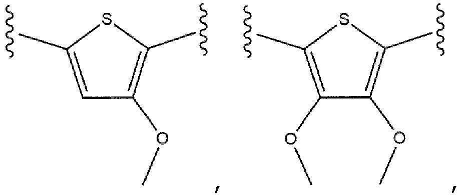

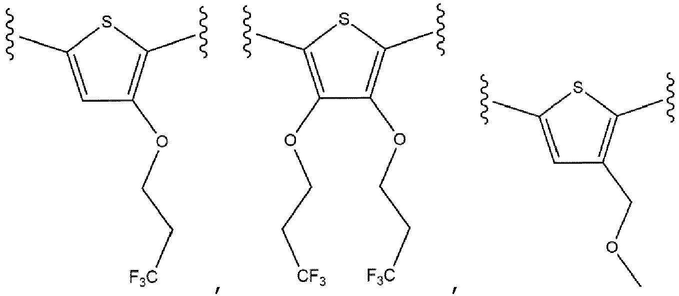

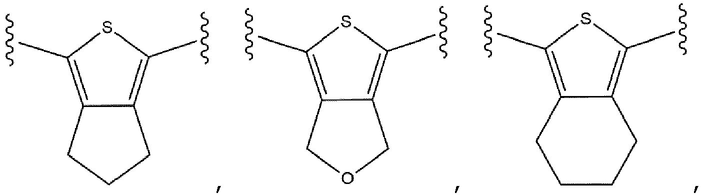

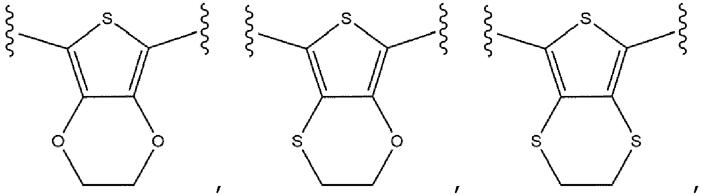

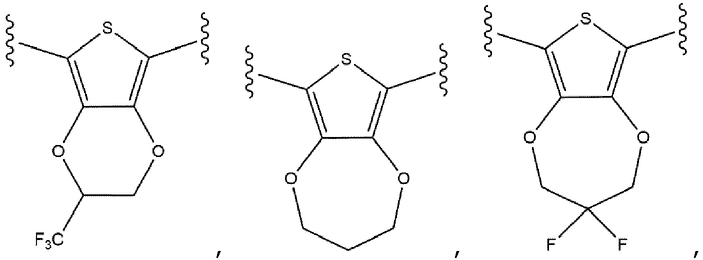

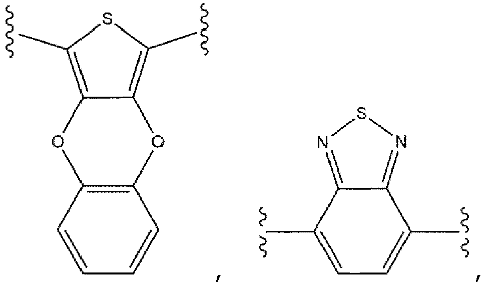

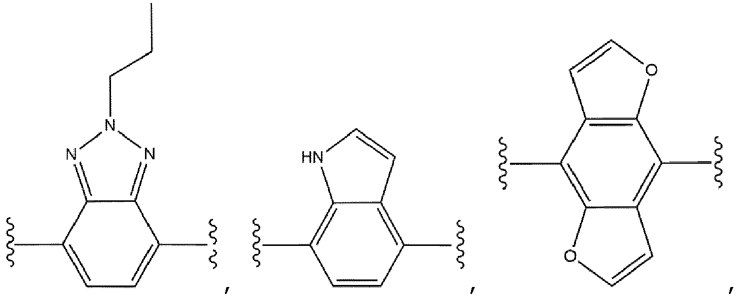

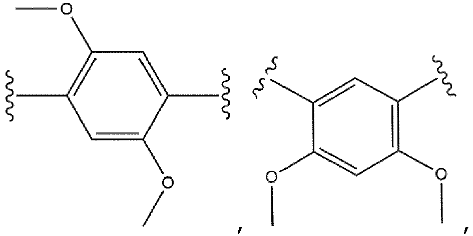

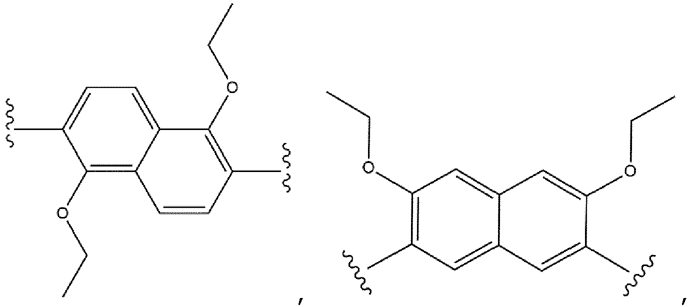

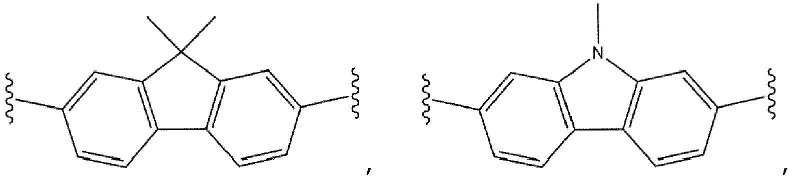

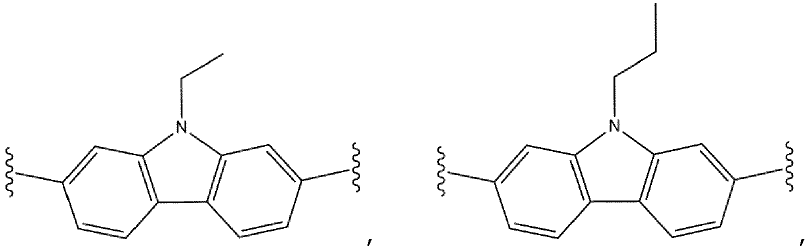

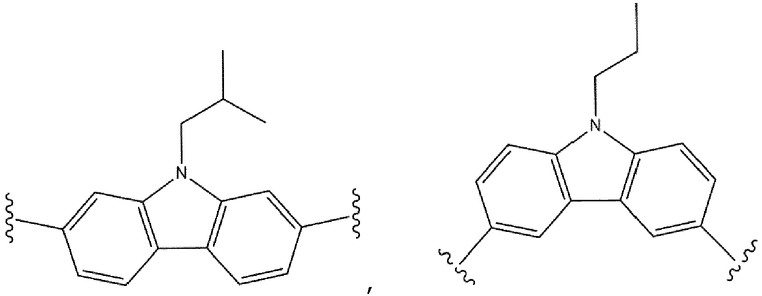

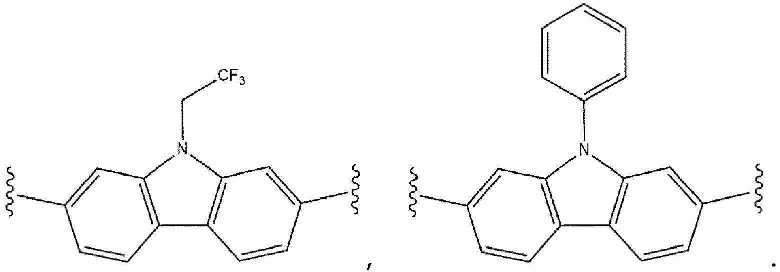

Die Erfinder der vorliegenden Erfindung haben beobachtet, dass Verbindungen aufweisend einen dieser konjugierten Blöcke für D² unerwartet hohe Absorption im kurzwelligen Bereich v.a. für Wellenlängen kleiner 500 nm aufweisen, welche die Absorption von herkömmlichen Verbindungen in der Regel deutlich übersteigen.

Die vorliegende Erfindung betrifft neben der erfindungsgemäßen Verbindung zudem die Verwendung der erfindungsgemäßen Verbindung in einem fotoaktiven organischen elektronischen Bauelement.

Fotoaktive organische elektronische Bauelemente wandeln elektromagnetische Strahlung in elektrische Energie um. Dieses Prinzip findet in verschiedenartigen fotoaktiven organischen elektronischen Bauelementen Anwendung. Der Einsatz von erfindungsgemäßen Verbindungen, die eine besonders hohe Absorption im gesamten Wellenlängenbereich des sichtbaren Lichts zeigen und zudem das kurzwellige sichtbare Licht (ca. 400 bis 600 nm) effektiver nutzen als herkömmliche organische Verbindungen eignen sich bereits aufgrund ihrer exzellenten Absorptionseigenschaften hervorragend für die Verwendung in fotoaktiven organischen elektronischen Bauelementen. Auch Photonen mit hoher Energie können absorbiert werden und somit Elektronenübergänge anregen. Da die erfindungsgemäßen Verbindungen Photonen mit höherer Energie besser absorbieren können, nutzen sie das kurzwellige Spektrum des sichtbaren Lichts effizienter. Mit dem Einsatz der erfindungsgemäßen Verbindung ist es somit möglich die Effizienz und Sensitivität von fotoaktiven organischen elektronischen Bauelementen zu verbessern. So kann bei Fotodetektoren, die die erfindungsgemäßen Verbindungen nutzen, eine bessere Sensitivität ermöglicht werden als bei vergleichbaren Fotodetektoren mit herkömmlichen organischen Verbindungen, die nicht die günstigen Absorptionseigenschaften der erfindungsgemäßen Verbindung zeigen. Beispielsweise können die erfindungsgemäßen Verbindungen neben Fotodetektoren auch für lichtempfindliche Feldeffekttransistoren eingesetzt werden. Auch ein Einsatz im Bereich der Photoleiter für Druck- & Kopiermaschinen ist denkbar.

Analog kann auch in anderen fotoaktiven organischen elektronischen Bauelementen die Sensitivität oder die Effizienz durch den Einsatz der erfindungsgemäßen Verbindungen gesteigert werden.

Am meisten bevorzugt ist hierbei der Einsatz der erfindungsgemäßen Verbindung in einem fotoaktiven organischen elektronischen Bauelement, bei dem es sich um eine organische Solarzelle handelt. Durch die effiziente Nutzung von sichtbarem Licht auch mit kurzen Wellenlängen mit den erfindungsgemäßen Verbindungen ist es möglich organische Solarzellen mit einer höheren Effizienz herzustellen, als Solarzellen, die auf der Basis von organischer Verbindungen basieren, die sich nicht durch diese günstigen Absorptionseigenschaften auszeichnen.

Zudem verfügen die erfindungsgemäßen Verbindungen auch über hinreichende Ladungsträgertransporteigenschaften, die sie für den Einsatz in fotoaktiven organischen elektronischen Bauelementen im Allgemeinen und für organische Solarzellen im Besonderen geeignet machen.

Die vorliegende Erfindung betrifft außerdem ein fotoaktives organisches elektronisches Bauelement, umfassend die erfindungsgemäße Verbindung.

Wie soeben beschrieben haben die Erfinder der vorliegenden Erfindung festgestellt, dass die erfindungsgemäßen Verbindungen aufgrund ihrer Absorptionseigenschaften hervorragend für den Einsatz in fotoaktiven organischen elektronischen Bauelementen geeignet sind.

Gemäß einer bevorzugten Ausführungsform weist das fotoaktive organische elektronische Bauelement eine fotoaktive Schicht auf, beispielsweise eine lichtempfindliche Schicht, welche die erfindungsgemäße Verbindung enthält.

Gemäß einer weiteren Ausführungsform weist das fotoaktive organische elektronische Bauelement zumindest zwei Elektroden auf, zwischen welchen die fotoaktive Schicht angeordnet ist.

Gemäß einer weiteren Ausführungsform weist das Bauelement weitere Schichten, insbesondere Ladungstransportschichten wie etwa Elektronentransportschichten und Lochtransportschichten auf.

Gemäß einer weiteren Ausführungsform weist das Bauelement ein Substrat auf. Insbesondere kann eine der Elektroden des Bauelements auf dem Substrat aufgebracht sein.

Gemäß einer weiteren Ausführungsform handelt es sich bei dem fotoaktiven organischen elektronischen Bauelement um organische Solarzellen, Photodetektoren, lösungsmittelprozessierte Solarzellen, xerographische Schichten, Photoleiter für Druck- oder Kopiermaschinen.

Gemäß einer besonders bevorzugten Ausführungsform handelt es sich bei dem fotoaktiven organischen elektronischen Bauelement um eine organische Solarzelle. Solarzellen umfassend die erfindungsgemäße Verbindung ermöglichen eine besonders effiziente Nutzung des kurzwelligen Spektrums des sichtbaren Lichts.

Gemäß einer Weiterbildung der soeben beschriebenen organischen Solarzelle weist die organische Solarzelle einen fotoaktiven Bereich auf, der wenigstens ein organisches Donormaterial in Kontakt mit wenigstens einem organischen Akzeptormaterial aufweist, wobei das Donormaterial und das Akzeptormaterial einen Donor-Akzeptor-Heteroübergang, im speziellen auch eine sogenannte bulk heterojunction (BHJ, dt. Volumen Mischschicht), ausbilden und wobei der photoaktive Bereich wenigstens eine Verbindung der Formel I enthält.

Beispielsweise kann die erfindungsgemäße Verbindung einem gängigen Aufbau von organischen Solarzellen, wie er in der Literatur beschrieben ist, eingesetzt werden.

Ein bereits literaturbekannter Aufbau einer gängigen organischen Solarzelle besteht beispielsweise in pin- oder nip-Dioden [Martin Pfeiffer, "Controlled doping of organic vacuum deposited dye layers: basics and applications", PhD thesis TU-Dresden, 1999 und WO 2011 161 108]: eine pin Solarzelle besteht dabei aus einem Träger/Substrat mit daran anschließendem meist transparentem Grundkontakt, p-Schicht(en), i-Schicht(en), n-Schicht(en) und einem Deckkontakt. Eine nip-Solarzelle besteht aus einem Träger/Substrat mit daran anschließendem meist transparentem Grundkontakt, n-Schicht(en), i-Schicht(en), p-Schicht(en) und einem Deckkontakt.

Dabei bedeutet, dass n bzw. p-Dotierung zu einer Erhöhung der Dichte freier Elektronen bzw. Löcher im thermischen Gleichgewichtszustand führt. Damit sind solche Schichten primär als Transportschichten zu verstehen. Es ist auch möglich, dass n- oder p-Schicht(en) zumindest teilweise nominell undotiert sind und nur aufgrund der Materialeigenschaften (z.B. unterschiedliche Beweglichkeit) oder aufgrund unterschiedlicher Verunreinigungen (z.B. verbliebene Reste aus der Synthese oder der Schichtherstellung) oder durch Einflüsse der Umgebung (z.B. angrenzende Schichten, Eindiffusion von Metallen oder anderen organischen Materialien, Gasdotierung aus der Umgebungsatmosphäre) bevorzugt elektronenleitende oder löcherleitende Eigenschaften besitzen. In diesem Sinne sind derartige Schichten bevorzugt als Transportschichten zu verstehen.

Die Exzitonen gelangen durch Diffusion an eine derartige Grenzfläche, wo Elektronen und Löcher voneinander getrennt werden. Das Material, welches die Elektronen aufnimmt wird als Akzeptor und das Material, welches die Löcher aufnimmt wird als Donor oder Donator bezeichnet.

Die Bezeichnung i-Schicht kennzeichnet eine undotierte oder intrinsiche Schicht. Eine oder mehrere i-Schichten können dabei aus einem Material (planar Heterojunctions, PHJ) als auch aus einer Mischung zweier oder mehrerer Materialien bestehen, sogenannten bulk heterojunctions (BHJ), die ein interpenetrierendes Netzwerk aufweisen.

Weiterhin aus der Literatur bekannt sind organische pin-Tandemzellen und pin-Mehrfachzellen DE 10 2004 014 046. Die WO 2011 161 108 A1 offenbart dazu einen Vorschlag zur Realisierung in Form eines photoaktives Bauelements mit einer Elektrode und einer Gegenelektrode, wobei zwischen den Elektroden zumindest ein organisches Schichtsystem angeordnet ist, weiterhin mit mindestens zwei photoaktiven Schichtsystemen und zwischen den photoaktiven Schichtsystemen zumindest zwei verschiedene Transportschichtsysteme des gleichen Ladungsträgertyps, dadurch gekennzeichnet, dass ein Transportschichtsystem energetisch an eines der beiden photoaktiven Schichtsysteme angepasst ist und das andere Transportschichtsystem transparent ausgeführt ist.

Die erfindungsgemäßen Schichten können insbesondere in den genannten Typen von fotoaktiven organischen elektronischen Bauelementen oder in ähnlichen allgemein bekannten fotoaktiven organischen elektronischen Bauelementen in der jeweiligen Absorberschicht als Absorbermaterial eingesetzt werden.

Gemäß einer weiteren Ausführungsform handelt es sich bei dem fotoaktiven organischen elektronischen Bauelement um eine Einzelzelle, eine Tandem-, Tripel-, Quadrupel- oder eine andere Mehrfachzelle. Unter Tandemzelle wird in vorliegender Anmeldung verstanden, dass zwei funktionale Zellen räumlich übereinander gestapelt und in Reihe verschaltet sind, wobei zwischen den Zellen eine oder mehrere Zwischenschichten angeordnet sein können. Ebenso wird unter Mehrfachzelle oder Multijunction-Zelle verstanden, dass mehr als zwei funktionale Zellen räumlich übereinander gestapelt und seriell verschaltet sind, wobei zwischen den Zellen eine Zwischenschicht angeordnet sein kann.

Bevorzugt besteht das Bauelement aus einer Kombination aus nip, ni, ip, pnip, pni, pip, nipn, nin, ipn, pnipn, pnin oder pipn-Strukturen, die der mehrere unabhängige Kombinationen, die mindestens eine i-Schicht enthalten, übereinander gestapelt sind.

Gemäß einer anderen Weiterbildung handelt es sich bei dem erfindungsgemäßen fotoaktiven organischen elektronischen Bauelement, um ein Bauelement, dass zumindest eine Schicht umfassend die erfindungsgemäße Verbindung aufweist, wobei die erfindungsgemäße Verbindung und/oder die besagte Schicht mittels Vakuumprozessierung, Gasphasenabscheidung oder Lösungsmittel-Prozessierung abgeschieden wird.

Die Erfindung betrifft zudem eine Verbindung für die Herstellung der erfindungsgemäßen Verbindung, aufweisend die folgende allgemeine Formel: 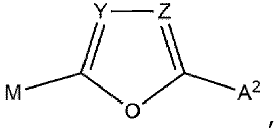 wobei Y, Z und A² so definiert sind, wie in einem der vorherigen Ansprüche und wobei M ausgewählt ist aus einer der folgenden funktionellen Gruppen:

-SnR*₃, -B(OR*)₂, -Zn-Hal*, -Mg-Hal*,

wobei es sich bei R* um ein C₁-C₁₀-Alkyl handelt und wobei es sich bei der Gruppe Hal* um ein Halogen handelt. Mit Halogen sind F, Cl, Br und I gemeint, wobei Cl, Br und I bevorzugt sind.

Die Verbindung ist ein wichtiges Ausgangsmaterial, für die Herstellung der erfindungsgemäßen Verbindung nach Anspruch 1.

Die Anmeldung offenbart weiterhin ein Verfahren zur Herstellung der erfindungsgemäßen Verbindung. Das Verfahren umfasst als Verfahrensschritt eine Kupplungsreaktion, in der die soeben beschriebene Verbindung der allgemeinen Formel 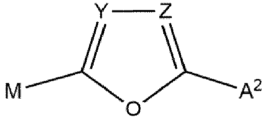 mit einer weiteren Verbindung der folgenden allgemeinen Formel 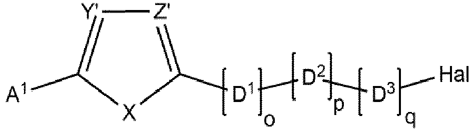 reagiert.

A¹, Y', Z', X, D¹ bis D³ und o, p und q sind für die weitere Verbindung dabei so definiert, wie für die erfindungsgemäße Verbindung beschrieben. Die Gruppe Hal steht zudem für ein Halogen. Dabei kann es sich um F, Cl, Br und I handeln, wobei Cl, Br und I bevorzugt sind, insbesondere Br und I.

In einer weiteren Ausführungsform des Verfahrens umfasst das Verfahren eine Kupplungsreaktion, bei welcher zwei Äquivalente der beschriebenen Verbindung mit der allgemeinen Formel 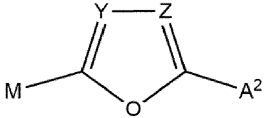 mit einer weiteren Verbindung der allgemeinen Formel 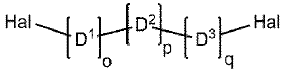 reagieren, wodurch auf vereinfachtem Weg spiegelsymmetrische erfindungsgemäße Verbindungen hergestellt werden können, bei denen X = O, Y = Y', Z = Z' und A¹ = A².

Die Erfinder der vorliegenden Erfindung haben herausgefunden, dass die erfindungsgemäßen Verbindungen in guten Ausbeuten und Selektivitäten mit vertretbarem experimentellem Aufwand mittels Kupplungsreaktionen zugänglich sind.

Gemäß einer bevorzugten Ausführungsform des Verfahrens kommen bei der Kupplungsreaktion Pd-basierte Katalysatoren zum Einsatz. Besonders gut geeignet ist beispielsweise Pd(PPh₃)₄.

Bevorzugt handelt es sich bei der Kupplungsreaktion des Verfahrens um eine Reaktion ausgewählt aus der Gruppe der folgenden Kupplungsreaktionen: Stille-Kupplung, Negishi-Kupplung oder Suzuki-Kupplung. Diese Kupplungsreaktionen haben sich als besonders geeignet für die Synthese der erfindungsgemäßen Verbindungen erwiesen.

Im Anschluss soll die Synthese der erfindungsgemäßen Verbindungen im Allgemeinen und insbesondere Anhand einer Reihe von konkreten Beispielen erläutert werden. Vorteilhafterweise können die erfindungsgemäßen Verbindungen nach einem einfachen Baukastensystem leicht und in guten Ausbeuten zugänglich gemacht werden.

Die folgende schematische Darstellung ist eine vereinfachte Repräsentation einer Syntheseroute für erfindungsgemäße Verbindungen. Der Buchstabe k bezeichnet hierbei eine erfindungsgemäße Verbindung: 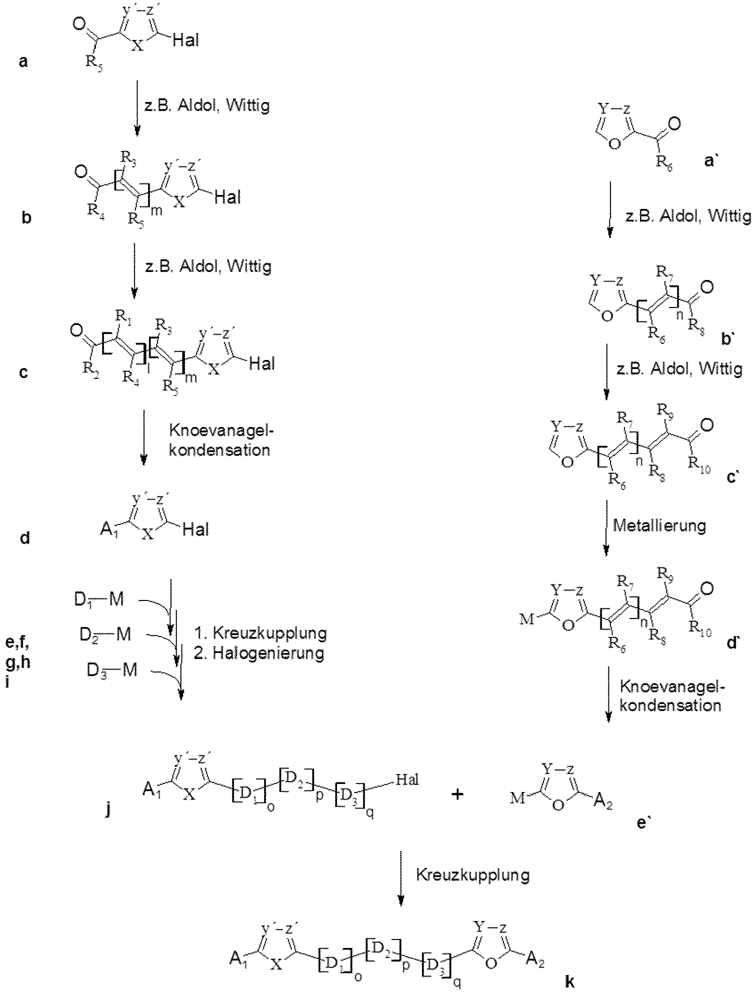 M kann dabei beispielsweise aus einer der folgenden funktionellen Gruppen ausgewählt sein:

-SnR*₃, -B(OR*)₂, -Zn-Hal*, -Mg-Hal*,

wobei es sich bei R* um ein C₁-C₁₀-Alkyl handelt und wobei es sich bei der Gruppe Hal* um ein Halogen (F, Cl, Br, I) handelt. Auch die Abkürzung Hal steht für ein Halogen ausgewählt aus F, Cl, Br, I.

Verbindung **a** ist kommerziell erhältlich, oder kann aus kommerziell verfügbaren Bausteinen durch den Fachmann mittels bekannter Methoden (Halogenierung, Acetylierung oder Carbonylierung) hergestellt werden. Die Umwandlung zu **b** kann beispielsweise durch eine Aldolreaktion oder Wittigreaktion oder andere typische Reaktionen für die Erzeugung einer Doppelbindung aus einer Carbonylfunktion erfolgen. Auch die direkte Herstellung von **b** aus nicht halogenhaltigen (hetero-) aromatischen Komponenten, beispielsweise durch Heck-Reaktion, ist möglich. Zur Herstellung von Verbindung **c** ist dieser Schritt beliebig wiederholbar. Verbindung **d** kann beispielsweise durch eine Knoevenagel-Kondensation mit einer eine Methyleneinheit tragenden Verbindung hergestellt werden. In einer beliebig wiederholbaren Reihe von Kreuzkupplungsreaktion, wie beispielsweise Suzuki-, Negishi- oder Stille-Kupplungen und deren Varianten, mit anschließender Einführung eines Halogenatoms (insbesondere Cl, Br oder I) werden die Struktureinheiten D¹ bis D³ in die Struktur der Verbindung eingebaut. Auf diesem Wege werden die Intermediate **e-j** erhalten. Anstelle des Einsatzes der metallierten Struktureinheiten D¹ bis D³ ist ebenfalls die Verwendung nicht metallierter Einheiten D¹ bis D³, oder anderweitig aktivierter Einheiten D¹ bis D³ denkbar.

Verbindung **a'** ist kommerziell erhältlich, oder kann aus kommerziell verfügbaren Bausteinen durch den Fachmann bekannte Methoden zur Acetylierung oder Carbonylierung hergestellt werden. Die Umwandlung zu **b'** kann beispielsweise durch Aldolreaktion oder Wittigreaktion oder andere typische Synthesen zur Erzeugung einer Doppelbindung aus einer Carbonylfunktion erfolgen. Zur Herstellung von Verbindung **c'** ist dieser Schritt beliebig wiederholbar. Auch die direkte Herstellung von **b'** oder **c'** aus einem unsubstituierten (hetero-) aromaten beispielsweise durch Heck-Reaktion, oder Umsetzung mit ungesättigten Dialkylaminosubstituierten Carbonylen ist möglich. Die Verbindung von **d'** kann durch Metallierung von Verbindung **c'** erfolgen. Bei dieser Umsetzung kann die Carbonylfunktion intermediär geschützt werden. Verbindung **e'** kann beispielsweise durch Knoevenagel-Kondensation mit einer eine Methyleneinheit tragenden Verbindung hergestellt werden. Durch eine typische Kreuzkupplungsreaktion wie beispielsweise Suzuki-, Negishi- oder Stille-Kupplung und deren Varianten, kann eine Komponente des Typs **j** mit einer Komponente des Typs **e'** zu den beanspruchten Verbindungen des Typs **k** umgesetzt werden.

Bei Vorhandensein einer Spiegelebene senkrecht zur Moleküllängsachse kann die Synthese aufgrund des symmetrischen Molekülaufbaus entsprechend vereinfacht gestaltet werden.

Grundsätzlich ist bei allen C-C-Kupplungsreaktionen die Umkehrung der Metall- und Halogenfunktionalitäten denkbar, sowie der beiderseitige Einsatz anderer funktioneller Gruppen, wie beispielsweise aber nicht limitierend Sulfonate, Triflate und Carbonsäuren, oder nicht aktivierte, sogenannte C-H-aktivierte Spezies. In der Regel, aber nicht ausschließlich erfolgen die C-C-Kreuzkupplungsreaktionen unter Verwendung eines Katalysators.

Unter Anderem wurden entsprechende Bromidverbindungen und Stannylverbindungen aus ihren Vorläufermolekülen synthetisiert und anschließend zu erfindungsgemäßen Verbindungen umgesetzt. Im Folgenden sollen drei verschiedene Wege beispielhaft aufgezeigt werden.

Die Kopplung mittels einer
a. doppelt inversen Stille-Kopplung,
b. doppelten Stille-Kopplung oder
c. einfachen Stille-Kopplung.

Analog dazu eigenen sich auch weitere literaturbekannte Kopplungsreaktionen wie beispielsweise die Heck-Reaktion oder Kumada-Kupplung. Suzuki-, Negishi- oder Stille-, Kumada- oder Hiyama- und weitere Kopplungsreaktionen sind unter anderem in "Metal-Catalyzed Cross-Coupling Reactions, 2nd, Completely Revised and Enlarged Edition" (Wiley VCH, ISBN: 978-3-527-30518-6) beschrieben (Suzuki: Seiten 41-123, Negishi: Seiten 619-670, Stille: Seiten 125-161, Kumada: Seiten 671-698, Hiyama: Seiten 163-216, weitere Kopplungsreaktionen: Seiten 815-889).

Nachfolgend sind die entsprechenden Allgemeinen Arbeitsvorschriften (AAV1 bis AAV3) für die Versionen a, b und c aufgeführt:

### a) Allgemeine Arbeitsvorschrift (AAV1)

1 mmol Dibromverbindung (Edukt1) und 2,5 mmol 2-[(E)-3-(5-Trimethylstannanyl-furan-2-yl)-allyliden]-malononitril **B4** (Edukt2) wurden in 4 mL entsprechendem Lösemittel (Tab. 1) gelöst und die Lösung wurde entgast. Anschließend wurde 0,05 mmol Pd-Katalysator dazu gegeben und das Reaktionsgemisch wurde über Nacht erhitzt. Das Reaktionsgemisch wurde auf Raumtemperatur gebracht, dabei ausgefallener Niederschlag abfiltriert und mit Methanol nachgewaschen. Das Rohprodukt wurde aus entsprechendem Lösemittel (Tab. 1) umkristallisiert.

**Tabelle 1: Reaktionsbedingungen**

| **Nr.** | **Edukt 1** | **Edukt 2** | **Reaktionsbedingungen** | **Ausbeute (%)** | **Umkristallisiert aus** |
|---|---|---|---|---|---|
| 1 | A11 | B4 | Pd(PPh₃)₄/DMF (N,N-Dimethylformamid)/80°C | 75 | Toluol |
| 2 | A21 | B4 | Pd₂dba₃ / P(t-Bu)₃·HBF₄ / Toluol/110°C | 55 | Chlorbenzol |
| 3 | A20 | B4 | Pd(PPh₃)₄/Chlorbenzol/ 136°C | 38 | Chlorbenzol |
| 4 | A9 | B4 | Pd(PPh₃)₄ /Dioxan/80°C | 34 | Chlorbenzol |
| 5 | A12 | B4 | Pd(PPh₃)₄/Dioxan/80°C | 71 | Chlorbenzol |
| 8 | A6 | B4 | Pd(PPh₃)₄/Dioxan/80°C | 41 | Chlorbenzol |
| 10 | A7 | B4 | Pd(PPh₃)₄/Dioxan/80°C | 52 | Chlorbenzol |
| 12 | A3 | B4 | Pd(PPh₃)₄/DMF/80°C | 21 | Toluol |
| 20 | A24 | B4 | Pd(PPh₃)₄/DMF/80°C | 18 | Chlorbenzol |
| 18 | A23 | B4 | Pd(PPh₃)₄/DMF/80°C | 23 | Toluol |
| 19 | A5 | B4 | Pd(PPh₃)₄/DMF/80°C | 10 | Toluol |
| 25 | A22 | B4 | Pd(PPh₃)₄/Toluol/110°C | 57 | Chlorbenzol |
| 27 | A2 | B4 | Pd(PPh₃)₄/Dioxan/80°C | 27 | Chlorbenzol |
| 28 | A10 | B4 | P(PPh₃)₄/Dioxan/80°C | 35 | Chlorbenzol |
| 29 | A18 | B4 | Pd(PPh₃)₄/Dioxan/80°C | 66 | Chlorbenzol |
| 31 | A26 | B4 | Pd-PEPPSI-IPent/CsF/Dioxan/80°C | 13 | Chlorbenzol |
| 30 | A25 | B4 | Pd₂dba₃/P(t-Bu)₃/ Dioxan/80°C | 64 | Chlorbenzol |
| 33 | A28 | B4 | Pd2dba3/P(t-Bu)3 / Dioxan/80°C | 52 | Chlorbenzol |
| 35 | A30 | B4 | Pd2dba3/P(t-Bu)3 / Dioxan/80°C | 49 | Chlorbenzol |
| 36 | A31 | B4 | Pd2dba3/P(t-Bu)3 / Dioxan/80°C | 16 | Chlorbenzol |
| 38 | A35 | B4 | Pd2dba3/P(t-Bu)3 / Dioxan/80°C | 48 | Chlorbenzol |
| 39 | A34 | B4 | Pd2dba3/P(t-Bu)3 / Dioxan/80°C | 68 | Chlorbenzol |
| 40 | A36 | B4 | Pd2dba3/P(t-Bu)3 / Dioxan/80°C | 14 | Chlorbenzol |
| 41 | A37 | B4 | Pd2dba3/P(t-Bu)3 / Dioxan/80°C | 61 | Chlorbenzol |
| 42 | A33 | B4 | Pd2dba3/P(t-Bu)3 / Dioxan/80°C | 47 | Chlorbenzol |
| 47 | A39 | B4 | Pd(t-Bu)3/Dioxan/80°C | 70 | Chlorbenzol |

### b) Allgemeine Arbeitsvorschrift (AAV2)

1 mmol Distannylverbindung (Edukt1) und 2,5 mmol Brom-furan-2-yl-allyliden-malononitril **B2,** oder **B6,** oder **B10,** oder **B12,** oder **B14,** oder **B16** (Edukt2) wurden in 4 mL entsprechendem Lösemittel (Tab. 2) gelöst und die Lösung wurde entgast. Anschließend wurde 0,05 mmol Pd-Katalysator dazu gegeben und das Reaktionsgemisch wurde über Nacht erhitzt. Das Reaktionsgemisch wurde auf Raumtemperatur gebracht, dabei ausgefallener Niederschlag abfiltriert und mit Methanol nachgewaschen. Das Rohprodukt wurde aus entsprechendem Lösemittel (Tab. 2) umkristallisiert.

**Tabelle 2: Reaktionsbedingungen**

| **Nr.** | **Edukt 1** | **Edukt 2** | **Reaktionsbedingungen** | **Ausbeute %)** | **Umkristallisiert aus** |
|---|---|---|---|---|---|
| 6 | A4 | B16 | Pd(PPh₃)₄/DMF/80°C | 42 | Chlorbenzol |
| 7 | A1 | B16 | Pd(PPh₃)₄/DMF/80°C | 46 | Chlorbenzol |
| 9 | A8 | B2 | Pd(PPh₃)₄/DMF/80°C | 42 | Chlorbenzol |
| 11 | A14 | B2 | Pd(PPh₃)₄/DMF/80°C | 46 | Chlorbenzol |
| 13 | A4 | B14 | Pd(PPh₃)₄/DMF/80°C | 42 | Chlorbenzol |
| 14 | A19 | B2 | Pd(PPh₃)₄/DMF/80°C | 55 | Chlorbenzol |
| 15 | A1 | B14 | Pd(PPh₃)₄/DMF/80°C | 36 | Chlorbenzol |
| 16 | A1 | B6 | Pd(PPh₃)₄/DMF/80°C | 52 | Chlorbenzol |
| 17 | A13 | B2 | Pd(PPh₃)₄/DMF/80°C | 32 | Chlorbenzol |
| 22 | A1 | B10 | Pd(PPh₃)₄/DMF/80°C | 41 | Chlorbenzol |
| 23 | A1 | B2 | Pd(PPh3)4/DMF/80°C | 37 | Chlorbenzol |
| 24 | A4 | B2 | Pd(PPh₃)₄/DMF/80°C | 34 | Chlorbenzol |
| 26 | A1 | B12 | Pd(PPh₃)₄/DMF/80°C | 9 | Chlorbenzol |
| 32 | A27 | B2 | Pd(PPh₃)₄/1,4-Dioxan/100°C | 38 | Toluol |
| 37 | A32 | B6 | Pd(PPh₃)₄/1,4-Dioxan/80°C | 14 | Tetrahydrofuran |

### c) Allgemeine Arbeitsvorschrift (AAV3)

In einem mit Argon inertisierten Schlenkgefäß wurde 1 mmol Halogenverbindung (Edukt1) und 1.2 mmol 2-[3-(5-Trimethylstannanyl-furan-2-yl)-allyliden]-malononitril **B4** (Edukt2) in 3 mL Lösungsmittel (Tab. 3) gelöst. Die Lösung wurde entgast, anschließend 0.05 mmol Pd-Katalysator zugegeben und das Reaktionsgemisch über Nacht unter Rühren erhitzt. Das Reaktionsgemisch wurde auf Raumtemperatur abgekühlt, der entstandene Niederschlag abfiltriert und mit Methanol nachgewaschen. Das Rohprodukt wurde aus dem jeweiligen Lösungsmittel (Tab. 3) umkristallisiert.

**Tabelle 3: Reaktionsbedingungen**

| **Nr.** | **Edukt 3** | **Edukt 2** | **Reaktionsbedingungen** | **Ausbeute (%)** | **Umkristallisiert aus** |
|---|---|---|---|---|---|
| 21 | C2 | B4 | Pd(PPh₃)₄, Tetrahydrofuran, 65°C | 24 | Chlorbenzol |
| 34 | C8 | B4 | Pd(PPh₃)₄, 1,4-Dioxan, 100°C | 14 | Chlorbenzol |
| 40 | C6 | B4 | Pd2dba3 / P(t-Bu)3 / Dioxan/80°C | 72 | Chlorbenzol |

Alternativ können erfindungsgemäße Verbindungen auch über andere bekannte Alkylkupplungsreaktionen wie beispielsweise Suzuki- oder Neghishi-Reaktion erfolgen.

### Synthese der Edukte

Die Synthese der Edukte 1 (A), Edukte 2 (B) und Edukt 3 (C) erfolgt gemäß den folgenden Vorschriften:

### Synthese der Edukte A

Aus den ensprechend substituierten oder unsubstituierten Thiophenen lassen sich beispielsweise mit *tert*-BuLi und Trimethylzinnchlorid die ensprechenden Stannylverbindungen herstellen. So reagieren Zinn(IV)halogenide, bei Umsetzung mit metallorganischen Verbindungen, wie Grignard-Reagenzien zu den entsprechenden Zinnorganylen. Organozinnhalogenide werden meist aus Tetraorganozinnverbindungen durch elektrophile Substitution gewonnen.

Die Bromide können nach vielen literaturbekannten Reaktionen dargestellt werden (beispielsweise mit Kaliumbromat und Bromwasserstoff), analog ist auch die Umsetzung nach einer Vorschrift aus der entsprechenden Trimethylsilylverbindung mit N-Bromsuccinimid denkbar.

### 5,7-Bis-trimethylstannyl-2,3-dihydro-thieno[3,4-b][1,4]dioxin (A1)

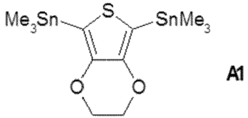

Verbindung **A1** wurde entsprechend J.Mater.Chem.1999, 2189 synthetisiert.

### 4, 6-Dibrom-1H, 3H-thieno [3, 4-c] furan (A2)

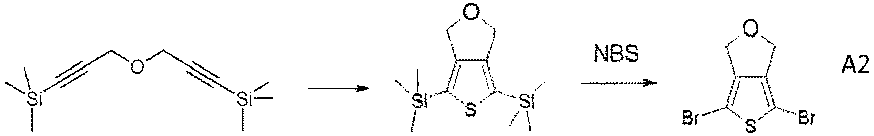

### 4,6-Bis-trimethylsilanyl-1H,3H-thieno[3,4-c]furan

1,92 g (8,27 mmol) Bis(3-trimethylsilyl-2-propinyl)ether und 5,70 g (19,8 mmol) Titan(IV)-isopropoxid wurden in 110 mL Diethylether unter Argonatmosphäre bei -70°C gelöst. Es wurden 19,8 mL (39,7 mmol) einer 2M Isopropylmagnesiumchlorid-Lösung in Diethylether hinzugetropft. Nach 15 min rühren bei -70°C wurde auf -50°C erwärmt und weitere 3,5 h gerührt. 2,12 g (66,2 mmol) Schwefel wurde portionsweise hinzugegeben und anschließend wurde die Reaktionsmischung innerhalb von 16 h auf RT (Raumtemperatur) erwärmt. Eine 1N HCl-Lösung (90 mL) wurde hinzugegeben und man extrahierte die wässrige Phase dreimal mit Hexan. Die vereinigten organischen Phasen wurden mit ges. Na₂C0₃-Lösung und ges. NaCl-Lösung gewaschen. Man trocknete über Na₂SO₄, filtrierte und entfernte die Lösemittel im Vakuum. Das Rohprodukt wurde chromatographisch gereinigt (SiO₂, R_{f} (DCM (Dichlormethan))= 0.88) und man erhielt 1,87 g Produkt (84%) als braunes Öl. ¹H-NMR (CDCl₃) : 4.83 ppm (s, 4H), 0.28 (s, 18H).

### 4,6-Dibrom-1H,3H-thieno[3,4-c]furan (A2)

1,87 g (6,92 mmol) 4,6-Bis-trimethylsilanyl-1*H*,3*H*-thieno[3,4-c]furan wurden in 18 mL DMF bei 0°C unter Argon-Atmosphäre gelöst. 2,77 g (15,2 mmol) NBS (N-Brom-succinimid) gelöst 16 mL DMF wurden hinzugegeben und für 1h bei 0°C gerührt. Es wurde auf RT erwärmt und für weitere 16 h gerührt. Die Reaktionsmischung wurde mit 50 mL Wasser versetzt und zweimal mit MTBE (Methyl-tert-butylether) extrahiert. Die vereinigten organischen Phasen wurden mit 5%iger LiCl-Lösung und ges. NaCl-Lösung gewaschen. Man trocknete über Na₂SO₄, filtrierte und entfernte die Lösemittel im Vakuum. Der Rückstand wurde chromatographisch gereinigt (SiO₂, PE:DCM (2:1), R_{f} = 0.54) und man erhielt 703 mg Produkt **A2** (36%) als gelbes Öl. ¹H-NMR(CDCl₃): 4.75 ppm (s, 4H).

### 2,5-Dibrom-3-thiomethylthiophen (A3)

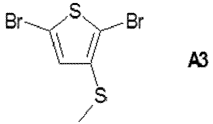

Verbindung **A3** wurde entsprechend M. Lanzi et al. Reactive & Functional Polymers 2014 (83) 33-41 hergestellt.

### 2,5-Bis-(trimethylstannyl)-3-methoxythiophen (A4)

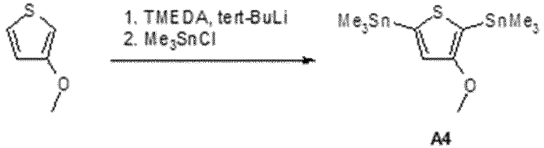

Eine Lösung aus 3-Methoxythiophen (342 mg, 3.00 mmol) und N,N,N',N'-Tetramethylethylendiamin (1.05 g, 9.00 mmol) in Hexan (50 mL) wurde bei -78 °C unter Argon-Atmosphäre tropfenweise mit *tert*-Butyllithium-Lösung (1.48 M in Hexan, 6.10 mL, 9.00 mmol) versetzt. Die Reaktionsmischung wurde 10 min bei -65 °C gerührt, auf Raumtemperatur erwärmt und weitere 4.5 h gerührt. Anschließend wurde die Reaktionslösung auf -65 °C abgekühlt, mit Trimethylzinnchlorid-Lösung (1M in Tetrahydrofuran, 9 mL, 9.00 mmol) versetzt, über Nacht im Kältebad auf Raumtemperatur erwärmt, auf Wasser (40 mL) gegossen und mit Hexan (3 x 30 mL) extrahiert. Die vereinigten organischen Phasen wurden mit gesättigter Ammoniumchlorid-Lösung (2 x 30 mL), Salzsäure (1M, 4 x 30 mL) und Wasser (30 mL) gewaschen, über Natriumsulfat getrocknet und filtriert. Das Abtrennen des Lösungsmittels unter vermindertem Druck lieferte rohes 3-Methoxy-2,5-bis-trimethylstannanylthiophene (1.18 g, 2.58 mmol, 86%) als farbloses viskoses Öl, welches ohne weitere Aufreinigung im nächsten Reaktionsschritt eingesetzt wurde. ¹H-NMR (CDCl₃) : 7.04 ppm (s, 1H), 3.83 (s, 3H), 0.35 (s, 9H), 0.33 (s, 9H).

### 2,5-Dibrom-3-(3,3,3-trifluor-propoxy)-thiophen (A5)

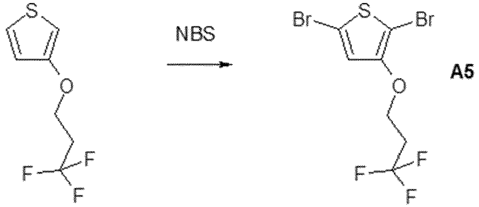

432 mg (2,20 mmol) 3-(3,3,3-Trifluor-propoxy)-thiophen (Synthese analog A18a) wurde in 5 mL trockenes DMF bei 0°C unter Argon gelöst. 1,03 g (5,72 mmol) NBS wurde in kleinen Portionen dazugegeben und 1h bei 0°C gerührt. Nachdem das Kältebad entfernt worden ist, wurde das Reaktionsgemisch 24h bei Raumtemperatur gerührt. Das Reaktionsgemisch wurde mit Wasser versetzt und zwei Mal mit 20 mL MTBE extrahiert. Die organische Phase wurde über Natriumsulfat getrocknet. Die Lösemittel wurden am Rotationsverdampfer entfernt und Rückstand am Silikagel in Ethylacetat/Petrolether 1/9 chromatographiert. Man erhielt 319 mg **A5.** EI-MS, m/z 353.89 [M]. ¹H-NMR (CDCl3) ppm: 6.77 (s, 1H), 4.22 (t, 2H), 2.61 (m, 2H).

### 2,5-Dibrom-3,4-dimethylthiophen (A6)

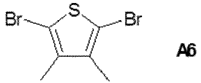

Verbindung **A6** wurde entsprechend Yasuyuki Kiya, Jay C. Henderson, Geoffrey R. Hutchison, Hector D. Abruña J. Mater. Chem. 2007, 17, 4366-4376 hergestellt.

### 2,5-Dibrom-3-methylthiophen (A7)

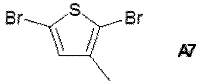

**A7** ist kommerziell erhältlich

### 3-Ethyl-2,5-bis-trimethylstannyl-thiophen (A8)

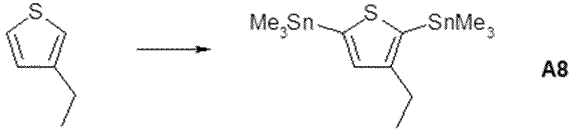

Zu einer Lösung aus 224mg (2.00 mmol) 3-Ethylthiophen in 10 mL trockenem n-Hexan und 0.85 mL TMEDA (N,N,N,N-Tetramethylethylendiamin), wurden 5.0 mL (8.00 mmol) 1.6 M *n-*Butyllithium in *n*-Hexan bei -78°C getropft. Nach 10 min. Rühren bei - 78°C wurde das Kältebad entfernt und die Reaktionsmischung 20 h bei R.T. gerührt. Die Suspension wurde erneut auf -78°C abgekühlt und 8.0 mL (8.00 mmol) einer 1.0M Trimethylstannylchloridlösung in THF (Tetrahydrofuran) zugetropft. Die Reaktionsmischung wurde 1h bei - 78°C und anschließend 3h bei R.T. gerührt. Nach Zugabe von 50 mL *n-*Hexan wurde mit Wasser hydrolysiert. Die organische Phase wurde drei Mal mit 50 mL Wasser gewaschen und über Natriumsulfat getrocknet. Nach dem Entfernen der Lösungsmittel wurde der Rückstand im Vakuum getrocknet. Das Rohprodukt (869 mg, 99%) wurde ohne weitere Reinigungsschritte in die nächste Stufe eingesetzt. ¹H-NMR (CDCl₃): 7.21 ppm (s, 1H), 2.71 (q, 2H), 1.24 (t, 3H), 0.37 (s, 9H), 0.35 (s, 9H) .

### 1,3-Dibrom-4,5,6,7-tetrahydro-benzo[c]thiophen (A9)

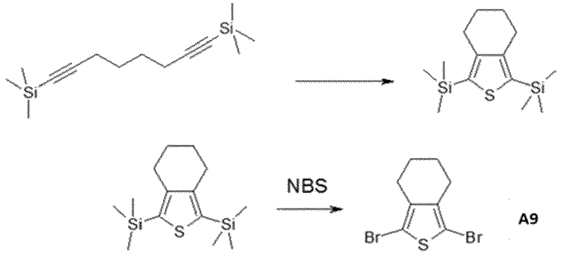

### 1,3-Bis-trimethylsilanyl-4,5,6,7-tetrahydro-benzo[c]thiophen

7,13 g (28,5 mmol) 1,8-Bis-trimethylsilanyl-octa-1,7-diin und 19,6 g (68,4 mmol) Titan(IV)-isopropoxid wurden in 380 mL Diethylether unter Argon-Atmosphäre bei -70°C gelöst. Man tropfte 68,4 mL (137 mmol) einer 2M Isopropylmagnesiumchlorid-Lösung in Diethylether hinzu. Nach 15 min rühren bei -70°C wurde auf -50°C erwärmt und weitere 3,5 h gerührt. 7,31 g (228 mmol) Schwefel wurde portionsweise hinzugegeben und anschließend wurde die Reaktionsmischung innerhalb von 16 h auf RT erwärmt. Eine 1N HCl-Lösung (300 mL) wurde hinzugegeben und man extrahierte die wässrige Phase dreimal mit Hexan. Die vereinigten organischen Phasen wurden mit ges. Na₂CO₃-Lösung und ges. NaCl-Lösung gewaschen. Man trocknete über Na₂SO₄, filtrierte und entfernte die Lösemittel im Vakuum. Der Rückstand wurde chromatographisch gereinigt (SiO₂, PE, R_{f} = 0.74) und man erhielt 5,20 g (18,4 mmol) Produkt (65%) als leicht gelbes Öl. ¹H-NMR (CDCl₃): 2.80 ppm (m, 4H), 1.76 (m, 4H), 0.31 (s, 18H).

### 1,3-Dibrom-4,5,6,7-tetrahydro-benzo[c]thiophen (A9)

4,35 g (15,4 mmol) 1,3-Bis-trimethylsilanyl-4,5,6,7-tetrahydrobenzo[c]thiophen wurden in 40 mL DMF bei 0°C unter Argon-Atmosphäre gelöst. 6,09 g (33,9 mmol) NBS gelöst 37 mL DMF wurden hinzugegeben und für 1h bei 0°C gerührt. Es wurde auf RT erwärmt und für weitere 16 h gerührt. Die Reaktionsmischung wurde mit 200 mL Wasser versetzt und zweimal mit MTBE extrahiert. Die vereinigten organischen Phasen wurden mit 2N NaOH-Lösung, 5%iger LiCl-Lösung und ges. NaCl-Lösung gewaschen. Man trocknete über Na₂SO₄, filtrierte und entfernte die Lösemittel im Vakuum. Der Rückstand wurde chromatographisch gereinigt (SiO₂, PE, R_{f} = 0.73) und man erhielt 4,31 g Produkt **A9** (79%) als leicht gelbes Öl. ¹H-NMR (CDCl₃): 2.50 ppm (m, 4H), 1.72 (m, 4H).

### 1,3-Dibrom-5,6-dihydro-4H-cyclopenta[c]thiophen (A10)

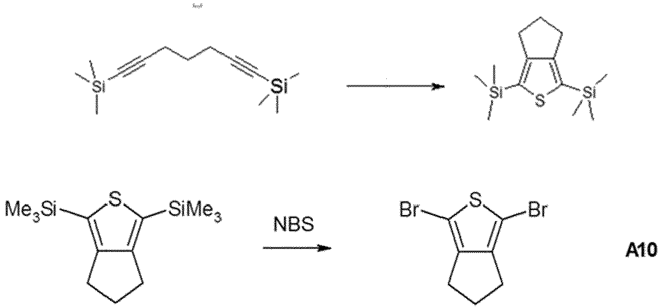

### 1,3-Bis-trimethylsilanyl-5,6-dihydro-4H-cyclopenta[c]thiophen (A10a)

2,37 g (10,0 mmol) 1,7-Bis-trimethylsilanyl-hepta-1,6-diin und 6,91 g (24,0 mmol) Titan(IV)-isopropoxid wurden in 130 mL Diethylether unter Argon-Atmosphäre bei -70°C gelöst. Man tropfte 24,0 mL (48,0 mmol) einer 2M Isopropylmagnesiumchlorid-Lösung in Diethylether hinzu. Nach 15 min rühren bei -70°C wurde auf -50°C erwärmt und weitere 3,5 h gerührt. 2,57 g (80,0 mmol) Schwefel wurden portionsweise hinzugegeben und anschließend wurde die Reaktionsmischung innerhalb von 16 h auf RT erwärmt. Eine 1N HCl-Lösung (105 mL) wurde hinzugegeben und man extrahierte die wässrige Phase dreimal mit Hexan. Die vereinigten organischen Phasen wurden mit ges. Na₂CO₃-Lösung und ges. NaCl-Lösung gewaschen. Man trocknete über Na₂SO₄, filtrierte und entfernte die Lösemittel im Vakuum. Das Rohprodukt wurde chromatographisch gereinigt (SiO₂, R_{f} (PE)= 0.63) und man erhielt 2,38 g Produkt (89%) als leicht gelbes Öl. ¹H-NMR (CDCl₃): 2.69 ppm (t, 4H), 2.43(m, 2H), 0.30 (s, 18H).

### 1,3-Dibrom-5,6-dihydro-4H-cyclopenta[c]thiophen (A10)

2,38 g (8,88 mmol) 1,3-Bis-trimethylsilanyl-5,6-dihydro-4*H-*cyclopenta[c]thiophen wurden in 22 mL DMF bei 0°C unter Argon-Atmosphäre gelöst. 3,19 g (17,8 mmol) NBS gelöst 20 mL DMF wurden hinzugegeben und für 1h bei 0°C gerührt. Es wurde auf RT erwärmt und für weitere 16 h gerührt. Die Reaktionsmischung wurde mit 50 mL Wasser versetzt und zweimal mit MTBE extrahiert. Die vereinigten organischen Phasen wurden mit 5%iger LiCl-Lösung und ges. NaCl-Lösung gewaschen. Man trocknete über Na₂SO₄, filtrierte und entfernte die Lösemittel im Vakuum. Der Rückstand wurde chromatographisch gereinigt (SiO₂, PE, R_{f} = 0.54) und man erhielt 1,25 g Produkt **A10** (50%) als farbloses Öl. ¹H-NMR (CDCl₃): 2.62 ppm (t, 4H), 2.37 (m, 2H).

### 2,5-Dibrom-3,4-bis-(3,3,3-trifluor-propoxy)-thiophen (A11)

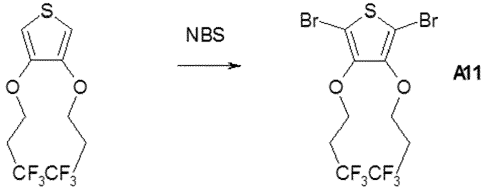

2,75 g (8,9 mmol) 3,4-Bis-(3,3,3-trifluor-propoxy)-thiophen (Synthese analog A18a) wurden in 33 mL trockenes DMF gelöst und auf 0°C abgekühlt. 3,21 g (17,8 mmol) NBS wurden in kleinen Portion dazu gegeben und das Reaktionsgemisch wurde über Nacht bei Raumtemperatur gerührt. Anschließend wurde das Reaktionsgemisch auf 100 mL Eiswasser gegeben. Die wässrige Phase wurde drei Mal mit 100 mL DCM extrahiert. Die organische Phase wurde drei Mal mit 100 mL ges. NaCl gewaschen und über Natriumsulfat getrocknet. Die Lösemittel wurden am Rotationsverdampfer entfernt und der Rückstand über chromatographisch gereinigt (SiO₂, DCM/Petrolether 1/9). Man erhält 2,20g **A11.** EI-MS, m/z 465.82 [M].

### 2,5-Dibromthiophen (A12)

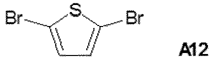

**A12** ist kommerziell erhältlich.

### 3,4-Dimethoxy-2,5-bis-trimethylstannanyl-thiophen (A13)

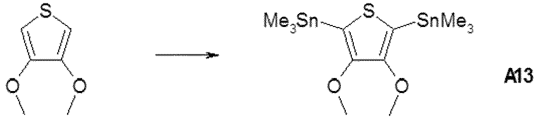

Zu einer Lösung aus 288 mg (2.00 mmol) 3,4-Dimethoxythiophen in 10mL trockenem n-Hexan und 0.85mL TMEDA wurden bei -78°C 4.05 mL (6.08 mmol) einer 1.5 M tert-Butyllithiumlösung in n-Pentan getropft. Die Mischung wurde 10min bei -78°C und weitere 3.5h bei R.T. gerührt. Die entstandene Suspension wurde erneut auf -78°C abgekühlt und 6.0 mL (6.00 mmol) einer 1.0 M Trimethylstannylchloridlösung in THF zugetropft. Das Reaktionsgemisch wurde 1h bei -78°C und weitere 16h bei R.T. gerührt. Anschließend wurden 50mL n-Hexan zugegeben und mit Wasser hydrolysiert. Die organische Phase wurde abgetrennt, drei Mal mit 50mL Wasser gewaschen und über Natriumsulfat getrocknet. Nach dem Entfernen der Lösungsmittel wurde der Rückstand im Vakuum getrocknet (681 mg, 72%). ¹H-NMR (acetone-d6): 3.77 ppm (s, 6H), 0.34 (s, 18H).

### 3,4-Diethyl-2,5-bis-trimethylstannyl-thiophen (A14)

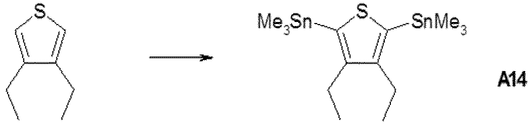

Zu einer Lösung aus 280 mg (2.00 mmol) 3,4-Diethylthiophen in 10mL trockenem *n*-Hexan und 0.85mL TMEDA wurden bei -78°C 2.4 mL (6.00 mmol) einer 2.5 M *n*-Butyllithiumlösung in n-Hexan getropft. Die Mischung wurde 10min bei -78°C und weitere 20h bei R.T. gerührt. Die entstandene Suspension wurde erneut auf -78°C abgekühlt und 6.0 mL (6.00 mmol) einer 1.0 M Trimethylstannylchloridlösung in THF zugetropft. Das Reaktionsgemisch wurde 1h bei -78°C und weitere 3h bei R.T. gerührt. Anschließend wurden 50mL *n*-Hexan zugegeben und mit Wasser hydrolysiert. Die organische Phase wurde abgetrennt, drei Mal mit 50mL Wasser gewaschen und über Natriumsulfat getrocknet. Nach dem Entfernen der Lösungsmittel wurde der Rückstand im Vakuum getrocknet (887 mg, 95%). ¹H-NMR (CDCl₃): 2.66 ppm (q, 4H), 1.17 (t, 6H), 0.36 (s, 18H).

### 6,8-Dibrom-3,3-difluor-3,4-dihydro-2H-thieno[3,4-b][1,4]dioxepin (A18)

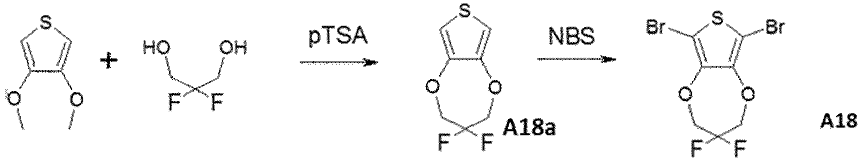

### 3,3-Difluor-3,4-dihydro-2H-thieno[3,4-b] [1,4]dioxepin (A18a)

In eine Soxhlet-Apparatur wurde in die Extraktionshülse Molsieb (4Ä) gefüllt und unter Argon-Atmosphäre erhitzte man 105 mg (0,55 mmol) p-Toluolsulfonsäure Monohydrat in 52 mL Toluol unter Rückfluss für 1h. Man ließ auf RT abkühlen und gab 787 mg (5,46 mmol) 3,4-Dimethoxythiophen und 1,28 g (10,9 mmol) 2,2-Difluorpropan-1,3-diol hinzu und erhitzte die Mischung für 16h unter Rückfluss. Die Reaktionsmischung wurde mit Wasser (50 mL) versetzt und man extrahierte die wässrige Phase einmal mit Toluol. Die vereinigten organischen Phasen wurden mit ges. NaCl-Lösung gewaschen. Man trocknete über Na₂SO₄, filtrierte und entfernte die Lösemittel im Vakuum. Das Rohprodukt wurde chromatographisch über Kieselgel gereinigt und man erhielt 237 mg Produkt (23%) als farbloses Öl. ¹H-NMR (CDCl₃) : 6.58 ppm (s, 2H), 4.30 (t, 4H).

### 6,8-Dibrom-3,3-difluor-3,4-dihydro-2H-thieno[3,4-b][1,4]dioxepin (A18)

236 mg (1,23 mmol) 3,3-Difluor-3,4-dihydro-2*H*-thieno[3,4-b][1,4]dioxepin wurden in 15 mL DMF bei 0°C unter Argonatmosphäre gelöst. 442 mg (2,46 mmol) NBS wurden bei 0°C hinzugegeben. Es wurde auf RT erwärmt und für weitere 16 h gerührt. Die Reaktionsmischung wurde mit 30 mL 5%ige LiCl-Lösung versetzt und dreimal mit DCM extrahiert. Die vereinigten organischen Phasen wurden mit 5%ige LiCl-Lösung und ges. NaCl-Lösung gewaschen. Man trocknete über Na₂SO₄, filtrierte und entfernte die Lösemittel im Vakuum. Der Rückstand wurde chromatographisch über Kieselgel gereinigt und man erhielt 300 mg Produkt **A18** (70%) als farbloses Öl. ¹H-NMR (CDCl₃): 4.38 ppm (t, 4H) .

### 6,8-Bis-trimethylstannyl-3,4-dihydro-2H-thieno[3,4-b] [1,4]dioxepin (A19)

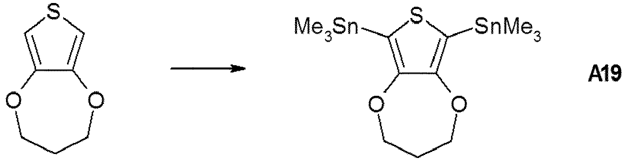

Zu einer Lösung von 312 mg (2,00 mmol) 3,4-dihydro-2*H*-thieno[3,4-b][1,4]dioxepin in 10 mL n-Hexan und 0,85 mL TMEDA wurden 5.33 mL (8.00 mmol) 1.5 M *tert*-Butyllithium-Lösung in n-Pentan bei -78°C hinzugetropft. Man rührte die Mischung 10 min bei -78°C und rührte sie anschließend 3.5 h bei RT. Die Suspension wurde auf -78°C gekühlt und 8.0 mL (8.00 mmol) 1.0M Trimethylstannylchlorid-Lösung in THF wurde hinzugegeben. Die Reaktionsmischung wurde über Nacht auf RT gebracht, mit 20 mL n-Hexan verdünnt und mit 1N HCl-Lösung und Wasser gewaschen. Man trocknete die org. Phase über Na₂SO₄, filtriert und destillierte die Lösemittel im Vakuum ab. Der Rückstand **A19** (548 mg, 57%) wurde ohne weitere Aufreinigung in der nächsten Stufe eingesetzt. ¹H-NMR (CDCl₃): 3.99 ppm (m, 4H), 2.15 (m, 2H), 0.32 (s, 18H) .

### 1,4-Dibrom-2,5-dimethoxybenzol (A20)

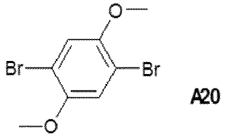

**A20** ist kommerziell erhältlich.

### 1,3-Dibrom-4,9-dioxa-2-thia-cyclopenta[b]naphthalin (A21)

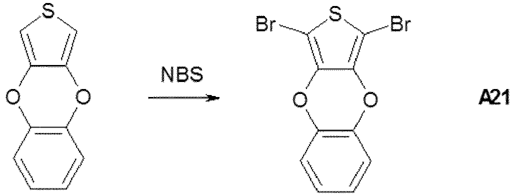

368 mg (1,93 mmol) 4,9-Dioxa-2-thia-cyclopenta[b]naphthalin (Roquet, J. Mater. Chem. 2004, 14, 1396-1400) wurden in 10 mL DMF bei 0°C unter Argon-Atmosphäre gelöst. 696 mg (3,87 mmol) NBS wurden hinzugegeben und für 1h bei 0°C gerührt. Es wurde auf RT erwärmt und für weitere 16 h gerührt. Die Reaktionsmischung wurde mit 30 mL Wasser versetzt und dreimal mit DCM extrahiert. Die vereinigten organischen Phasen wurden mit ges. Na₂S₂O₃-Lösung und ges. NaCl-Lösung gewaschen. Man trocknete über Na₂SO₄, filtrierte und entfernte die Lösemittel im Vakuum. Der Rückstand wurde aus Methanol umkristallisiert und man erhielt 523 mg Produkt **A21** (78%) als farblosen Feststoff. ¹H-NMR (CDCl₃): 6.98-7.05 ppm (m, 4H).

### 1,3-Dibrom-5,6-dihydro-4-oxa-2,7-dithia-inden (A22)

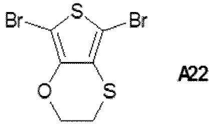

Verbindung **A22** wurde entsprechend Verbindung **A24** synthetisiert.

### 5,7-Dibrom-2-trifluormethyl-2,3-dihydro-thieno[3,4-b] [1,4]dioxin (A23)

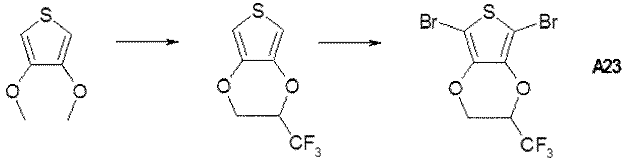

### 2-Trifluormethyl-2,3-dihydro-thieno[3,4-b] [1,4]dioxin

In eine Soxhlet-Apparatur wurde in die Extraktionshülse Molsieb (4Ä) gefüllt und unter Argon-Atmosphäre erhitzte man 74 mg (0,39 mmol) p-Toluolsulfonsäure Monohydrat in 36 mL Toluol unter Rückfluss für 1h. Man ließ auf RT abkühlen und gab 555 mg (3,85 mmol) 3,4-Dimethoxythiophen und 1,00 g (7,70 mmol) 3,3,3-Trifluorpropan-1,2-diol hinzu und erhitzte die Mischung für 16h unter Rückfluss. Die Reaktionsmischung wurde mit Wasser (50 mL) versetzt und man extrahierte die wässrige Phase einmal mit Toluol. Die vereinigten organischen Phasen wurden mit ges. NaCl-Lösung gewaschen. Man trocknete über Na₂SO₄, filtrierte und entfernte die Lösemittel im Vakuum. Das Rohprodukt wurde chromatographisch über Kieselgel gereinigt und man erhielt 322 mg Produkt (40%) als farblosen Feststoff. GC-MS (EI, 75eV) *m*/*z* 209.9 (M⁺, 100%).

### 5,7-Dibrom-2-trifluormethyl-2,3-dihydro-thieno[3,4-b] [1,4]dioxin (A23)

322 mg (1,53 mmol) 2-Trifluormethyl-2,3-dihydro-thieno[3,4-b][1,4]dioxin wurden in 15 mL THF bei -10°C unter Argon-Atmosphäre gelöst. 597 mg (3,32 mmol) NBS wurden hinzugegeben und für 1h bei - 7°C gerührt. Es wurde auf RT erwärmt und für weitere 4 h gerührt. Die Reaktionsmischung wurde mit 50 mL Wasser versetzt und dreimal mit MTBE extrahiert. Die vereinigten organischen Phasen wurden mit 2M NaOH-Lösung und ges. NaCl-Lösung gewaschen. Man trocknete über Na₂SO₄, filtrierte und entfernte die Lösemittel im Vakuum. Der Rückstand wurde chromatographisch über Kieselgel gereinigt und man erhielt 375 mg Produkt **A23** (67%) als leicht gelben Feststoff. ¹H-NMR (Aceton-d6): 5.15-5.21 ppm (m, 1H), 4.57-4.61 (m, 2H).

### 5,7-Dibrom-2,3-dihydro-thieno[3,4-b][1,4]dithiin (A24)

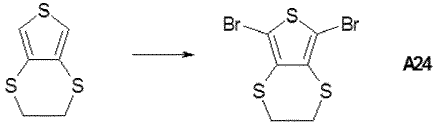

1,12 g (6,41 mmol) 2,3-Dihydro-thieno[3,4-b][1,4]dithiin (Wijsboom, Angew. Chem. Int. Ed. 2009 (48), 30, 5443-5447) wurden in 16 mL DMF bei 0°C unter Argon-Atmosphäre gelöst. 2,36 g (13,1 mmol) NBS wurden in 15 mL DMF gelöst und hinzugetropft bei 0°C. Es wurde auf RT erwärmt und für weitere 4 h gerührt. Die Reaktionsmischung wurde mit 100 mL 5%ige LiCl-Lösung versetzt und viermal mit MTBE extrahiert. Die vereinigten organischen Phasen wurden mit 5%ige LiCl-Lösung und ges. NaCl-Lösung gewaschen. Man trocknete über Na₂SO₄, filtrierte und entfernte die Lösemittel im Vakuum. Der Rückstand wurde chromatographisch über Kieselgel gereinigt und man erhielt 1.80g Produkt **A24** (85%) als farbloses Öl. GC-MS (EI, 75eV) *m*/*z* 331.8 (M⁺, 100%) .

### 2,7-Dibrom-9-propyl-9H-carbazol (A25)

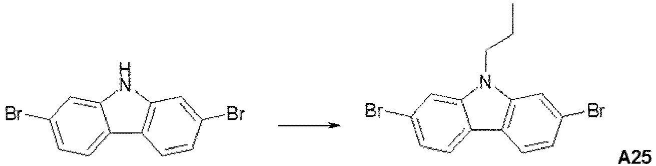

Eine Lösung aus Kaliumhydroxid (812 mg, 12.3 mmol) in trockenem Dimethylsulfoxid (25 mL) wurde mit 2,7-Dibrom-9H-carbazol (2.5 g, 7.69 mmol) versetzt und 15 min bei Raumtemperatur gerührt. Die Reaktionsmischung wurde mit Propylbromid (1.43 g, 11.5 mmol) versetzt, 16 h bei Raumtemperatur gerührt, anschließend auf Wasser (200 mL) gegossen und mit Diethylether (3 x 100 mL) extrahiert. Die vereinigten organischen Phasen wurden über Natriumsulfat getrocknet, filtriert und das Lösungsmittel unter vermindertem Druck entfernt. Säulenchromatographische Reinigung (Kieselgel, Petrolether) lieferte 2,7-Dibrom-9-propyl-9H-carbazol (2.80 g, 7.63 mmol, 99%) als farblosen kristallinen Feststoff. ¹H-NMR (CDCl₃): 7.89 ppm (2, 2H), 7.54 (d, 2H), 7.34 (dd, 2H), 4.17 (t, 2H), 1.89 (sext, 2H), 0.99 (t, 3H) .

### 4,8-Dibrom-benzo[1,2-b;4,5-b']difuran (A26)

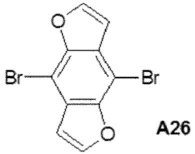

**A26** wurde entsprechend der literaturbekannten Vorschrift (Bian, J. Material. Chem.A 2015, 3, 1920) synthetisiert.

### 3-Ethyl-5,5'-bis-trimethylstannanyl-[2,2']bithiophenyl (A27)

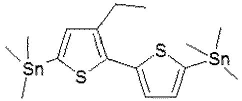

Die Verbindung wurde entsprechend **A19** hergestellt. ¹H-NMR (CDCl₃): 7.14 (d, 1H), 7.06(d, 1H), 6.95 (s, 1H), 2.74 (q, 2H), 1.19 (t, 3H), 0.37 (m, 18H).

### 2,7-Dibromo-9,9-dimethyl-9H-fluoren (A28)

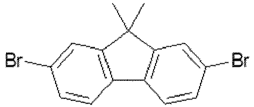

Verbindung **A28** ist kommerziell erhältlich.

### 2-Brom-3-methoxythiophen (A29)

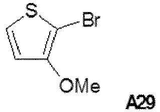

Die Synthese von **A29** erfolgte entsprechend der Literaturvorschrift von Canesi, Eleonora V. et al. Journal of the American Chemical Society, 134(46), 19070-19083; 2012

### 2,7-Dibrom-9-methyl-9H-carbazol (A30)

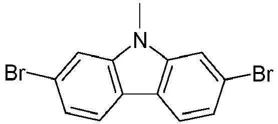

Eine Lösung aus Kaliumhydroxid (317 mg, 4.80 mmol) in trockenem Dimethylsulfoxid (12 mL) wurde vorgelegt, mit 2,7-Dibrom-9H-carbazol (975 mg, 3.00 mmol) versetzt und 15 min bei Raumtemperatur gerührt. Anschließend wurde Methyliodid (426 mg, 0.456 mL, 3.00 mmol) zugetropft und weitere 16 h bei Raumtemperatur gerührt. Die Reaktionsmischung wurde auf Wasser (100 mL) gegossen und mit Diethylether (3 x 50 mL) extrahiert. Die vereinigten organischen Phasen wurden über Natriumsulfat getrocknet, abfiltriert und das Lösungsmittel unter vermindertem Druck entfernt. Säulenchromatographie (Kieselgel, Petrolether) lieferte 2,7-Dibrom-9-methyl-9H-carbazol (902 mg, 2.66 mmol, 89%) als farblosen kristallinen Feststoff. ¹H-NMR (CDCl3): 7.89 ppm (dd, 2H), 7.55 (d, 2H), 7.35 (dd, 2H), 3.79 (s, 3H).

### 2,7-Dibrom-9-ethyl-9H-carbazol (A31)

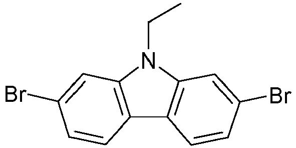

Eine Lösung aus Kaliumhydroxid (330 mg, 4.93 mmol) in trockenem Dimethylsulfoxid (11 mL) wurde vorgelegt, mit 2,7-Dibrom-9H-carbazol (1.00 g, 3.08 mmol) versetzt und 15 min bei Raumtemperatur gerührt. Anschließend wurde Ethylbromid (503 mg, 0.345 mL, 4.62 mmol) zugetropft und weitere 16 h bei Raumtemperatur gerührt. Die Reaktionsmischung wurde auf Wasser (80 mL) gegossen und mit Diethylether (2 x 100 mL) extrahiert. Die vereinigten organischen Phasen wurden über Natriumsulfat getrocknet, abfiltriert und das Lösungsmittel unter vermindertem Druck entfernt. Säulenchromatographie (Kieselgel, Petrolether) lieferte 2,7-Dibrom-9-ethyl-9H-carbazol (890 mg, 2.52 mmol, 82%) als farblosen kristallinen Feststoff. ¹H-NMR (CDCl₃) : 7.90 ppm (d, 2H), 7.55 (d, 2H), 7.35 (dd, 2H), 4.28 (q, 2H), 1.43 (t, 3H). GC-MS (EI) *m*/*z* 352.81 (M⁺, 100%).

### 9-Propyl-2,7-bis-trimethylstannanyl-9H-carbazol (A32)

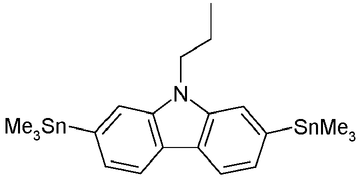

2,7-Dibrom-9-propyl-9H-carbazol (1.00 g, 2.72 mmol) wurde unter Argon in trockenem Tetrahydrofuran (50 mL) bei -78 °C vorgelegt, innerhalb von 15 min tropfenweise mit tert-Butyllithium (1.5 M in Pentan, 3.63 mL) versetzt und weitere 1.5 h bei -78 °C gerührt. Danach wurde das Reaktionsgemisch mit Trimethylstannylchlorid (1 M in Tetrahydrofuran, 5.72 mL) versetzt und über Nacht unter Rühren im Kältebad auf Raumtemperatur erwärmt. Anschließend wurde die Reaktionsmischung auf Wasser (200 mL) gegossen und mit Diethylether (3 x 100 mL) extrahiert. Die vereinigten organischen Phasen wurden mit gesättigter NaCl-Lösung (3 x 100 mL) gewaschen, über Natriumsulfat getrocknet, abfiltriert und das Lösungsmittel unter vermindertem Druck entfernt.

Umkristallisation aus Methanol/Ethanol (2:1) lieferte 9-Propyl-2,7-bis-trimethylstannanyl-9H-carbazol (780 mg, 1.46 mmol, 54%) als farblosen kristallinen Feststoff. ¹H-NMR (Aceton-D₆): 8.11 ppm (dd, 2H), 7.72-7.73 (m, 2H), 7.32 (dd, 2H), 4.44 (t, 2H), 1.87-1.97 (m, 2H), 0.94 (t, 3H), 0.35 (s, 18H).

### 3,6-Dibrom-9-propyl-9H-carbazol (A33)

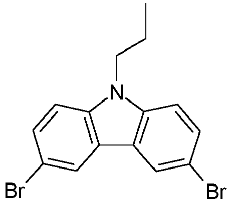

Eine Lösung aus Kaliumhydroxid (315 mg, 4.77 mmol) in trockenem Dimethylsulfoxid (11 mL) wurde vorgelegt, mit 3,6-Dibrom-9H-carbazol (1.00 g, 2.98 mmol) versetzt und 15 min bei Raumtemperatur gerührt. Anschließend wurde Ethylbromid (503 mg, 0.345 mL, 4.62 mmol) zugetropft und weitere 16 h bei Raumtemperatur gerührt. Die Reaktionsmischung wurde auf Wasser (80 mL) gegossen und mit Diethylether (2 x 100 mL) extrahiert. Die vereinigten organischen Phasen wurden über Natriumsulfat getrocknet, abfiltriert und das Lösungsmittel unter vermindertem Druck entfernt. Säulenchromatographie (Kieselgel, Petrolether) lieferte 2,7-Dibrom-9-ethyl-9H-carbazol (790 mg, 2.15 mmol, 72%) als farblosen kristallinen Feststoff. ¹H-NMR (Aceton-D₆): 8.37-8.39 ppm (m, 2H), 7.58-7.60 (m, 4H), 4.41 (t, 2H), 1.90 (m, 2H), 0.93 (t, 3H). GC-MS (EI) *m*/*z* 367.00 (M⁺, 83%) .

### 2,7-Dibrom-9-isobutyl-9H-carbazol (A34)

Die Synthese von **A34** erfolgt analog zur Synthese von **A31.**

### 1,5-Dibromo-2,4-dimethoxy-benzol (A35)

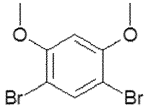

Verbindung **A35** ist kommerziell erhältlich.

### 4,7-Dibromo-benzo[1,2,5]triazol (A37)

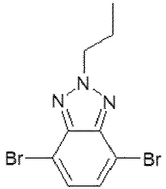

Verbindung A37 ist kommerziell erhältlich.

### 5,6-Dihydro-4H-cyclopenta[c]thiophen (A38a)

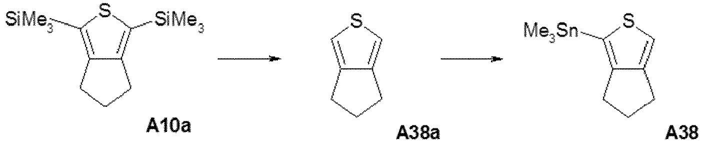

8.00 g (29.8 mmol) 1,3-Bis-trimethylsilanyl-5,6-dihydro-4H-cyclopenta[c]thiophen **(A10a)** wurden in 150 mL THF unter Argon-Atmosphäre gelöst. 89.4 mL (89.4 mmol, 1.0 M in THF) Tetrabutylammoniumfluorid-Lsg. wurde hinzugegeben und für 3h gerührt. Die Reaktionsmischung wurde mit 50 mL ges. Na2CO3-Lösung versetzt und dreimal mit n-Hexan extrahiert. Die vereinigten organischen Phasen wurden mit ges. Na2CO3-Lösung, Wasser und ges. NaCl-Lösung gewaschen. Man trocknete über Na2SO4, filtrierte und entfernte die Lösemittel im Vakuum. Der Rückstand wurde durch fraktionierte Destillation gereinigt (7 mbar, 50°C-55°C) und man erhielt 2.89 g Produkt 5,6-Dihydro-4H-cyclopenta[c]thiophen (78%) als farbloses Öl. 1H-NMR (CDCl₃): 6.76 ppm (s, 2H), 2.67 (t, 4H), 2.37 (m, 2H).

### 2-Trimethylstannyl-5,6-Dihydro-4H-cyclopenta[c]thiophen (A38)

2.66 g (21.4 mmol) 5,6-Dihydro-4H-cyclopenta[c]thiophen wurde in 75 mL THF gelöst und auf -78°C gekühlt. 9.0 mL (22.5 mmol) 2.5 M n-Butyllithium-Lösung in Hexan wurde hinzugetropft und für 2h bei -78°C gerührt. Es wurde 22.5 mL (22.5 mmol) einer 1.0 M Trimethylstannylchlorid-Lösung in THF hinzugegeben und über Nacht auf Raumtemperatur erwärmt. Man hydrolysierte mit 15 mL Wasser und extrahierte die wässrige Phase dreimal mit n-Hexan. Die vereinigten org. Phasen wurden mit ges. NaCl-Lösung gewaschen und über Na2SO4 getrocknet. Nach dem filtriert wurde, wurden die Lösemittel im Vakuum abdestilliert und der Rückstand wurde ohne weitere Reinigung in der nächsten Stufe verwendet. Man erhielt 5.40 g Produkt **A38** (88%) als gelbes Öl. 1H-NMR (CDCl₃): 7.04 ppm (m, 2H), 2.63-2.72 (m, 4H), 2.36-2.43 (m, 2H), 0.34 (s, 9H).

### 4,7-Dibrom-1H-Indol (A39)

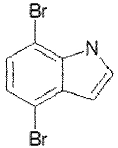

Die Synthese von A39 erfolgt nach der Literaturvorschrift: Dobbs, Adrian P.; Voyle, Martyn; Whittall, Neil, Syntlett, 1999, 10, 1594-1596.

### Synthese der Edukte 2 (B)

Endständigen Akzeptorgruppen können beispielsweise durch bekannte Methoden, wie z.B. Gattermann, Gattermann-Koch, Houben-Hoesch, Vilsmeier/ Vilsmeier-Haack, Friedel-Crafts-Acylierung oder nach Lithiierung durch eine Umsetzung mit einem Säurederivat oder Carbonylierungsreagenz synthetisiert werden.

Weitere Akzeptorgruppen sind durch Umfunktionalisierung der zuvor beschriebenen Carbonylfunktion C(O)R beispielsweise durch Knoevenagel-Kondensation, wie für das Edukt **B4** gezeigt, realisierbar.

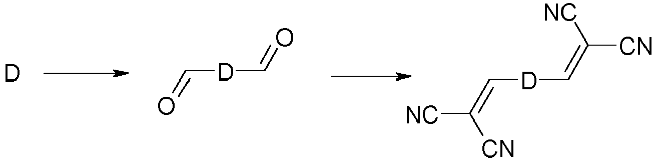

Die Einführung der Akzeptorendgruppen kann beispielsweise mit BuLi und Tetracyanoethylen erfolgen (Cai et al, J. Phys. Chem. B 2006, 110, 14590).

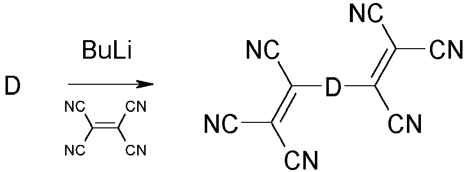

Alternativ kann die Umsetzung auch ohne BuLi in DMF durchgeführt werden (Pappenfus et. al, Org. Lett. 2008, 10, 8, 1553).

### B1, B5, B9, B11

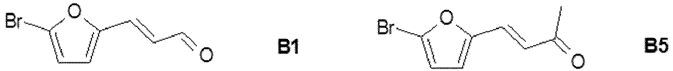

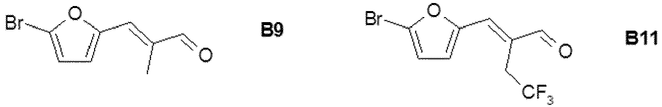

Die Synthese von **B1, B5, B9** und **B11** erfolgt gemäß der Literatur von I. I. Popov, Z. N. Nazarova, A. P. Chumak, Chem. Heterocycl. Compd., 1978, 14, (3), 253-255.

50 mmol 5-Brom-2-furfural (Edukt1) wurde in 100 mL 6%ige NaOH-Lösung suspendiert. Man tropfte Carbonylverbindung (Edukt2) in 15 mL Wasser zum Reaktionsgemisch bei 0°C. Es wurde 1h bei 0°C nachgerührt. Der Niederschlag wurde abfiltriert, mit Wasser gewaschen und getrocknet. Das Rohprodukt wurde chromatographisch über Kieselgel gereinigt.

**Tabelle 4: Synthese**

| **Name** | **Edukt2** | **Ausbeute, %** | **¹H-NMR** |
|---|---|---|---|
| B1 | Acetaldehyd | 74 | Aceton-d6: 9.64 ppm (d, 1H), 7.44 (d, 1H), 7.04 (d, 1H), 6.73 (d, 1H), 6.47 (dd, 1H) . |
| B5 | Aceton | 55 | CDCl₃: 7.17 ppm (d, 1H), 6.63 (d, 1H), 6.61 (d, 1H), 6.43 (d, 1H), 2.32 (s, 3H). |
| B9 | Propionaldehyd | 98 | Aceton-d6: 9.52 ppm (s, 1H), 7.18 (s, 1H), 7.00 (s, 1H), 6.75 (s, 1H), 2.03 (d, 3H) . |
| B11 | 4,4,4-Trifluorbutanal | 46 | Aceton-d6: 9.60 ppm (s, 1H), 7.53 (s, 1H), 7.19 (s, 1H), 6.82 (s, 1H), 3.65 (q, 2H) . |

### (E)-3-(5-Brom-furan-2-yl)-allyliden]-malononitril (B2)

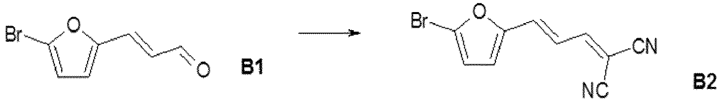

36,7 mmol (E)-3-(5-Brom-furan-2-yl)-propenal und 44,0 mmol Malonitril wurden in 50 mL Ethanol gelöst. 3,7 mmol β-Alanin wurde dazu gegeben und das Reaktionsgemisch wurde 24 h bei Raumtemperatur gerührt. Der ausgefallene Niederschlag wurde kurz zum Sieden erhitzt und anschließend im Eisbad abgekühlt. Der auskristallisierte Feststoff wurde abfiltriert und mit wenig Ethanol nachgewaschen. Nach dem Trocknen im Exsikkator wurden 3,49 g [(E)-3-(5-Brom-furan-2-yl)-allyliden]-malononitril **B2** (38% Ausbeute) isoliert. EI m/z: 250[M], 169, 141, 114.

### (E)-3-(5-Trimethylstannyl-furan-2-yl)-propenal (B3)

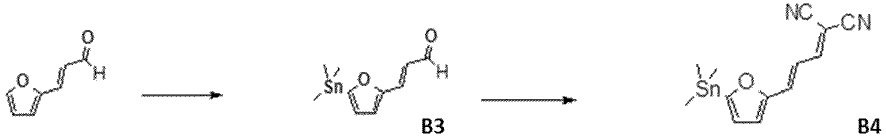

Zu einer Lösung aus 3.06 g (29.9mmol) 1-Methylpiperazin in 82mL wasserfreiem THF wurden unter Argon-Atmosphäre bei -78°C 12mL (30mmol) n-Butyllithiumlösung (2.5M in Hexan) zugetropft. Nach 15 min Rühren wurden 3.15g (25.0mmol) trans-3-(2-Furyl)acrolein zugetropft. Nach weiteren 15 min Rühren wurden 3.95g (33.7mmol) N,N,N',N'-Tetramethylethylendiamin zugetropft. Nach 15 min Rühren wurden 13.4mL (33.5mmol) n-Butyllithiumlösung (2.5M in Hexan) zugetropft. Die Reaktionsmischung wurde 3h bei -20°C gerührt und anschließend erneut auf -78°C abgekühlt. Bei dieser Temperatur wurden 29.9mL (29.9mmol) einer 1M Lösung von Trimethylzinnchlorid in THF zugegeben und die Mischung anschließend 16h bei R.T. gerührt. Anschließend wurden 100mL Wasser zugegeben, die organische Phase abgetrennt, die wässrige Phase dreimal mit MTBE extrahiert und die vereinigten organischen Phasen mit je 80mL einer 1M Salzsäure, gesättigter Ammoniumchloridlösung und Brine gewaschen. Nach dem Trocknen über Natriumsulfat wurden die Lösungsmittel abdestilliert und der Rückstand chromatographisch gereinigt (SiO2, Petrolether/MTBE 5/1). Ausbeute 5.52g (76%). ¹H-NMR (400 MHz) in Aceton-d6: 0.38 (s, 9H), 6.48 (dd, 1H), 6.84 (d, 1H), 6.97(d, 1H), 7.51(d, 1H), 9.63(d, 1H).

### 2-[(E)-3-(5-Trimethylstannanyl-furan-2-yl)-allyliden]-malononitril (B4)

Unter Argon-Atmosphäre wurden 9.52g (33.4mmol) **B3** und 2.23g (33.4mmol) Malodinitril in 19mL Ethanol gelöst. 152mg (1.67mmol) Beta-Alanin wurden hinzugegeben und die Mischung 4h bei R.T. gerührt. Anschließend wurde auf Rückflusstemperatur erhitzt und langsam unter Rühren auf 0°C abgekühlt. Der Niederschlag wurde abfiltriert, mit 2mL Ethanol gewaschen und im Vakuum getrocknet: 9.10g (82%) oranger kristalliner Feststoff. ¹H-NMR (400 MHz) in Aceton-d6: 0.41 (s, 9H), 6.90 (d, 1H), 7.07 (m, 2H), 7.46 (d, 1H), 8.01(d, 1H)

### 2-[(E)-3-(5-Brom-furan-2-yl)-1-methyl-allyliden]-malononitril (B6)

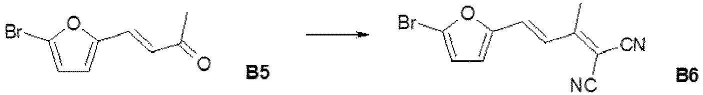

1.61 g (7.47 mmol) (E)-4-(5-Brom-furan-2-yl)-but-3-en-2-on (**B5**) und 2.47 g (37.4 mmol) Malononitril wurden in 60 mL 1,2-Dichlorethan unter Argon-Atmosphäre gelöst. 4.29 g (14.9 mmol) Titan(IV)-isopropylat wurden hinzugegeben und die Reaktionsmischung wurde auf 110°C für 20h erhitzt. Man ließ auf RT abkühlen und gab 100 mL 1N HCL-Lösung hinzu. Es wurde 1h bei RT gerührt, anschließend wurden die Phasen getrennt und die wässrige Phase wurde mit DCM extrahiert. Die vereinigten org. Phasen wurden mit 1N HCl- Lösung und ges. NaHCO₃-Lösung gewaschen, über Na₂SO₄ getrocknet und filtriert. Die Lösemittel wurden im Vakuum abdestilliert und der Rückstand chromatographisch über Kieselgel (RF (PE:DCM (1:2)) = 0,30). Man erhielt 1,08 g Produkt **B6** (55%) als orangen Feststoff. ¹H-NMR (Aceton-d6): 7.46 ppm (d, 1H), 7.17 (d, 1H), 7.03 (d, 1H), 6.76 (d, 1H), 2.48 (s, 3H).

### 5-Trimethylstannyl-furan-2-carbaldehyd (B7)

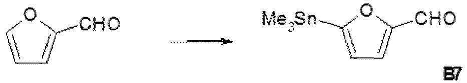

Zu einer Lösung aus 6.13 g (60.0 mmol) 1-Methylpiperazin in 160mL wasserfreiem THF wurden unter Argonatmosphäre bei -78°C 22.4 mL (56.0 mmol) n-Butyllithiumlösung (2.5 M in Hexan) zu getropft. Nach 15 min Rühren wurden 4.85g (50.0 mmol) 2-Furaldehyd zu getropft. Nach weiteren 15 min Rühren wurden 7.92g (67.5 mmol) N,N,N',N'-Tetramethylethylendiamin zugetropft. Nach 15 min Rühren wurden 24.0 mL (60.0 mmol) n-Butyllithiumlösung (2.5 M in Hexan) zu getropft. Die Reaktionsmischung wurde 3h bei -20°C gerührt und anschließend erneut auf -78°C abgekühlt. Bei dieser Temperatur wurden 60.0 mL (60.0 mmol) einer 1.0 M Lösung von Trimethylzinnchlorid in THF zugegeben und die Mischung anschließend 16h bei RT gerührt. Anschließend wurden 100mL Wasser zugegeben, die organische Phase abgetrennt, die wässrige Phase dreimal mit MTBE extrahiert und die vereinigten organischen Phasen mit je 80 mL einer 1 M Salzsäure und ges. Natriumchloridlösung gewaschen. Nach dem Trocknen über Natriumsulfat wurden die Lösungsmittel abdestilliert und der Rückstand chromatographisch gereinigt (SiO2, PE/MTBE 4/1). Ausbeute 8.69g (67%) als gelbes Öl. 1H-NMR (400 MHz) in Aceton-d6: 9.67 ppm (s, 1H), 7.39 (d, 1H), 6.90 (d, 1H), 0.40 (s, 9H) .

### (E)-4-(5-Trimethylstannyl-furan-2-yl)-but-3-en-2-on (B8)

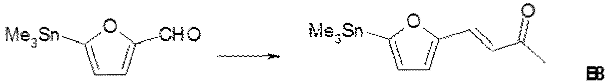

Zu einer Lösung von 8.69 g (33.5 mmol) 5-Trimethylstannyl-furan-2-carbaldehyd (**B7**) in 67 mL THF wurden, unter Argonatmosphäre, 11.3 g (35.2 mmol) 1-(Triphenylphosphoranyliden)-2-propanon gegeben. Die Reaktionsmischung wurde für 96h auf 50°C erhitzt. Man destillierte das Lösemittel ab und reinigte den Rückstand chromatographisch (SiO2, PE/EA 3/1). Ausbeute 9.14 g (91%) als gelbes Öl. 1H-NMR (400 MHz) in Aceton-d6: 7.42 ppm (d, 1H), 6.85 (d, 1H), 6.80 (d, 1H), 6.54 (d, 1H), 2.28 (s, 3H), 0.34 (s, 9H).

### 2-[(E)-3-(5-Brom-furan-2-yl)-2-methyl-allyliden]-malononitril (B10)

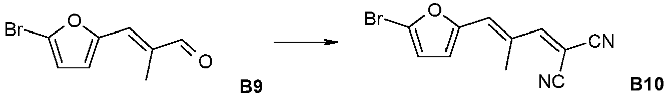

**B10 (wie B2):** Ausbeute 81%, ¹H-NMR in d6-Aceton, ppm: 7,82 (s, 1H), 7,13 (s, 1H), 7,09 (d, 1H), 6,81 (d, 1H), 2,48 (s, 3H).

### 2-{2-[1-(5-Brom-furan-2-yl)-meth-(E)-ylidene]-4,4,4-trifluorbutylidene}-malononitril (B12)

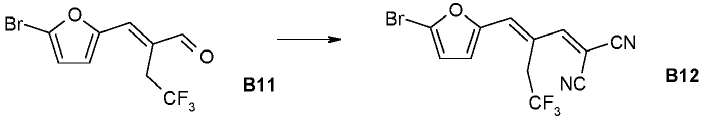

Die Synthese von **B12** erfolgt in zu **B2** analoger Weise. Ausbeute 58%, ¹H-NMR in d6-Aceton, ppm: 7,93 (s, 1H), 7,47 (s, 1H), 7,24 (d, 1H), 6,87 (d, 1H), 4,10 (qa, 2H).

### 3-(5-Brom-furan-2-yl)-cyclohex-2-enon (B13)

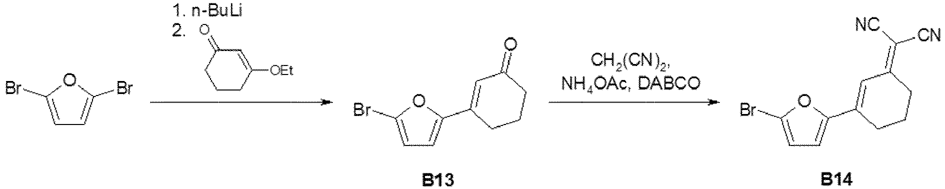

Unter Argon-Atmosphäre wurde zu einer Lösung aus 2.00g 2,5-Dibromfuran (8.85mmol) in 25mL Diethylether bei -65°C unter Rühren 5.53mL n-Butyllithium (1.6M in Hexan) innerhalb von 15min zugetropft. Nach weiteren 15min wurden 1.86g 3-Ethoxy-2-cyclohexen-1-on (13.3 mmol) zugegeben und die Mischung über Nacht auf R.T. erwärmt. Die Mischung wurde auf 150mL Brine gegeben und mit 3 x 100mL Dichlormethan extrahiert. Die vereinigten organischen Extrakte wurden mit 2M Salzsäure gewaschen, über Natriumsulfat getrocknet und die Lösungsmittel im Vakuum entfernt. Nach säulenchromathographischer Reinigung (SiO₂, Dichlormethan) wurde **B13** als gelber, kristalliner Feststoff erhalten (1.08g, 4.48mmol, 51%). ¹H-NMR (CDCl₃): 6.68 ppm (d, 1H), 6.44-6.43 (m, 2H), 2.60 (td, 2H), 2.46 (t, 2H), 2.14-2.07 (m, 2H).

### 2-[3-(5-Brom-furan-2-yl)-cyclohex-2-enyliden]-malononitril (B14)

Unter Argonatmosphäre wurden 1.68g Ammoniumacetat (21.8mmol) zu einer Lösung aus 1.74 g **B13** (7.14mmol) und 1.42g Malononitril (21.5mmol) in Dichlorethan gegeben. Die Mischung wurde 2h refluxiert, dann 20mg 1,4-diazabicyclo[2.2.2]octane (0.178mmol) zugegeben und anschließend weitere 16h refluxiert. Die Reaktionsmischung wurde auf 100mL Wasser gegeben und mit 3 x 50mL Dichlormethan extrahiert. Die vereinigten organischen Extrakte wurden mit 100mL Wasser gewaschen, über Natriumsulfat getrocknet und die Lösungsmittel im Vakuum entfernt. Nach säulenchromathographischer Reinigung (SiO₂, Hexan) wurde **B14** als oranger, kristalliner Feststoff erhalten (1.15 g, 3.98 mmol, 91%). ¹H-NMR (CDCl₃) : 7.19 ppm (s, 1H), 6.79 (d, 1H), 6.49 (d, 1H), 2.80 (t, 2H), 2.64-2.61 (m, 2H), 2.00-1.94 (m, 2H).

### 3-(5-Brom-furan-2-yl)-2-methyl-cyclopent-2-enon (B15)

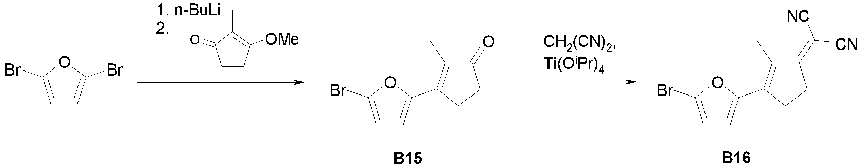

Unter Argonatmosphäre wurden zu einer Lösung aus 3.46g 2,5-Dibromfuran (15mmol) in 45mL Diethylether bei -65°C unter Rühren 6.00mL n-Butyllithium (2.5M in Hexan, 15mmol) innerhalb von 30min zugetropft. Nach weiteren 15min wurden 2.94g 3-Ethoxy-2-methyl-2-cyclopenten-1-on (21.0mmol) gelöst in 15mL Diethylether zugegeben und die Mischung für 1.5h bei -65°C gerührt und anschließend über Nacht auf R.T. erwärmt. Nach der Zugabe von 150mL Dichlormethan wurde die Mischung auf 300mL 1M Salzsäure gegeben. Die organische Phase wurde abgetrennt und die wässrige Phase einmal mit 100mL Dichlormethan extrahiert. Die vereinigten organischen Phasen wurden mit 2M Salzsäure (150mL) und Wasser (100mL) gewaschen, über Natriumsulfat getrocknet und die Lösungsmittel im Vakuum entfernt. Nach säulenchromathographischer Reinigung (SiO₂, Dichlormethan/Hexan) wurde **B15** als gelber, kristalliner Feststoff erhalten (2.10g, 8.71mmol, 58%). ¹H-NMR (CDCl₃) : 6.75 ppm (d, 1H), 6.50 (d, 1H), 2.86-2.82 (m, 2H), 2.52-2.49 (m, 2H), 2.02 (t, 3H).

### 2-[3-(5-Brom-furan-2-yl)-2-methyl-cyclopent-2-enyliden]-malononitril (B16)

Eine Lösung aus 1.30g 3-(5-Bromfuran-2-yl)-2-methylcyclopent-2-enon (**B15**) (5.39mmol) und 3.60g Malononitril (53.9 mmol) in 1,2-Dichlorethan wurde unter Argonatmosphäre mit 3.09g Tetraisopropylorthotitanat (10.8 mmol) versetzt und 3d unter Rückfluss gerührt. Die Reaktionsmischung wurde auf Salzsäure (1M, 200mL) gegossen, 30 min heftig gerührt und mit Dichlormethan (3 x 100 mL) extrahiert. Die vereinigten organischen Phasen wurden mit Wasser (100mL) gewaschen, über Natriumsulfat getrocknet, filtriert und das Lösungsmittel unter vermindertem Druck entfernt. Säulenchromatographische Reinigung (Kieselgel, Dichlormethan) lieferte **B16** (1.37 mg, 4.75 mmol, 88%) als orangefarbenen kristallinen Feststoff. ¹H-NMR (CDCl₃): 6.82 ppm (d, 1H), 6.55 (d, 1H), 3.09-3.06 (m, 2H), 3.00-2.96 (m, 2H), 2.40 (t, 3H).

### 2-(5-Trimethylstannyl-furan-2-ylmethylen)-malononitril (B17)

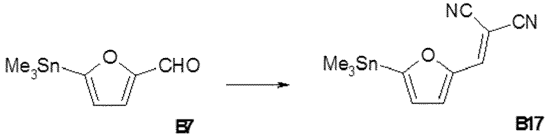

Die Synthese von **B17** ausgehend von **B7** erfolgt analog zu **B4**

### Synthese des Eduktes C2

Die für die einfache Stille-Kupplung notwendigen Edukte können, wie bereits beschrieben, aus der elektronenreichen Stannylverbindung und dem elektronenarmen Bromid hergestellt werden. Analog bieten sich dafür viele verschiedene literaturbekannte Möglichkeiten. Die Darstellung des Edukts **C1** wurde hier unter Stille-Kopplungsreaktionen realisiert. Mit N-Jod-Succinimid konnte **C1** als Ausgang für eine erneute Stille-Kopplungsreaktion zu **C2** umgesetzt werden.

### 2-[5-(2,3-Dihydro-thieno[3,4-b] [1,4]dioxin-5-yl)-furan-2-ylmethylen]-malononitril (C1)

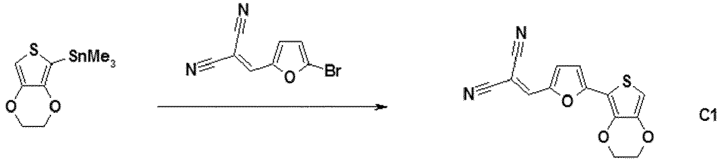

1,49 g (4 mmol) (2,3-Dihydro-thieno[3,4-b][1,4]dioxin-5-yl)-trimethyl-stannan und 0,89 g (4 mmol) **C4** wurden in 20 mL trocknem Toluol gelöst und das Reaktionsgemisch wurde entgast. Anschließend wurden 140 mg (0,12 mmol) Tetrakis(triphenylphosphin)-palladium(0) hinzugegeben und das Reaktionsgemisch über Nacht zum Sieden erhitzt. Das Reaktionsgemisch wurde auf Raumtemperatur gebracht und mit 300 mL Dichlormethan versetzt. Die organische Phase wurde abgetrennt, drei Mal mit ges. NaCl-Lösung gewaschen und über Natriumsulfat getrocknet. Das Lösungsmittel wurde am Rotationsverdampfer entfernt. Der Rückstand wurde über Silikagel in Petrolether/DCM = 1/3 chromatographiert (Rf 0,1). Man erhält 982 mg **C1.** ¹H-NMR (CDCl3) ppm: 7,18 (s, 2H), 6,81 (d, 1H), 6,47 (d, 1H), 4,30 (m, 2H), 4,21 (m, 2H) .

### 2-[5-(7-Iod-2,3-dihydro-thieno[3,4-b] [1,4]dioxin-5-yl)-furan-2-ylmethylen]-malononitril (C2)

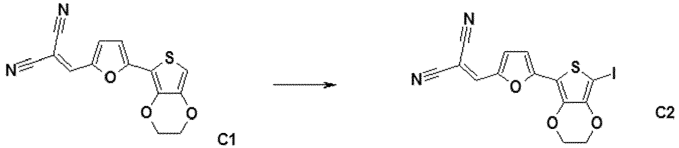

301 mg (1,06 mmol) **C1** wurden in 6 mL trockenem DMF gelöst und unter Argon auf 0°C abgekühlt. 241 mg (1,06 mmol) N-Jodsuccinimid wurden portionsweise zugegeben und anschließend über Nacht bei Raumtemperatur gerührt. Das Reaktionsgemisch wurde mit Eiswasser versetzt und unter Rühren auf Raumtemperatur gebracht. Die wässrige Mischung wurde drei Mal mit 50 mL Dichlormethan extrahiert. Die organische Phase wurde zwei Mal mit 50 mL ges. NaCl-Lösung und einmal mit 50 mL 5% LiCl-Lösung gewaschen und über Natriumsulfat getrocknet. Das Lösungsmittel wurde am Rotationsverdampfer entfernt, der Rückstand über Silikagel mit Dichlormethan chromatographiert (Rf 0,58). Man erhält 413 mg **C2.** ¹H-NMR (CDCl3) ppm: 7,35 (s, 1H), 6,55 (d, 2H), 4,32 (m, 4H).

### 2-(5-Brom-thiophen-2-ylmethylen)-malononitril (C3)

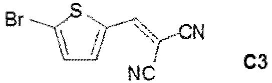

### 2-(5-Brom-furan-2-ylmethylen)-malononitril (C4)

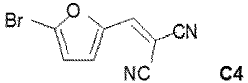

Verbindungen **C3** und **C4** werden entsprechend der literaturbeschriebenen Synthese (Qi et al., J. Mat. Chem. 2008, 18, 1131) hergestellt.

### 2-[5-(5,6-Dihydro-4H-cyclopenta[c]thiophen-1-yl)-furan-2-ylmethylen]-malononitril (C5)

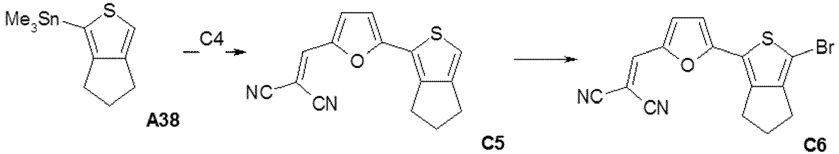

2.10 g (7.32 mmol) 2-Trimethylstannyl-5,6-Dihydro-4H-cyclopenta[c]thiophen (**A38**) und 1.63 g (7.32 mmol) 2-[(5-Bromfuran-2-yl)methylen]malononitril (**C4**) wuden in 28 mL 1,4-Dioxan gelöst. Man gab 37 mg (73 pmol) Bis-(tri-tert-butylphosphin)-palladium(0) hinzu und erhitzte auf 80°C für 16h. Nach dem Abkühlen auf Raumtemperatur wurde die Reaktionsmischung mit Wasser versetzt und dreimal mit Ethylacetat extrahiert. Die vereinigten org. Phasen wurden mit Wasser und ges. NaCl-Lösung gewaschen. Man trocknete über Na2SO4, filtrierte und entfernte die Lösemittel im Vakuum. Der Rückstand wurde chromatographisch gereinigt (SiO2, Rf (Hex:DCM (1:1)) = 0.19) und man erhielt 1.50 g Produkt C5 (68%) roter Feststoff. 1H-NMR (Aceton-D6): 7.91 ppm (s, 1H), 7.54 (d, 1H), 7.19 (1s, 1H), 6.91 (d, 1H), 2.97 (t, 2H), 2.72 (t, 2H), 2.44-2.51 (m, 2H).

### 2-[5-(3-Brom-5,6-dihydro-4H-cyclopenta[c]thiophen-1-yl)-furan-2-ylmethylen]-malononitril (C6)

266 mg (1,00 mmol) 2-[5-(5,6-Dihydro-4H-cyclopenta[c]thiophen-1-yl)-furan-2-ylmethylen]-malononitril(**C5**) wurden in 10 mL THF bei 0°C unter Argonatmosphäre gelöst. 178 mg (1,00 mmol) NBS wurde portionsweise hinzugegeben und für 15 min bei 0°C gerührt. Es wurde auf RT erwärmt und für weitere 16 h gerührt. Die Reaktionsmischung wurde auf 0°C gekühlt und der Niederschlag abfiltriert. Man trocknete den Niederschlag im Vakuum und erhielt 244 mg Produkt C6 (71%) als roten Feststoff. 1H-NMR (Aceton-D6): 7.97 ppm (s, 1H), 7.54 (d, 1H), 6.91 (d, 1H), 3.05 (t, 2H), 2.65 (t, 2H), 2.47-2.55 (m, 2H).

### Synthese von C7 und C8

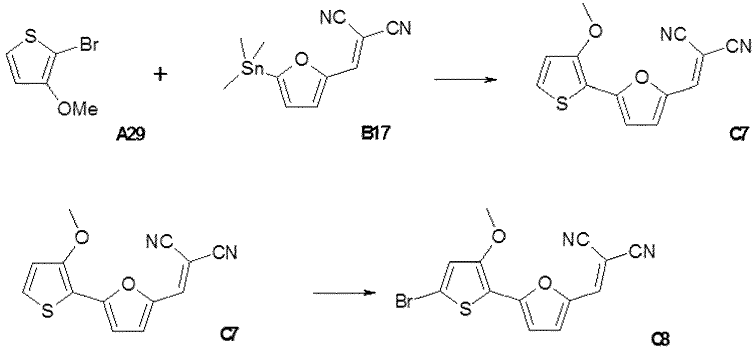

Die Synthese von **C7** und **C8** erfolgte in Analogie zu **C5** und **C6.**

### Eigenschaften der Verbindungen

Im Folgenden werden einige konkrete Ausführungsbeispiele für die erfindungsgemäße und nicht-erfindungsgemäße Verbindung sowie deren optischen Eigenschaften angegeben:

**Tabelle 5: Übersicht Ausführungsbeispiele und optische Eigenschaften ^{a} onset DSC ^{b} in Dichlormethan wenn nicht anders vermerkt ^{c} 30 nm im Vakuum aufgedampfter Film**

| **Nr.** | **Struktur** | **λmax (LM)/nm^{b}** | **HWB Film*/eV^{c}** |
|---|---|---|---|
| 1 | 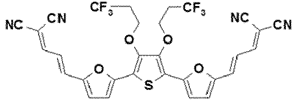 | 435 | 2,20 |
| 2 | 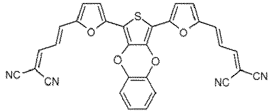 | 444 | 1,17 |
| 3 | 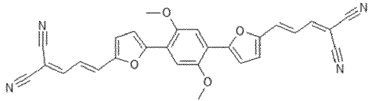 | 534 | 0,83 |
| 4 | 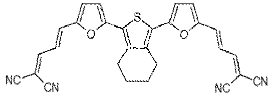 | 540 | 1,09 |
| 5 | 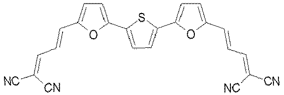 | 542 | 1,14 |
| 6 | 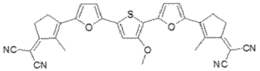 | 542 | 1,30 |
| 7 | 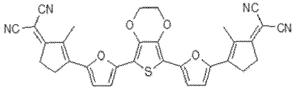 | 542 | 1,42 |
| 8 | 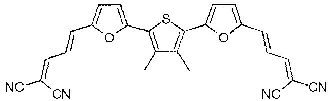 | 548 | 1,18 |
| 9 | 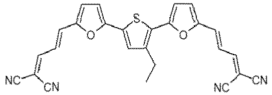 | 551 | 1,13 |
| 10 | 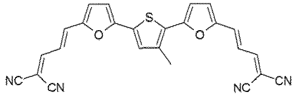 | 552 | 1,19 |
| 11 | 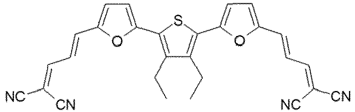 | 554 | 0,99 |
| 12 | 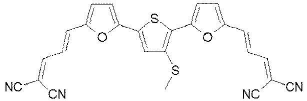 | 555 | 2,38 |
| 13 | 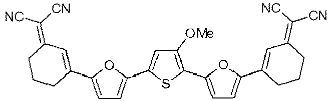 | 556 | 1,24 |
| 14 | 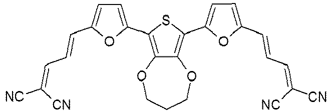 | 562 (THF) | 2,41 |
| 15 | 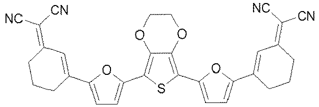 | 563 | 1,27 |
| 16 | 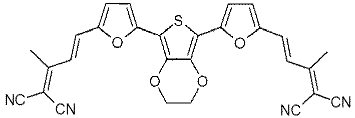 | 567 | 2,32 |
| 17 | 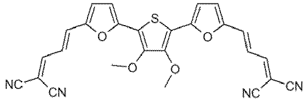 | 568 | 1,91 |
| 18 | 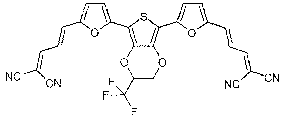 | 571 | 1,38 |
| 19 | 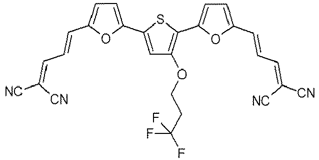 | 574 | 1,22 |
| 20 | 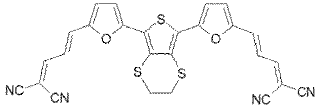 | 575 | 1,10 |
| 21 | 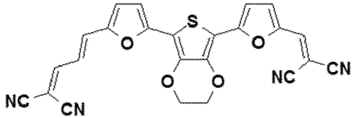 | 577 | 1,38 |
| 22 | 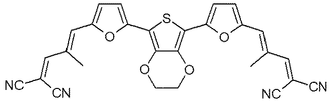 | 578 | 2,36 |
| 23 | 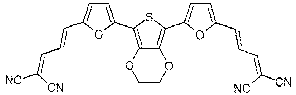 | 588 | 1,42 |
| 24 | 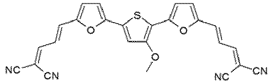 | 591 | 1,31 |
| 25 | 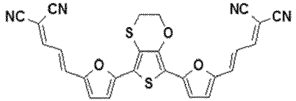 | 596 | 1,13 |
| 26 | 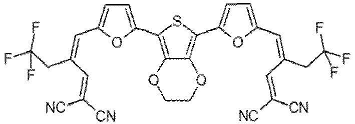 | 597 | 1,23 |
| 27 | 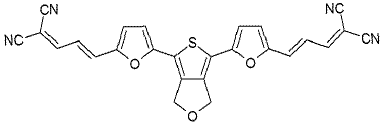 | 547 | 1,11 |
| 28 | 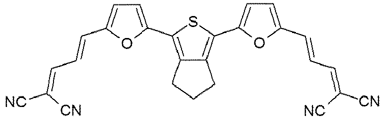 | 572 | 1,10 |
| 29 | 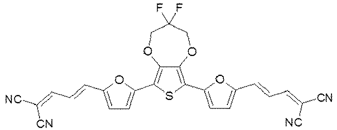 | 550 | 2,22 |
| 30 | 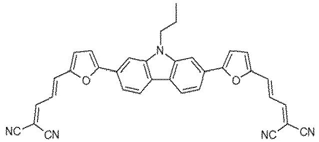 | 523 | 1,01 |
| 31 | 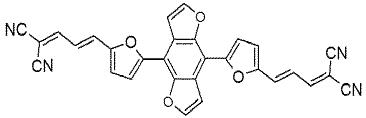 | 574 | 0,82 |
| 32 | 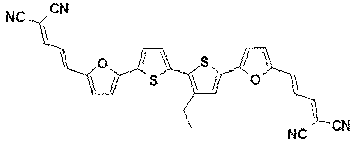 | 540 | 1,08 |
| 33 | 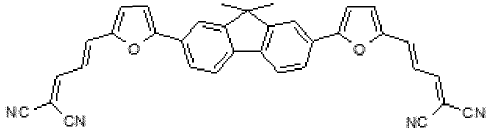 | 513 | 1,05 |
| 34 | 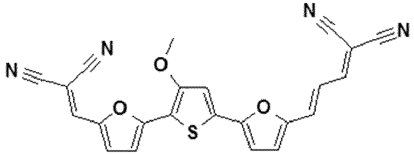 | 548 | 1,26 |
| 35 | 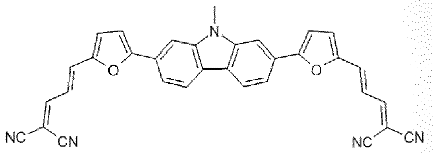 | 527 | 0,99 |
| 36 | 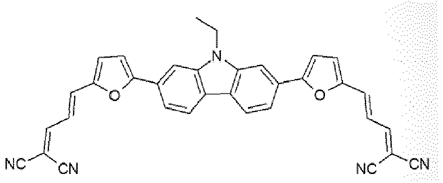 | 519 | 0,97 |
| 37 | 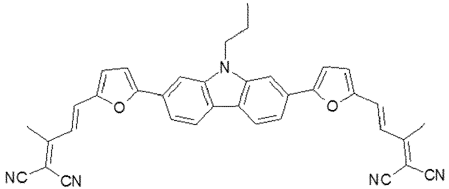 | 500 | 1,84 |
| 38 | 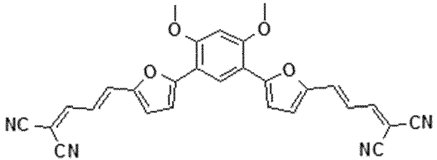 | 447 | 1,16 |
| 39 | 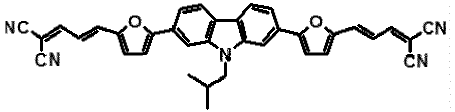 | 526 | 0,98 |
| 40 | 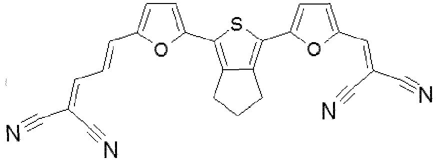 | 542 | 0,97 |
| 41 | 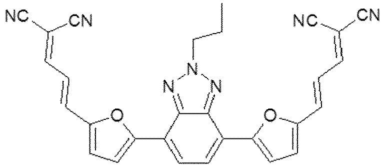 | 526 | 1,30 |
| 42 | 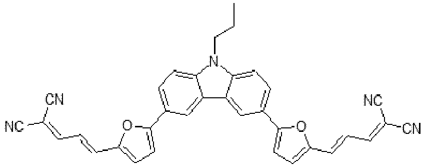 | 511 | 0,89 |
| 43 | 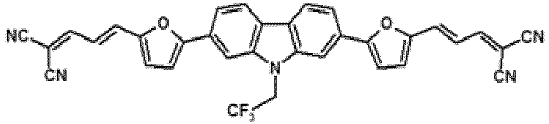 | 510 | 0,89 |
| 44 | 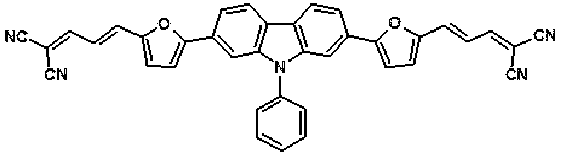 | 517 | 1,97 |
| 45 | 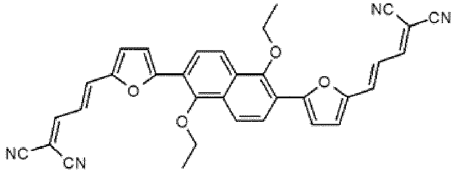 | 518 | 0,77 |
| 46 | 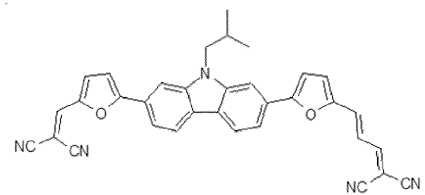 | 511 | 1,04 |
| 47 | 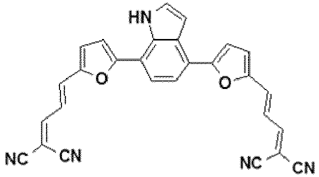 | 554 | 1,08 |
| 48 | 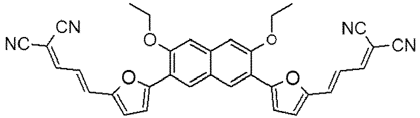 | 455 | 87 |

DSC steht für differential scanning calorimetry (dynamische Differenzkalorimatrie) .

Die optischen Eigenschaften wurden jeweils experimentell bestimmt. Das Absorptionsmaximum λₘₐₓ in nm wurde mit einer verdünnten Lösung in einer Küvette (falls nicht anders angegeben in Dichlormethan) mit Hilfe eines Photometers ermittelt. Die gemessenen Absorptionsmaxima aller beschriebenen Verbindungen liegen zwischen 400 und 600nm und überlappen damit besonders vorteilhaft mit dem energetischen Maximum des Sonnenspektrums.

Weitere optische Eigenschaften wie die Halbwertsbreite (HWB) wurden direkt an Filmen der organischen Verbindungen bestimmt. Zu diesem Zweck wurde mittels Vakuumsublimation bei 10⁻⁶ bis 10⁻⁷ mbar eine 30 nm dünne Schicht der jeweiligen erfindungsgemäßen Verbindung hergestellt. Die Schichtdicke wurde mittels Schwingquarzmonitor bestimmt.

Die erfindungsgemäßen Verbindungen zeichnen sich durch eine besonders hohe Absorption in einem breiten Spektrum des sichtbaren Lichts aus, was sich in den hohen erzielten Werten für die Halbwertsbreite wiederspiegelt. Die erfindungsgemäßen Verbindungen ermöglichen somit, Photonen über einen verhältnismäßig breiten Spektralbereich, der einen großen Anteil des kurzwelligen sichtbaren Sonnenlichtes umfasst, zu absorbieren und in elektrische Energie umzuwandeln.

Im Folgenden soll die Erfindung noch anhand einer Reihe von Figuren weiter erläutert werden:

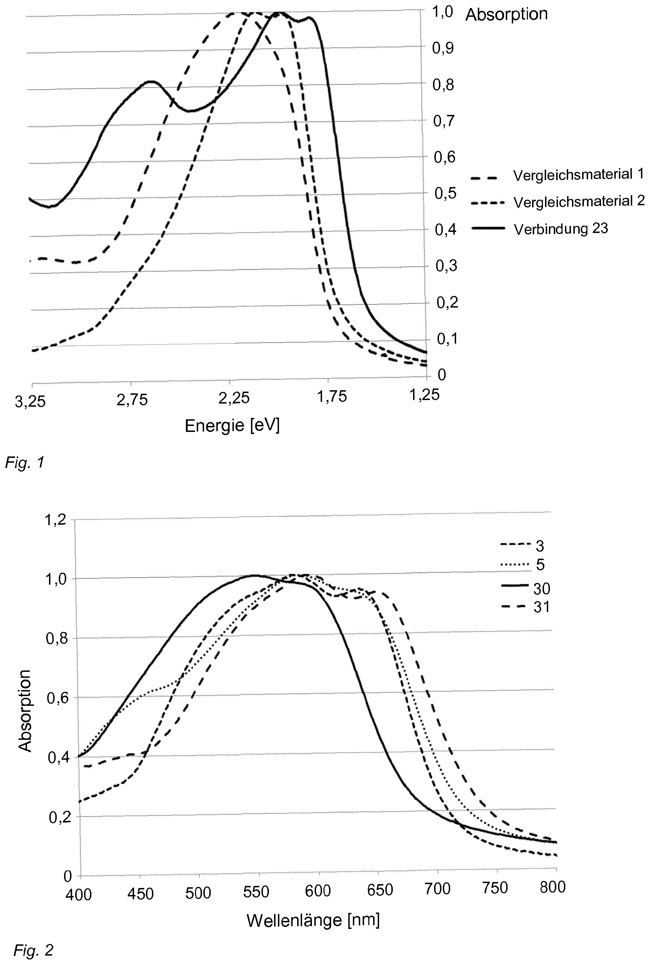

**Figur 1** Vergleicht normierte Absorptionsspektren in Elektronenvolt (gemessen an: 30 nm Film, vakuumgedampft, wie zuvor beschrieben). Gezeigt ist ein Vergleich von Verbindung **23** mit zwei Vergleichsmaterialien.

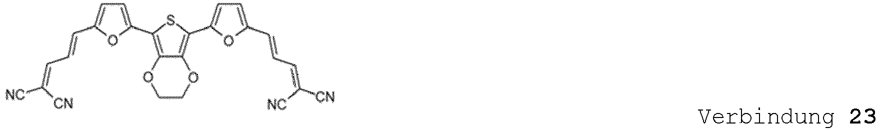

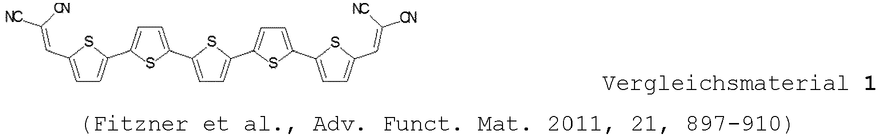

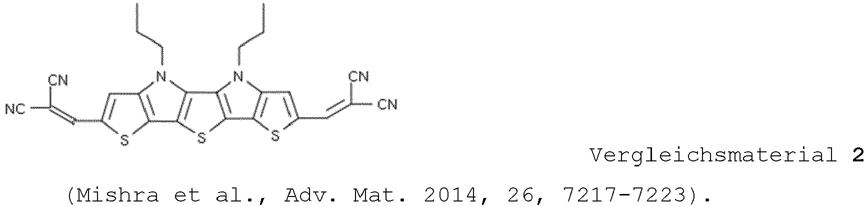

Die Messung vergleicht die Absorption aufgetragen gegen die Energie des eingestrahlten Lichts in Elektronenvolt. Es kann eine besonders hohe Halbwertsbreite im kurzwelligen Spektralbereich beobachtet werden. Insbesondere im Bereich von 2,5 eV und höheren Energien, also für 500 nm oder kürzere Wellenlängen zeigt Verbindung **23** eine deutlich höhere Absorption als die Vergleichsmaterialien. Allgemein zeigen die erfindungsgemäßen Verbindungen vor allem im Bereich zwischen 400 und 600 nm deutlich bessere Absorptionseigenschaften. Dadurch ist es möglich Fotoströme zu generieren, welche zu einem großen Teil kurzwellige Photonen nutzen. Diese Photonen werden von herkömmlichen organischen Verbindungen dagegen häufig nur unzulänglich genutzt.

**Figur 2** zeigt Absorptionsspektren der erfindungsgemäßen Verbindungen **3, 5, 30, 31:**

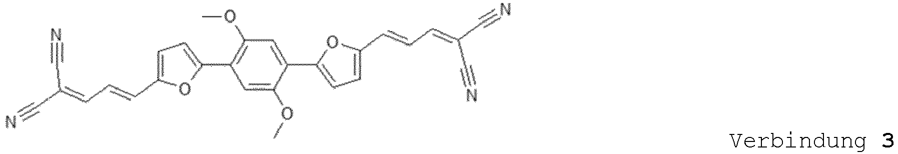

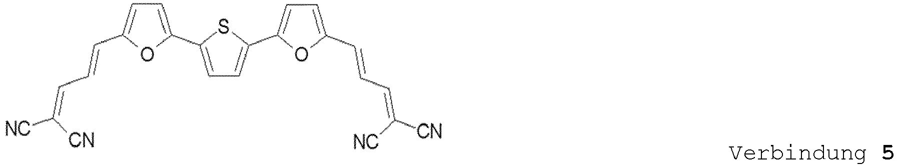

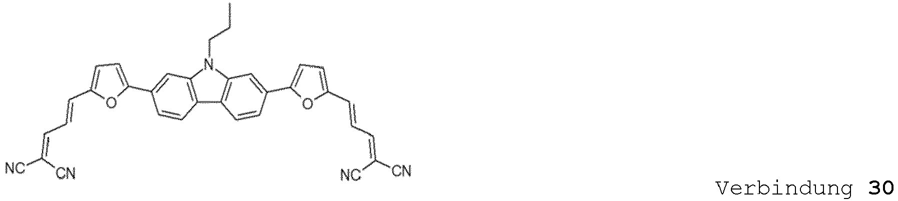

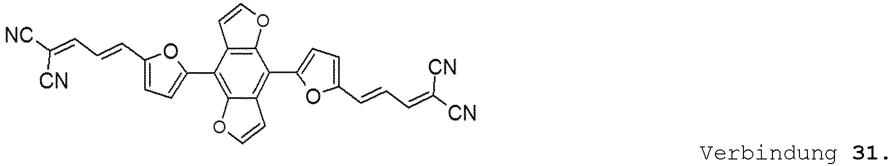

Die Absorptionsspektren wurden wiederum an Filmen der Verbindungen mit einer Dicke von 30 nm, die wie oben angegeben hergestellt und vermessen wurden, erhalten. Die Figur vergleicht also erfindungsgemäße Verbindungen, die sich in der Wahl der Struktureinheit für den mittleren konjugierten Block D² unterscheiden. Das Spektrum zeigt die Absorption aufgetragen gegen die Wellenlänge in Nanometern. Erstaunlicherweise zeigen alle angegebenen Verbindungen **3, 5, 30** und **31** trotz der verschiedenartigen mittleren konjugierten Blöcke ein sehr breites Absorptionsspektrum mit guter Absorption auch im Bereich kurzer Wellenlängen. Dabei lassen sich durch die Nutzung verschiedener mittlerer konjugierten Blöcke Feineinstellungen im Absorptionsspektrum erzielen.

**Figur 3** zeigt das Absorptionsspektrum von Verbindung **4** (gemessen an 30 nm Film von Verbindung **4;** PL: Photolumineszenz).

**Figur 4** zeigt die Strom-Spannungskurven (j-V) die an einem fotoaktiven organischen elektronischen Bauelement in Form einer organischen Solarzelle umfassend Verbindung **4** erhalten wurde. Die Messung wurde ohne Maske durchgeführt (without mask)

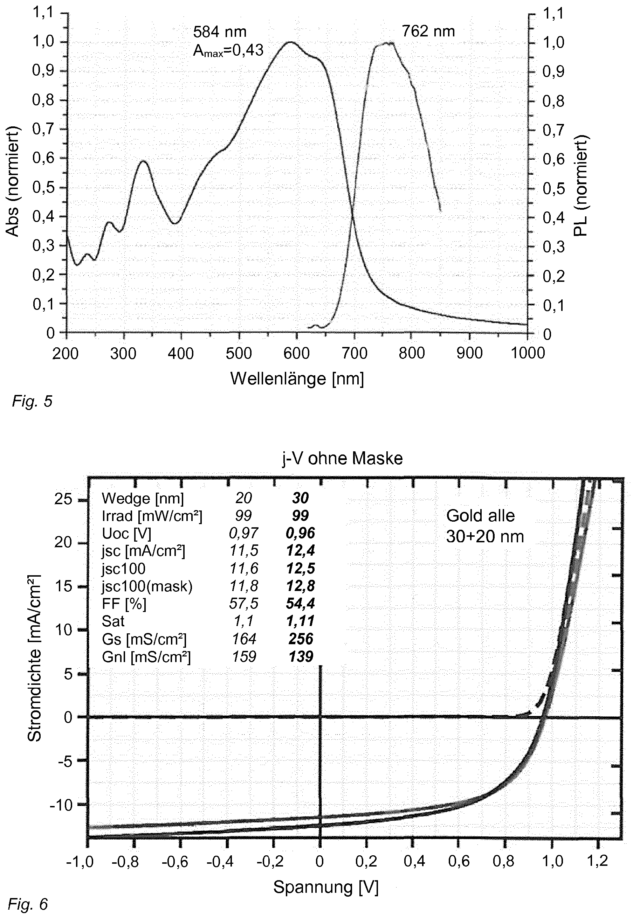

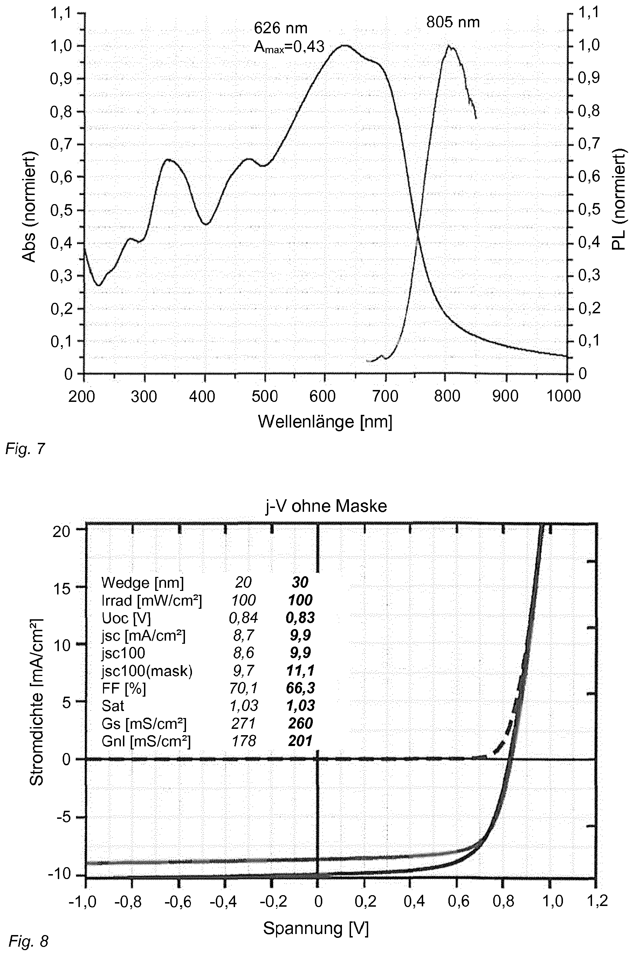

Messungen an fotoaktiven organischen elektronischen Bauelementen wurden hier und im Folgenden (siehe Figuren 6 und 8) jeweils mit einer sogenannten BHJ-Zelle durchgeführt. Die Zelle weist jeweils ein Glassubstrat und einen darauf aufgebrachten transparenten Deckkontakt aus ITO (ITO = Indium-Zinn-Oxid, engl. indium tin oxide) auf. Die Zelle besitzt zudem jeweils eine Schicht aufweisend Buckminster Fulleren (C60) und eine Mischschicht der jeweiligen erfindungsgemäßen Verbindungen mit C60 sowie eine p-dotierte Lochtransportschicht. Der Rückkontakt besteht aus einer aufgedampften Goldschicht.

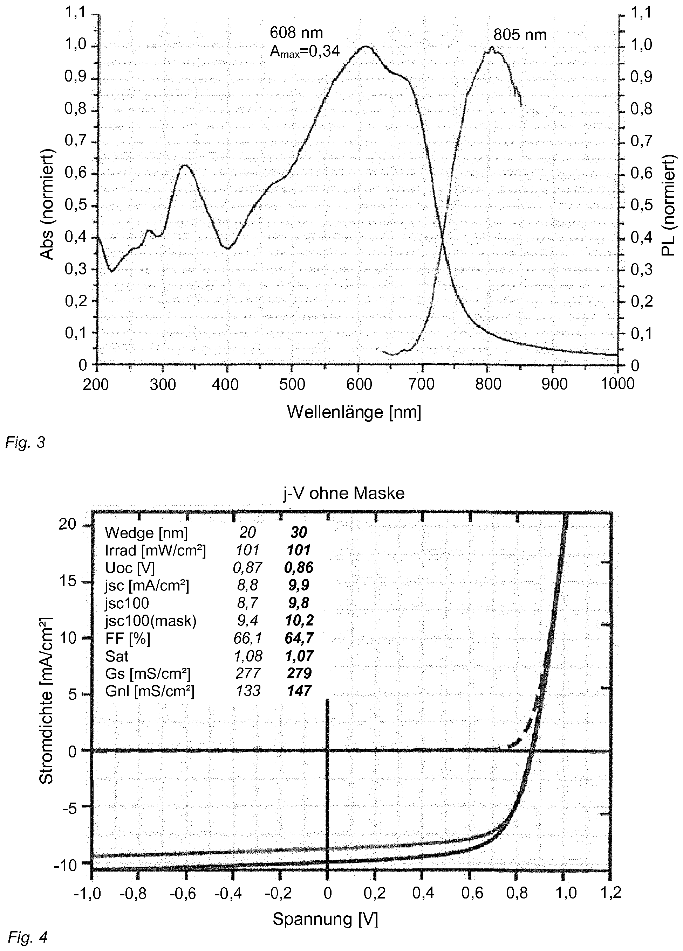

In Figur 4 ist die Strom-Spannungskurve von Verbindung **4** in einer BHJ-Zelle, wie soeben beschrieben gezeigt. Im konkreten Fall weist die BHJ-Zelle auf der ITO-Schicht eine Schicht von C60 mit einer Dicke von ca. 15 nm auf. Auf diese Schicht wurde Verbindung **4** zusammen mit C60 mit einer Dicke von 30 nm aufgetragen. Diese Schicht wird wiederum gefolgt von einer Schicht aus BPAPF mit einer Dicke von ca. 10 nm Auf der genannten Schicht befindet sich eine weitere Schicht umfassend BPAPF sowie NDP9 (kommerziell erhältlicher p-Dotand, Novaled AG) mit einer Dicke von 30 nm. Der Anteil an BPAPF an dieser Schicht beträgt bezogen auf die gesamte Schicht 90 Gewichtsprozent. An diese Schicht schließt sich eine weitere Schicht mit NDP9 mit einer Dicke von 1 nm an, woraufhin eine Goldschicht mit einer Dicke von 50 nm folgt.

In der Grafik sind zwei Strom-Spannungskurven abgebildet, die sich in der Dicke der Mischschicht aus Verbindung **4** und C60 unterscheidet (20 bzw. 30nm). Die Strom-Spannungskurve enthält zudem die wichtigsten Kennzahlen, welche die erfindungsgemäße organische Solarzelle kennzeichnen. Die wichtigsten Kennzahlen sind hierbei der Füllfaktor FF (Quotient aus der maximalen Leistung einer Solarzelle am Maximalleistungspunkt und dem Produkt aus Leerlaufspannung und Kurzschlussstrom, die Leerlaufspannung Uoc und der Kurzschlussstrom jsc) .

Die Strom-Spannungskurven, sowie die erhaltenen Kennzahlen belegen am Beispiel von Verbindung **4,** dass die erfindungsgemäßen Verbindungen sich für die Anwendung in organischen Solarzellen eignen.

**Figur 5** zeigt das Absorptionsspektrum von Verbindung **5** (gemessen an 30 nm Film von Verbindung **5**).

**Figur 6** zeigt zwei Strom-Spannungskurven von Verbindung **5** (20 bzw. 30nm Schichtdicke der Mischschicht mit C60, im Mischungsverhältnis 3:2 und bei einer Substrattemperatur von 50°C angefertigt) gemessen in einer BHJ-Zelle wie zuvor (im Zusammenhang mit Figur 3) bereits allgemein beschrieben. Die konkrete Schichtenfolge lautet dabei wie folgt: ITO/C60 (15nm)/Verbindung **5**:C60 (20/30nm, 3:2)/BPAPF (10nm)/BPAPF:NDP9 (30nm, 10wt%)/NDP (1nm) Au (50nm)

**Figur 7** zeigt das Absorptionsspektrum von Verbindung **24** (gemessen an 30 nm Film von Verbindung **24**).

**Figur 8****:** die Strom-Spannungskurven von Verbindung **24** gemessen in einer BHJ-Zelle wie zuvor (im Zusammenhang mit Figur 3) bereits allgemein beschrieben, jeweils 20 und 30nm Schichtdicke der Mischschicht von Verbindung **24** mit C60, im Mischungsverhältnis 1:1 bei 70°C Substrattemperatur angefertigt. Die konkrete Schichtenfolge lautet dabei wie folgt: ITO/C60 (15nm)/Verbindung **24** (20/30nm, 1:1)/BPAPF (5nm)/BPAPF:NDP9 (45nm, 10.1 wt%)/NDP9(1nm) Au (50nm).

Sowohl die in den Figuren 1, 2, 3, 5 und 7 untersuchten Absorptionseigenschaften der Verbindungen, wie auch die in den organischen Solarzellen der Figuren 4, 6 und 8 gemessenen Strom-Spannungsverläufe belegen, dass die erfindungsgemäßen Verbindungen sich für die Anwendung in organischen Solarzellen sowie anderen fotoaktiven organischen elektronischen Bauelementen eignen und eine gute Effizienz ermöglichen.

In **Tabelle 6** werden zudem noch einige optische Eigenschaften von erfindungsgemäßen Verbindungen und weiteren Vergleichsmaterialien zusammengefasst. Bei den Vergleichsmaterialien handelt es sich dabei um die Vergleichsmaterialien **3** bis **6:** 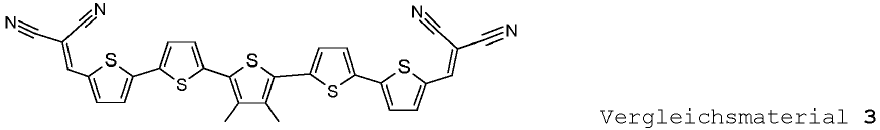 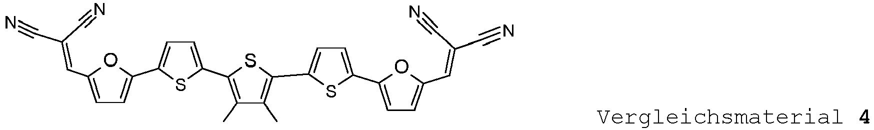 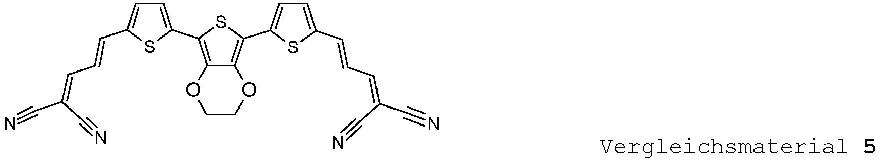 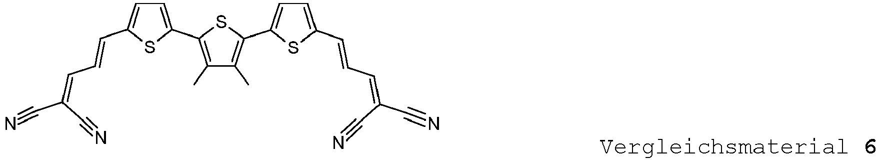 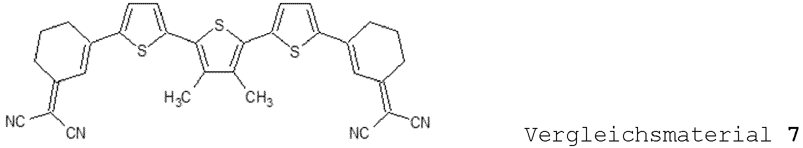 ("DCC3T", Fitzner, Adv. Funct. Mat. 2015, 25, 1845)

Die Vergleichsmaterialien sind in ihrer Struktur ähnlich zu den erfindungsgemäßen Verbindungen 23 und 8:

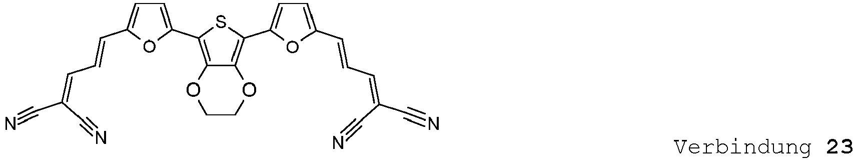

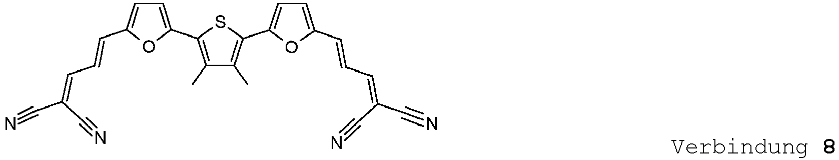

**Tabelle 6: Vergleich optischer Eigenschaften.**

| **Verbindung** | **ODₘₐₓ** | **OD-Integral (400-900 nm)** | **FWHM** |
|---|---|---|---|
| Vergleichsmaterial 3 | 0,45 | 101 | 209 |
| Vergleichsmaterial 4 | 0,4 | 89 | 212 |
| Vergleichsmaterial 5 | 0,42 | 104 | 251 |
| Vergleichsmaterial 6 | 0,38 | 92 | 257 |
| Vergleichsmaterial 7 | 0,45 | 84 | 192 |
| Verbindung 23 | 0,45 | 134 | 345 |
| Verbindung 8 | 0,42 | 106 | 284 |

**Tabelle 6** vergleicht die optische Dichte (ODmax, OD-Integral und die zugehörige Halbwertsbreite FWHM) von erfindungsgemäßen Verbindungen (Verbindung **23** und Verbindung **8**) mit einer Reihe von Vergleichsmaterialien mit ähnlicher Struktur. Die optische Dichte ist ein Maß für die Absorptionsstärke

Von den gezeigten Verbindungen weisen Verbindung **23** und Verbindung **8** die besten optischen Eigenschaften auf. Insbesondere wurden für diese Verbindungen die höchsten Halbwertsbreiten unter den angegebenen Bedingungen erhalten sowie die höchsten Werte für das OD-Integral. Dies weist auf eine besonders gute Nutzung von Photonen aus dem gesamten sichtbaren Spektrum hin.

Verbindung **23** unterscheidet sich von Vergleichsmaterial 5 ebenso wie von Verbindung 8 von Vergleichsmaterial **6** dadurch, dass die erfindungsgemäßen Verbindungen jeweils äußere Fünfringe aufweisend Sauerstoff anstelle von Schwefel besitzen. Die beobachteten besseren optischen Eigenschaften sind also auf diesen strukturellen Unterschied zurückzuführen. So ist also etwa ein Furan als äußerer Fünfring besser geeignet als ein Thiophenring. Diese Tatsache wird auch durch den Vergleich der erfindungsgemäßen Verbindung **8** mit dem literaturbekannten Vergleichsmaterial **7** ersichtlich. Diese Materialien unterscheiden sich zwar sowohl durch unterschiedliche Akzeptoreinheiten als auch durch den Austausch von Thiophen durch Furan, aber nur Verbindung **8** hat gleichzeitig eine starke und breite Absorption, was in einem wesentlich höheren Absorptionsintegral resultiert, während beim Vergleichsmaterial **6,** welches nur Thiophenringe, aber gleichzeitig eine offenkettige Akzeptorgruppe enthält, das Absorptionsintegral nur geringfügig höher ist. Somit kann der Effekt wiederum dem vorteilhaften Vorhandensein von Furanringen zugeordnet werden.

Die Erfinder der vorliegenden Erfindung haben zudem festgestellt, dass der besagte Effekt zudem eine elektronenziehnde Gruppe am besagten Fünfring mit zumindest zwei C=C-Doppelbindungen erfordert. Dies ergibt sich beispielsweise aus dem Vergleich von Vergleichsmaterial **3** und Vergleichsmaterial **4.** Obwohl letzteres als Fünfringe Furan-Ringe aufweist, zeigt es keine verbesserten optischen Eigenschaften. Dies ist darauf zurückzuführen, dass in Vergleichsmaterial **4** in Verbindung mit der Furan-Einheit nur eine Akzeptor-Gruppe am Furanring mit einer einzelnen C=C-Doppelbindung vorliegt. Die verbesserten Absorptionseigenschaften hingegen erfordern das gemeinsame Vorliegen einer Struktureinheit 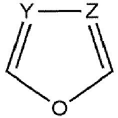 in Verbindung mit einer elektronenziehenden Gruppe A², die stets zumindest zwei C=C Doppelbindungen aufweist.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Patentansprüchen beinhaltet, auch wenn dieses Merkmal oder diese Kombination selbst nicht explizit in den Patentansprüchen oder Ausführungsbeispielen angegeben ist.

## Patentansprüche

1. Verbindung der allgemeinen Formel (I): 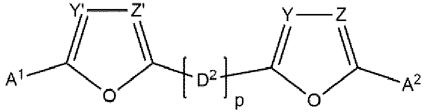
mit p ausgewählt aus: 1, 2, 3, 4 und 5,
mit Y, Y', Z und Z' jeweils unabhängig voneinander ausgewählt aus: N, CH, C-F, C-CH₃, C-CF₃, C-C₂H₅, C-C₃H₈, C-OCH₃, C-OC₂H₅, C-SCH₃, C-SC₂H₅; und
wobei die konjugierten Blöcke D² jeweils unabhängig voneinander ausgewählt sind aus der Gruppe der folgenden Struktureinheiten: 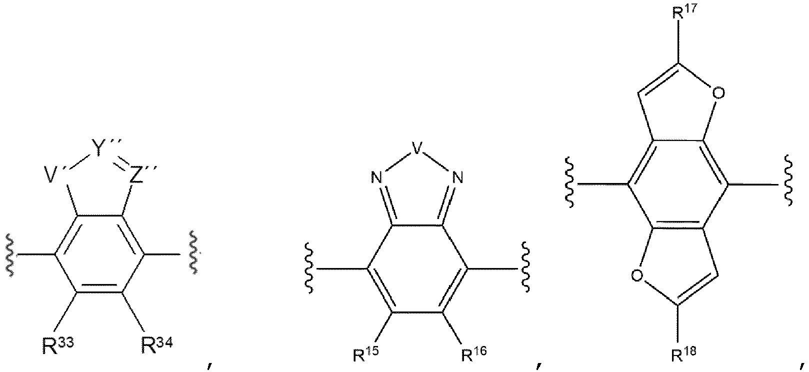 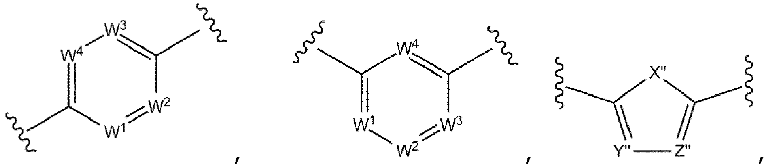 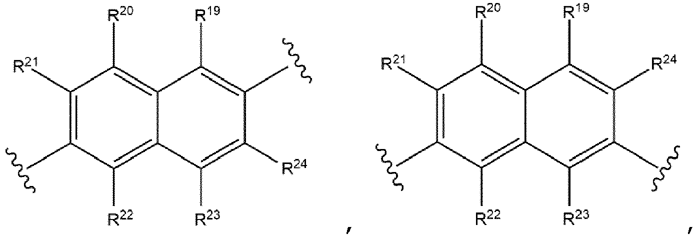 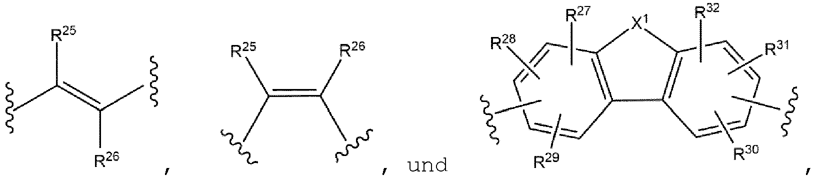
mit Y" ausgewählt aus: N oder CR^{g};
mit Z" ausgewählt aus: N oder CR^{h};
mit X" ausgewählt aus: O, S, Se, Si(RⁱR^{j}), P (Rⁱ) , P(O)Rⁱ;
mit V ausgewählt aus: O, S, Se oder NR^{k};
mit V' ausgewählt aus: O, S, Se oder NR^{k};
mit X¹ ausgewählt aus: NR^{l}, CR^{l}R^{m};
mit W¹ bis W⁴ unabhängig voneinander ausgewählt aus: N, CRⁿ;
wobei die Reste R^{g} bis Rⁿ und die Reste R¹⁵ bis R³⁴ jeweils unabhängig voneinander aus der Gruppe der Reste ausgewählt sind: H, Halogen, CN, NR^{d}R^{e}, wobei R^{d} und R^{e} jeweils unabhängig voneinander ausgewählt sind aus der Gruppe der Reste: H, sowie zyklisches oder offenkettiges C₁-C₂₀-Alkyl, wobei einzelne C-Atome durch Heteroatome ersetzt sein können und wobei Wasserstoffatome des Alkylrests substituiert sein können, zyklisches oder offenkettiges C₁-C₂₀-Alkyl, wobei einzelne C-Atome durch Heteroatome ersetzt sein können, zyklisches oder offenkettiges C₁-C₂₀-O-Alkyl, zyklisches oder offenkettiges C₁-C₂₀-S-Alkyl, zyklisches oder offenkettiges C₂-C₂₀-Alkenyl, zyklisches oder offenkettiges C₂-C₂₀-O-Alkenyl, zyklisches oder offenkettiges C₂-C₂₀-S-Alkenyl, zyklisches oder offenkettiges C₂-C₂₀-Alkinyl, Aryl, Heteroaryl,
wobei Wasserstoffatome der Reste Alkyl, O-Alkyl, S-Alkyl, Alkenyl, O-Alkenyl, S-Alkenyl, Alkinyl, Aryl und Heteroaryl jeweils unabhängig voneinander substituiert sein können; und
wobei die Reste R^{g} und R^{h} miteinander in Form einer Ringstruktur verbunden sein können und wobei an die Ringstruktur ein Aryl-Rest kondensiert sein kann, wobei der gegebenenfalls an die Ringstruktur kondensierte Aryl-Rest substituiert sein kann,
wobei A¹ ausgewählt ist aus der Gruppe der folgenden Reste: 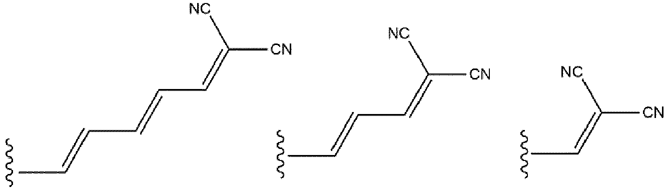 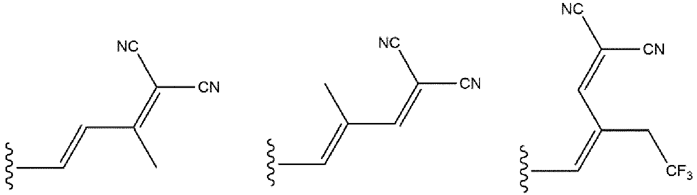 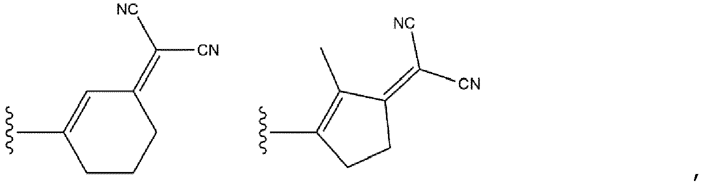 und
wobei A² ausgewählt ist aus der Gruppe der folgenden Reste:

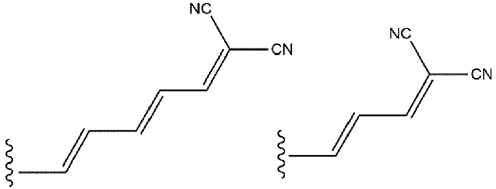

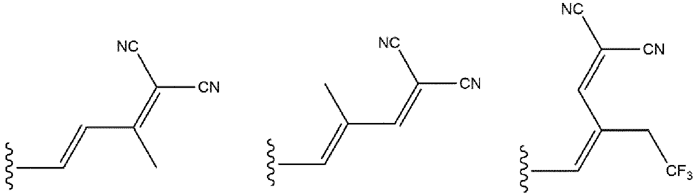

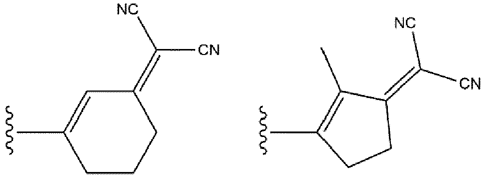

2. Verbindung gemäß Anspruch 1, wobei Y, Y', Z, Z' jeweils CH sind.

3. Verbindung gemäß einem der vorherigen Ansprüche, wobei zumindest einer der konjugierten Blöcke D² die allgemeine Formel 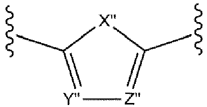 aufweist.

4. Verbindung gemäß einem der vorherigen Ansprüche, wobei zumindest einer der konjugierten Blöcke D² die allgemeine Formel 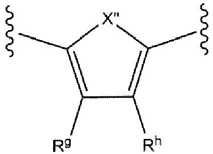 aufweist,
wobei die Reste R^{g} und R^{h} unabhängig voneinander aus der Gruppe der folgenden Reste ausgewählt sind: H, F, C₁-C₅-Alkyl, wobei einzelne C-Atome durch Heteroatome ersetzt sein können, C₁-C₅-O-Alkyl, C₁-C₅-S-Alkyl, wobei Wasserstoffatome der Reste Alkyl, O-Alkyl und S-Alkyl, jeweils unabhängig voneinander mit Fluor substituiert sein können,
wobei die Reste R^{g} und R^{h} miteinander in Form einer Ringstruktur verbunden sein können und wobei an die Ringstruktur ein Aryl-Rest kondensiert sein kann.

5. Verbindung gemäß einem der vorherigen Ansprüche, wobei X" gleich S ist.

6. Verbindung nach Anspruch 5 mit der folgenden allgemeinen Formel:

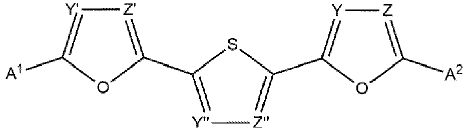

7. Verbindung gemäß einem der vorherigen Ansprüche, wobei zumindest einer der konjugierten Blöcke D² die allgemeine Formel 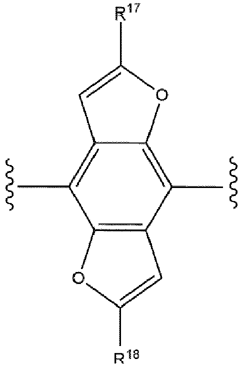 aufweist,
wobei R¹⁷ und R¹⁸ unabhängig voneinander ausgewählt sind aus der Gruppe der folgenden Reste: H, F, C₁-C₅-Alkyl, C₁-C₅-O-Alkyl, C₁-C₅-S-Alkyl, wobei Wasserstoffatome der Reste Alkyl, O-Alkyl und S-Alkyl jeweils unabhängig voneinander durch Fluoratome ersetzt sein können.

8. Verbindung gemäß einem der vorherigen Ansprüche, wobei zumindest einer der konjugierten Blöcke D² die allgemeine Formel 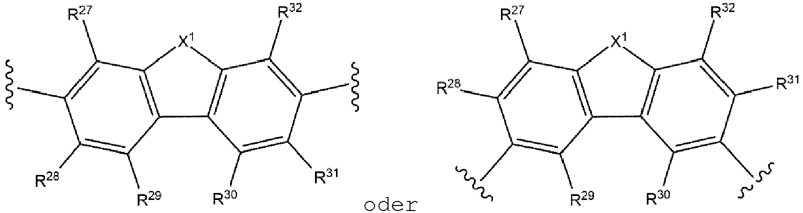 aufweist,
wobei X¹ ausgewählt ist aus: NR^{l}, CR^{l}R^{m},
wobei R^{l} und R^{m} jeweils unabhängig voneinander ausgewählt sind aus der Gruppe der Reste: H, offenkettiges C₁-C₅-Alkyl, C₅-C₁₂-Aryl, C₅-C₁₂-Heteroaryl, wobei Wasserstoffatome des offenkettigen C₁-C₅-Alkyls zumindest teilweise durch Fluoratome ersetzt sein können und wobei Wasserstoffatome des C₅-C₁₂-Aryls und des C₅-C₁₂-Heteroaryls zumindest teilweise substituiert sein können, und
wobei R²⁷ bis R³² unabhängig voneinander ausgewählt sind aus der Gruppe der folgenden Reste: H, F, C₁-C₅-Alkyl, C₁-C₅-O-Alkyl, C₁-C₅-S-Alkyl, wobei Wasserstoffatome der Reste Alkyl, O-Alkyl und S-Alkyl jeweils unabhängig voneinander durch Fluoratome ersetzt sein können.

9. Verbindung gemäß einem der vorherigen Ansprüche, wobei zumindest einer der konjugierten Blöcke D² die allgemeine Formel 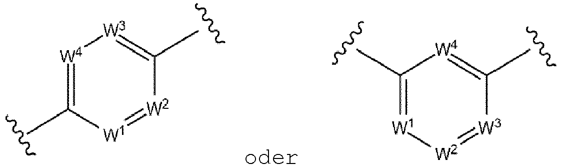 aufweist,
wobei W¹ bis W⁴ unabhängig voneinander ausgewählt sind aus: N und CRⁿ,
wobei Rⁿ ausgewählt ist aus der Gruppe der folgenden Reste: H, F, C₁-C₅-Alkyl, C₁-C₅-O-Alkyl, C₁-C₅-S-Alkyl, wobei
Wasserstoffatome der Reste Alkyl, O-Alkyl und S-Alkyl jeweils unabhängig voneinander durch Fluoratome ersetzt sein können.

10. Verbindung gemäß einem der vorherigen Ansprüche, wobei zumindest einer der konjugierten Blöcke D² die allgemeine Formel 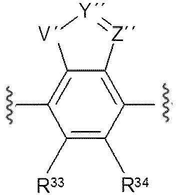 aufweist,
wobei R³³ bis R³⁴ jeweils unabhängig voneinander ausgewählt sind aus der Gruppe der folgenden Reste: H, F, C₁-C₅-Alkyl, C₁-C₅-O-Alkyl, C₁-C₅-S-Alkyl, wobei Wasserstoffatome der Reste Alkyl, O-Alkyl und S-Alkyl jeweils unabhängig voneinander durch Fluoratome ersetzt sein können und
mit Y" ausgewählt aus: N oder CR^{g};
mit Z" ausgewählt aus: N oder CR^{h};
mit V' ausgewählt aus: O, S, Se oder NR^{k};
wobei R^{g} und R^{h} ausgewählt sind aus:
H, Halogen, CN, NR^{d}R^{e}, wobei R^{d} und R^{e} jeweils unabhängig voneinander ausgewählt sind aus der Gruppe der Reste: H, sowie zyklisches oder offenkettiges C₁-C₂₀-Alkyl, wobei einzelne C-Atome durch Heteroatome ersetzt sein können und wobei Wasserstoffatome des Alkylrests substituiert sein können, zyklisches oder offenkettiges C₁-C₂₀-Alkyl, wobei einzelne C-Atome durch Heteroatome ersetzt sein können, zyklisches oder offenkettiges C₁-C₂₀-O-Alkyl, zyklisches oder offenkettiges C₁-C₂₀-S-Alkyl, zyklisches oder offenkettiges C₂-C₂₀-Alkenyl, zyklisches oder offenkettiges C₂-C₂₀-O-Alkenyl, zyklisches oder offenkettiges C₂-C₂₀-S-Alkenyl, zyklisches oder offenkettiges C₂-C₂₀-Alkinyl, Aryl, Heteroaryl,
wobei Wasserstoffatome der Reste Alkyl, O-Alkyl, S-Alkyl, Alkenyl, O-Alkenyl, S-Alkenyl, Alkinyl, Aryl und Heteroaryl jeweils unabhängig voneinander substituiert sein können,
wobei R^{k} ausgewählt ist aus der Gruppe der Reste: H, offenkettiges C₁-C₅-Alkyl, C₅-C₁₂-Aryl, C₅-C₁₂-Heteroaryl, wobei Wasserstoffatome des offenkettigen C₁-C₅-Alkyls zumindest teilweise durch Fluoratome ersetzt sein können und wobei Wasserstoffatome des C₅-C₁₂-Aryls und des C₅-C₁₂-Heteroaryls zumindest teilweise substituiert sein können.

11. Verbindung gemäß einem der vorherigen Ansprüche, wobei die konjugierten Blöcke D² unabhängig voneinander ausgewählt sind aus der Gruppe der folgenden Struktureinheiten:

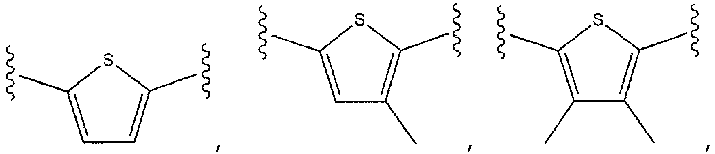

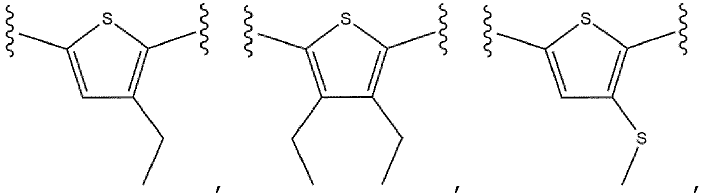

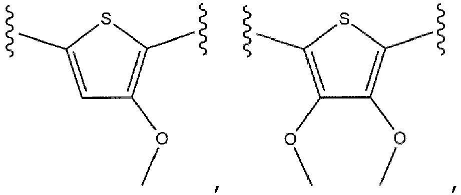

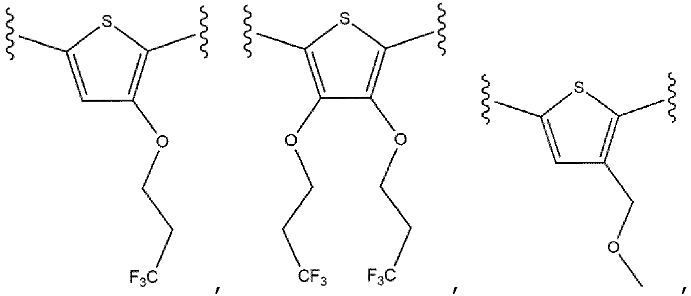

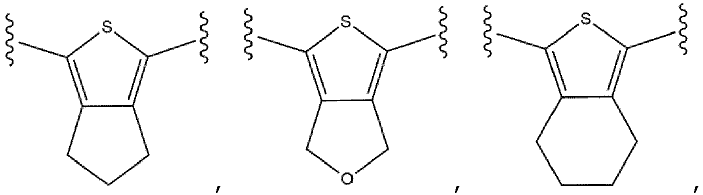

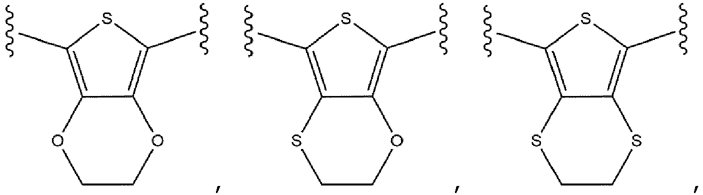

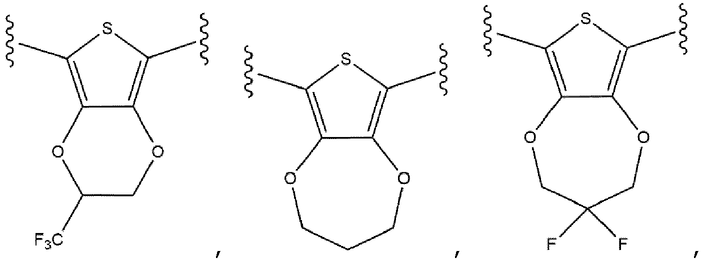

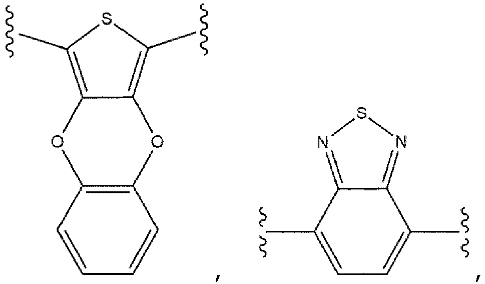

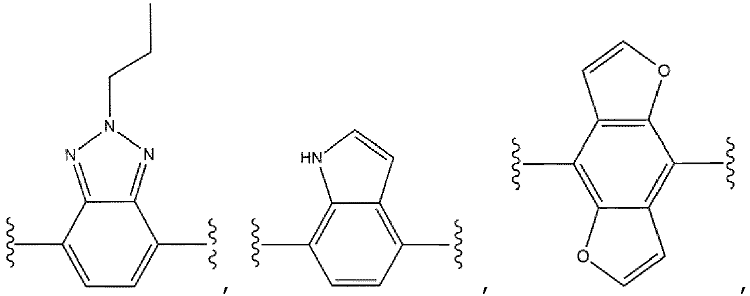

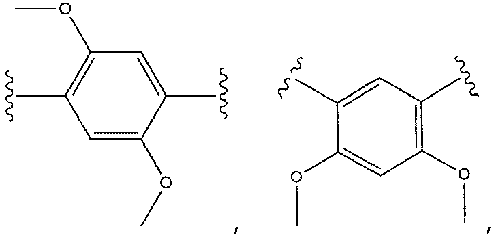

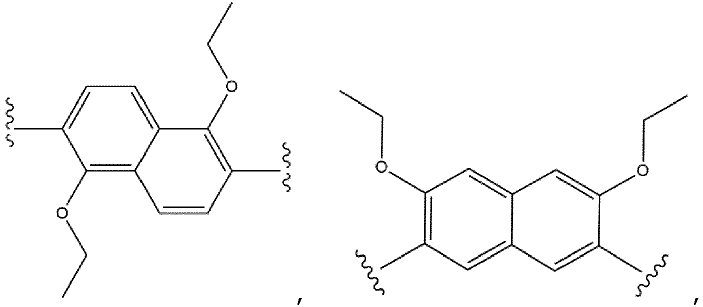

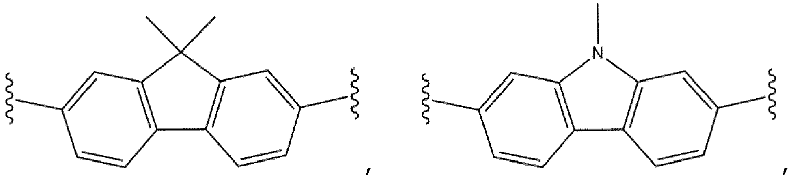

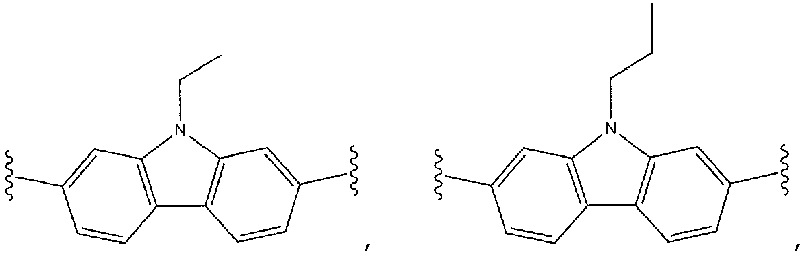

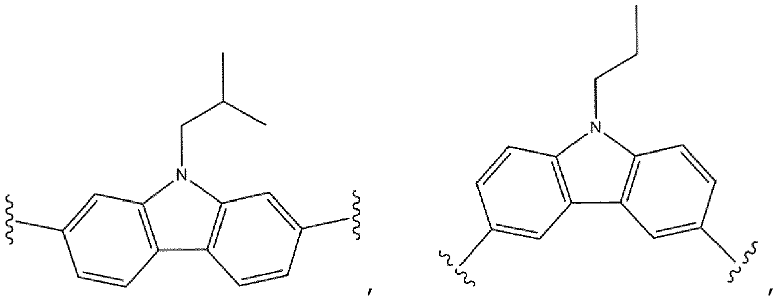

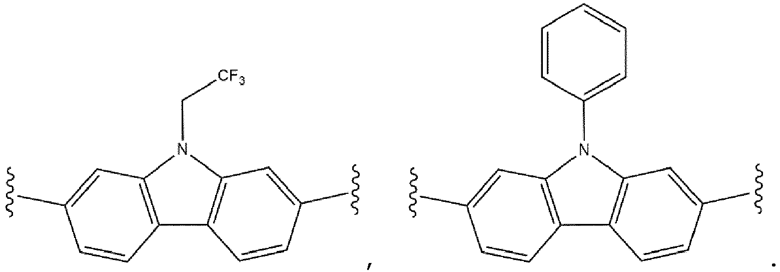

12. Verwendung einer oder mehrerer Verbindungen gemäß einem der Ansprüche 1 bis 11 in einem fotoaktiven organischen elektronischen Bauelement, bevorzugt einer organischen Solarzelle.

13. Fotoaktives organisches elektronisches Bauelement, umfassend eine Verbindung gemäß einem der Ansprüche 1 bis 11.

14. Verbindung für die Herstellung einer Verbindung gemäß einem der Ansprüche 1 bis 11, aufweisend die folgende allgemeine Formel 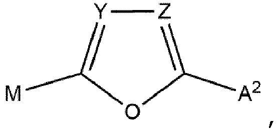 wobei Y, Z und A² so definiert sind, wie in einem der vorherigen Ansprüche und wobei M ausgewählt ist aus einer der folgenden funktionellen Gruppen:
-SnR*₃, -B(OR*)₂, -Zn-Hal*, -Mg-Hal*,
wobei es sich bei R* um ein C₁,-C₁₀-Alkyl handelt und wobei es sich bei der Gruppe Hal* um ein Halogen handelt.

## Claims

1. Compound of the general formula (I): 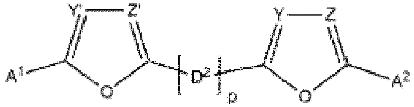
with p selected from: 1, 2, 3, 4 and 5, with Y, Y', Z and Z' each independently selected from: N, CH, C-F, C-CH₃, C-CF₃, C-C₂H₅, C-C₃H₈, C-OCH₃, C-OC₂H₅, C-SCH₃, C-SC₂H₅; and
wherein the conjugated D² blocks are each independently selected from the group of the following structural units: 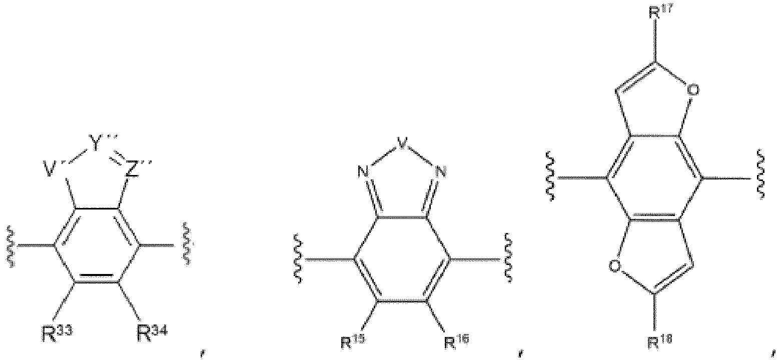 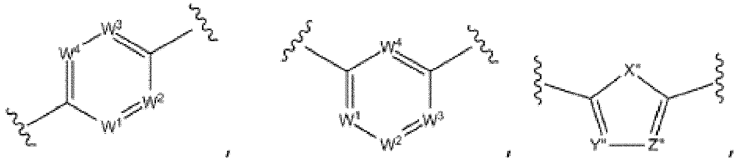 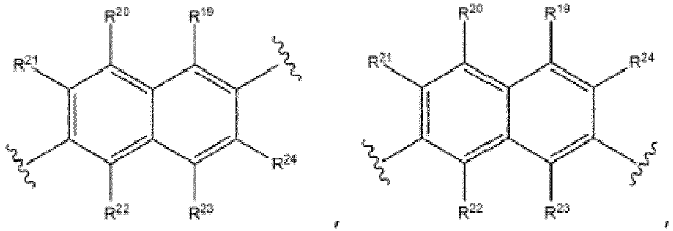 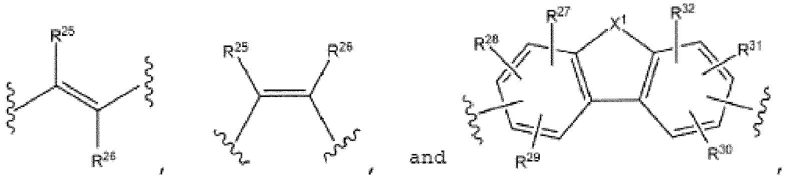
with Y" selected from: N or CR^{g};
with Z" selected from: N or CR^{h};
with X" selected from: O, S, Se, Si(RⁱR^{j}), P(Rⁱ), P(O)Rⁱ;
with V selected from: O, S, Se or NR^{k};
with V' selected from: O, S, Se or NR^{k};
with X¹ selected from: NR^{l}, CR^{l}R^{m};
with W¹ to W⁴ independently selected from: N, CRⁿ;
wherein the R^{g} to Rⁿ radicals and the R¹⁵ to R³⁴ radicals are each independently selected from the group of the radicals: H, halogen, CN, NR^{d}R^{e} wherein R^{d} and R^{e} are each independently selected from the group of the radicals: H, and cyclic or open-chain C₁-C₂₀-alkyl, wherein individual carbon atoms may be replaced by heteroatoms and wherein hydrogen atoms in the alkyl radical may be substituted,
cyclic or open-chain C₁-C₂₀-O-alkyl,
cyclic or open-chain C₁-C₂₀-S-alkyl,
cyclic or open-chain C₂-C₂₀-alkenyl,
cyclic or open-chain C₂-C₂₀-O-alkenyl,
cyclic or open-chain C₂-C₂₀-S-alkenyl,
cyclic or open-chain C₂-C₂₀-alkynyl, aryl, heteroaryl,
wherein hydrogen atoms in the alkyl, O-alkyl, S-alkyl, alkenyl, O-alkenyl, S-alkenyl, alkynyl, aryl and heteroaryl radicals may each independently be substituted; and wherein the R^{g} and R^{h} radicals may be joined to one another in the form of a ring structure and
wherein an aryl radical may be fused onto the ring structure, where any aryl radical fused to the ring structure may be substituted,
wherein A¹ is selected from the group of the following radicals: 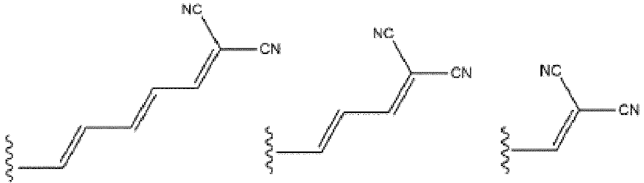 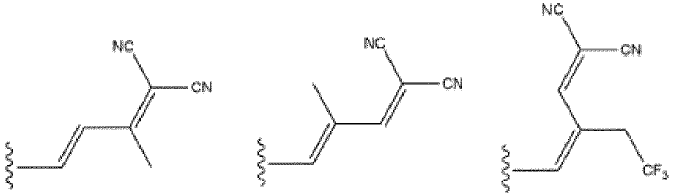 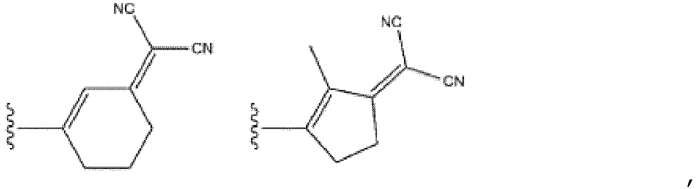 and
wherein A² is selected from the group of the following radicals:

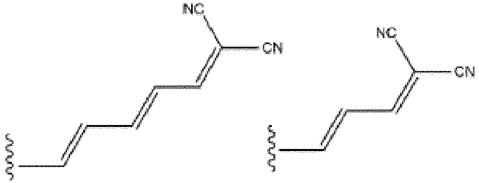

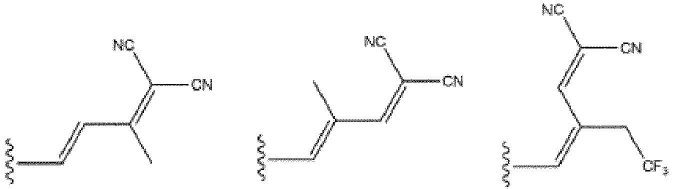

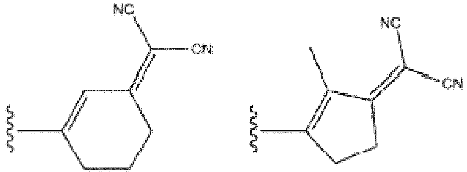

2. Compound according to Claim 1, wherein Y, Y', Z, Z' are each CH.

3. Compound according to any of the preceding claims, wherein at least one of the conjugated D² blocks has the general formula

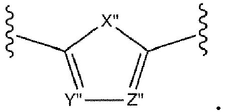

4. Compound according to any of the preceding claims, wherein at least one of the conjugated D² blocks has the general formula 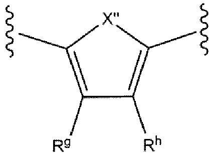
wherein the R^{g} and R^{h} radicals are independently selected from the group of the following radicals: H, F, C₁-C₅-alkyl, wherein individual carbon atoms may be replaced by heteroatoms,
C₁-C₅-O-alkyl, C₁-C₅-S-alkyl, wherein hydrogen atoms in the alkyl, O-alkyl and S-alkyl radicals may each independently be substituted by fluorine,
wherein the R^{g} and R^{h} radicals may be joined to one another in the form of a ring structure and wherein an aryl radical may be fused to the ring structure.

5. Compound according to any of the preceding claims, wherein X" = S.

6. Compound according to Claim 5 having the following general formula:

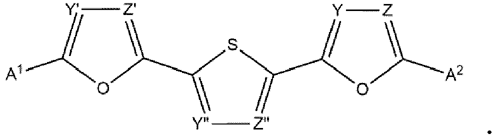

7. Compound according to any of the preceding claims, wherein at least one of the conjugated D² blocks has the general formula 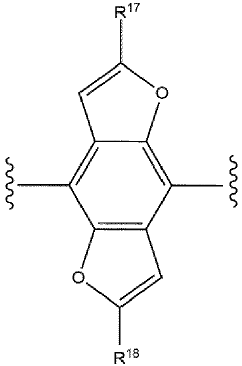 wherein R¹⁷ and R¹⁸ are independently selected from the group of the following radicals: H, F, C₁-C₅-alkyl, C₁-C₅-O-alkyl, C₁-C₅-S-alkyl, wherein hydrogen atoms in the alkyl, O-alkyl and S-alkyl radicals may each independently be replaced by fluorine atoms.

8. Compound according to any of the preceding claims, wherein at least one of the conjugated D² blocks has the general formula 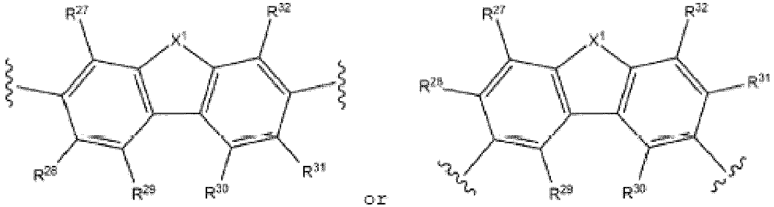
wherein X¹ is selected from: NR^{l}, CR^{l}R^{m},
wherein R^{l} and R^{m} are each independently selected from the group of the radicals: H, open-chain C₁-C₅-alkyl, C₅-C₁₂-aryl, C₅-C₁₂-heteroaryl, wherein hydrogen atoms in the open-chain C₁-C₅-alkyl may at least partly be replaced by fluorine atoms and wherein hydrogen atoms in the C₅-C₁₂-aryl and the C₅-C₁₂-heteroaryl may at least partly be substituted, and
wherein R²⁷ to R³² are independently selected from the group of the following radicals: H, F, C₁-C₅-alkyl, C₁-C₅-O-alkyl, C₁-C₅-S-alkyl, wherein hydrogen atoms in the alkyl, O-alkyl and S-alkyl radicals may each independently be replaced by fluorine atoms.

9. Compound according to any of the preceding claims, wherein at least one of the conjugated D² blocks has the general formula 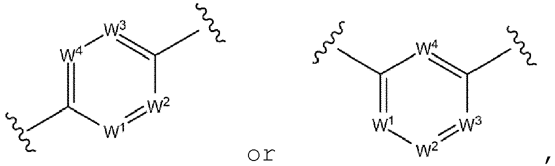
wherein W¹ to W⁴ are independently selected from: N and CRⁿ,
wherein Rⁿ is selected from the group of the following radicals: H, F, C₁-C₅-alkyl, C₁-C₅-O-alkyl, C₁-C₅-S-alkyl, wherein hydrogen atoms in the alkyl, O-alkyl and S-alkyl radicals may each independently be replaced by fluorine atoms.

10. Compound according to any of the preceding claims, wherein at least one of the conjugated D² blocks has the general formula 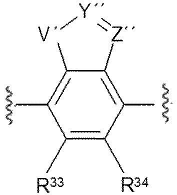
wherein R³³ to R³⁴ are each independently selected from the group of the following radicals: H, F, C₁-C₅-alkyl, C₁-C₅-O-alkyl, C₁-C₅-S-alkyl, wherein hydrogen atoms in the alkyl, O-alkyl and S-alkyl radicals may each independently be replaced by fluorine atoms and
with Y" selected from: N or CR^{g};
with Z" selected from: N or CR^{h};
with V' selected from: O, S, Se or NR^{k};
wherein R^{g} and R^{h} are selected from:
H, halogen, CN, NR^{d}R^{e} where R^{d} and R^{e} are each independently selected from the group of the radicals: H, and cyclic or open-chain C₁-C₂₀-alkyl, wherein individual carbon atoms may be replaced by heteroatoms and wherein hydrogen atoms in the alkyl radical may be substituted,
cyclic or open-chain C₁-C₂₀-O-alkyl,
cyclic or open-chain C₁-C₂₀-S-alkyl,
cyclic or open-chain C₂-C₂₀-alkenyl,
cyclic or open-chain C₂-C₂₀-O-alkenyl,
cyclic or open-chain C₂-C₂₀-S-alkenyl,
cyclic or open-chain C₂-C₂₀-alkynyl, aryl, heteroaryl,
wherein hydrogen atoms in the alkyl, O-alkyl, S-alkyl, alkenyl, O-alkenyl, S-alkenyl, alkynyl, aryl and heteroaryl radicals may each independently be substituted,
wherein R^{k} is selected from the group of the radicals: H, open-chain C₁-C₅-alkyl, C₅-C₁₂-aryl, C₅-C₁₂-heteroaryl, wherein hydrogen atoms in the open-chain C₁-C₅-alkyl may at least partly be replaced by fluorine atoms and wherein hydrogen atoms in the C₅-C₁₂-aryl and the C₅-C₁₂-heteroaryl may at least partly be substituted.

11. Compound according to any of the preceding claims, wherein the conjugated D² blocks are independently selected from the group of the following structural units:

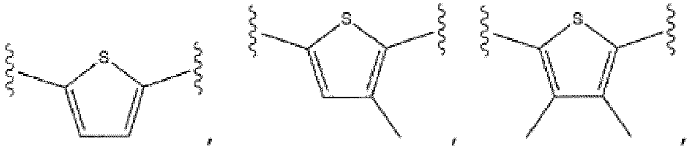

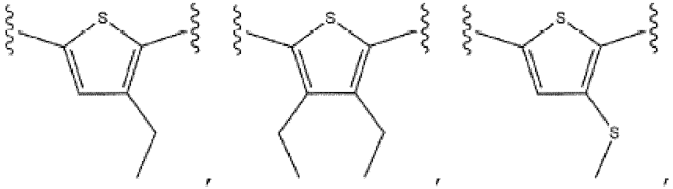

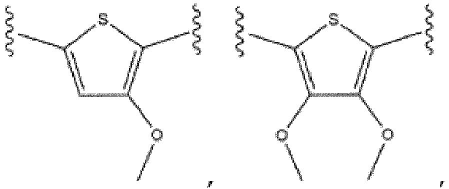

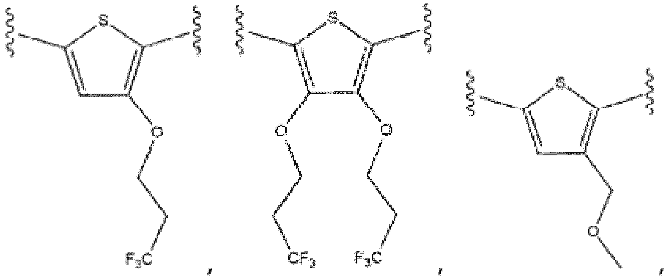

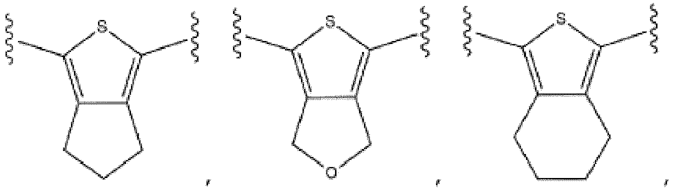

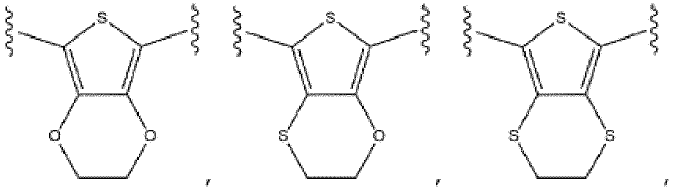

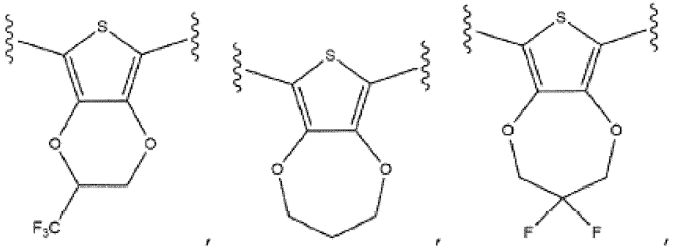

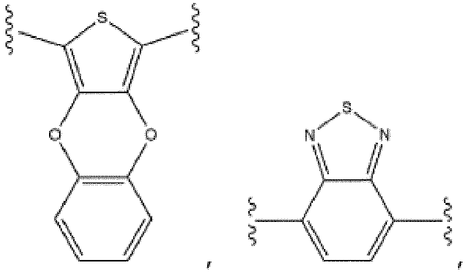

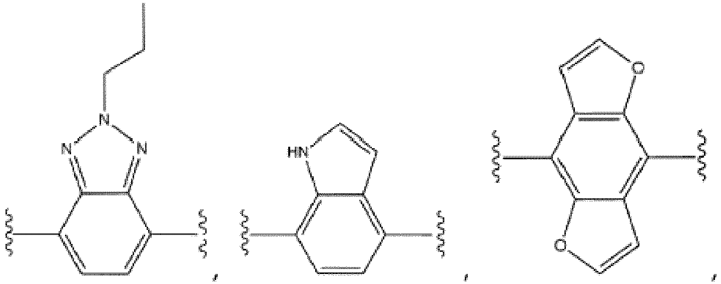

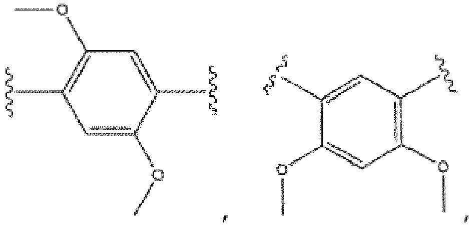

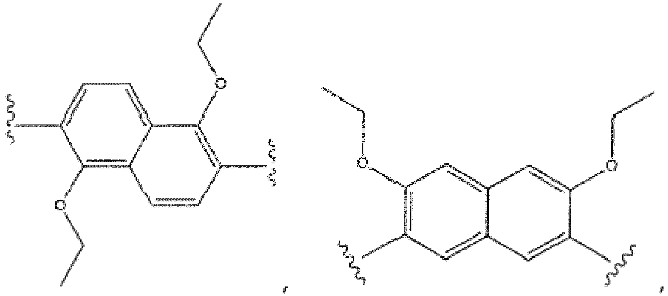

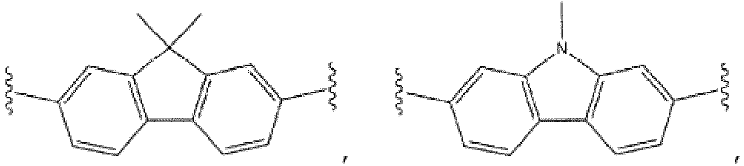

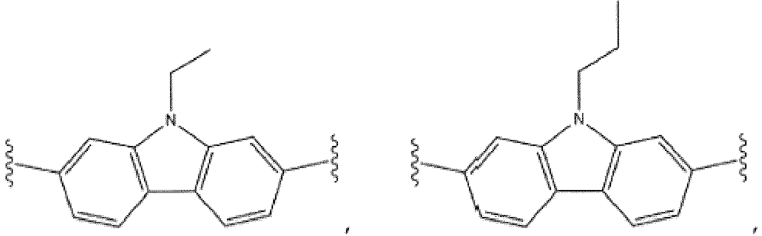

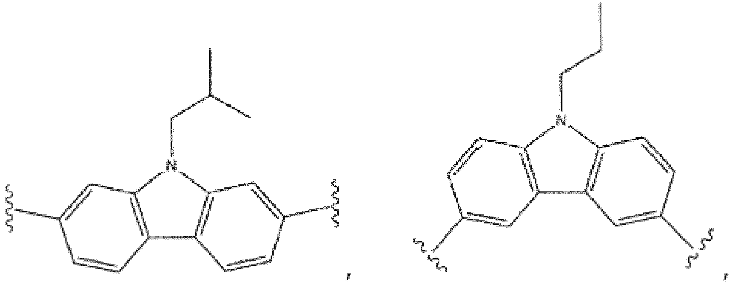

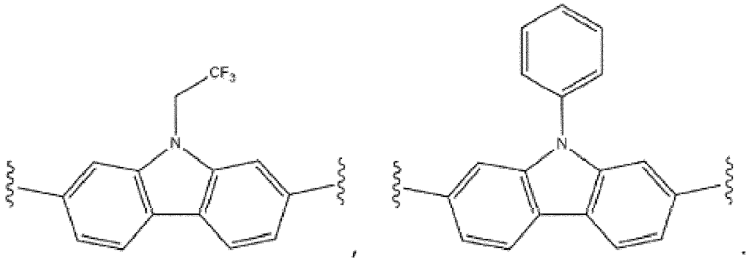

12. Use of one or more compounds according to any of Claims 1 to 11 in a photoactive organic electronic component, preferably an organic solar cell.

13. Photoactive organic electronic component comprising a compound according to any of Claims 1 to 11.

14. Compound for the preparation of a compound according to any of Claims 1 to 11, having the following general formula 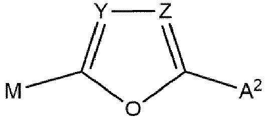 wherein Y, Z and A² are as defined in any of the preceding claims and wherein M is selected from one of the following functional groups:
-SnR*₃, -B(OR*)₂, -Zn-Hal*, -Mg-Hal*,
wherein R* is a C₁-C₁₀-alkyl and wherein the Hal* group is a halogen.

## Revendications

1. Composé de formule générale (I) : 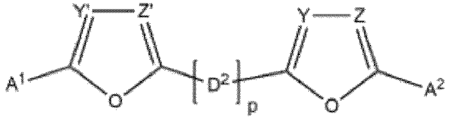
avec p choisi parmi : 1, 2, 3, 4 et 5,
avec Y, Y', Z et Z' choisis à chaque fois indépendamment les uns des autres parmi : N, CH, C-F, C-CH₃, C-CF₃, C-C₂H₅, C-C₃H₈, C-OCH₃, C-OC₂H₅, C-SCH₃, C-SC₂H₅ ; et
les blocs conjugués D² étant choisis à chaque fois indépendamment les uns des autres dans le groupe des motifs structuraux suivants : 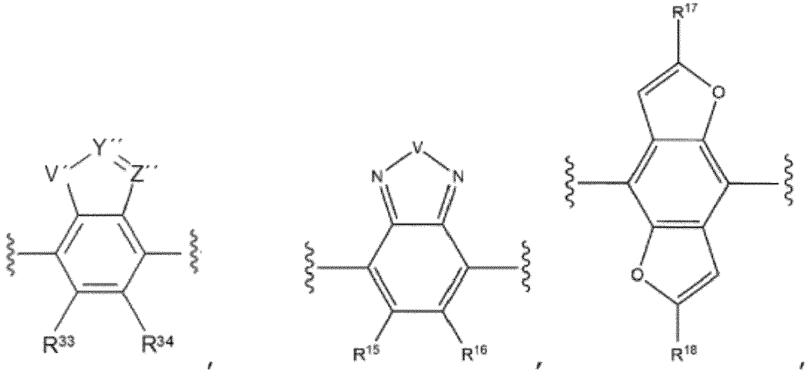 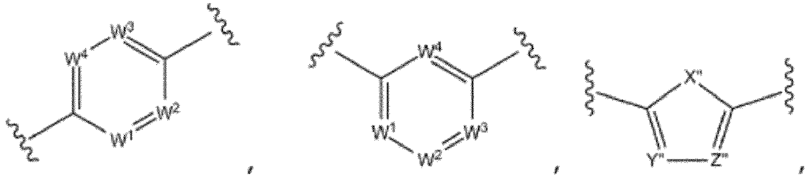 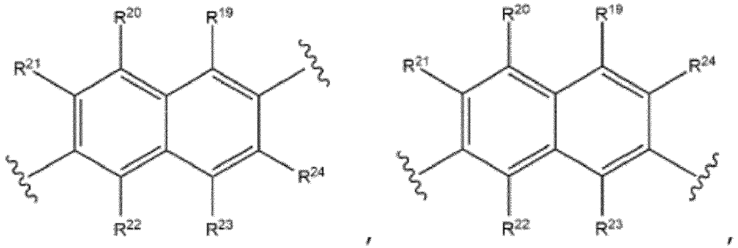 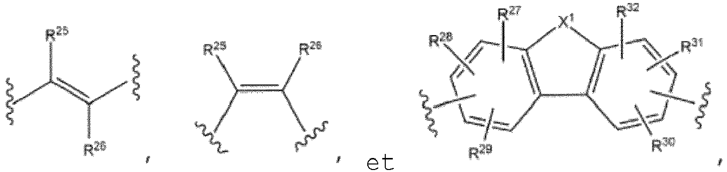
avec Y" choisi parmi : N et CR^{g} ;
avec Z" choisi parmi : N et CR^{h} ;
avec X" choisi parmi : O, S, Se, Si(RⁱR^{j}), P(Rⁱ), P(O)Rⁱ ;
avec V choisi parmi : O, S, Se et NR^{k} ;
avec V' choisi parmi : O, S, Se et NR^{k} ;
avec X¹ choisi parmi : NR^{l}, CR^{l}R^{m} ;
avec W¹ à W⁴ choisis indépendamment les uns des autres parmi : N, CRⁿ ;
les radicaux R^{g} à R^{h} et les radicaux R¹⁵ à R³⁴ étant choisis à chaque fois indépendamment les uns des autres dans le groupe des radicaux :
H, halogène, CN, NR^{d}R^{e}, R^{d} et R^{e} étant choisis à chaque fois indépendamment l'un de l'autre dans le groupe des radicaux : H, ainsi que C₁-C₂₀-alkyle cyclique ou à chaîne ouverte, des atomes de C individuels pouvant être remplacés par des hétéroatomes et des atomes d'hydrogène du radical alkyle pouvant être substitués, C₁-C₂₀-alkyle cyclique ou à chaîne ouverte, des atomes de C individuels pouvant être remplacés par des hétéroatomes, C₁-C₂₀-O-alkyle cyclique ou à chaîne ouverte, C₁-C₂₀-S-alkyle cyclique ou à chaîne ouverte, C₂-C₂₀-alcényle cyclique ou à chaîne ouverte, C₂-C₂₀-O-alcényle cyclique ou à chaîne ouverte, C₂-C₂₀-S-alcényle cyclique ou à chaîne ouverte, C-C₂₀-alcynyle cyclique ou à chaîne ouverte, aryle, hétéroaryle,
des atomes d'hydrogène des radicaux alkyle, O-alkyle, S-alkyle, alcényle, O-alcényle, S-alcényle, alcynyle, aryle et hétéroaryle pouvant être substitués à chaque fois indépendamment les uns des autres ; et les radicaux R^{g} et R^{h} pouvant être reliés l'un à l'autre sous forme d'une structure cyclique et un radical aryle pouvant être condensé à la structure cyclique, le radical aryle éventuellement condensé à la structure cyclique pouvant être substitué,
A¹ étant choisi dans le groupe des radicaux suivants : 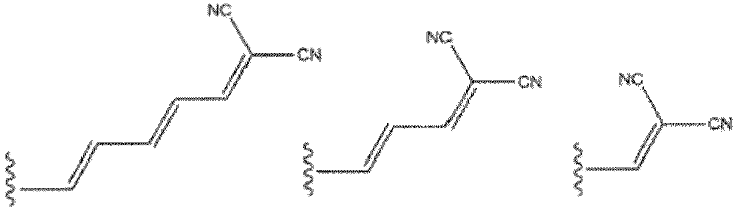 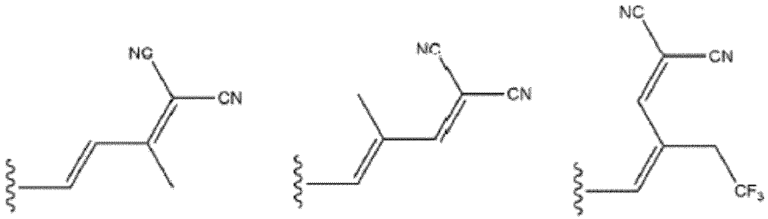 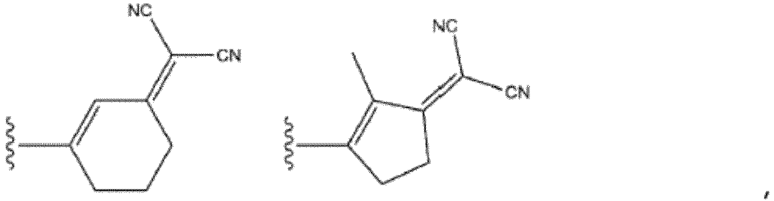
et
A² étant choisi dans le groupe des radicaux suivants :

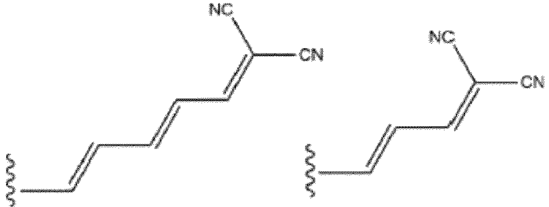

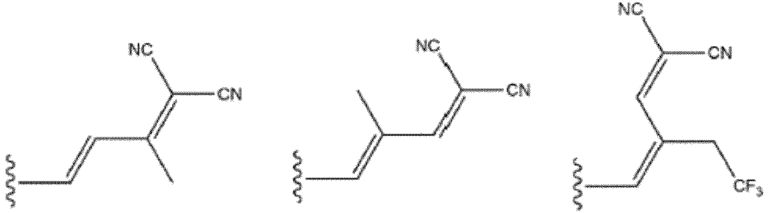

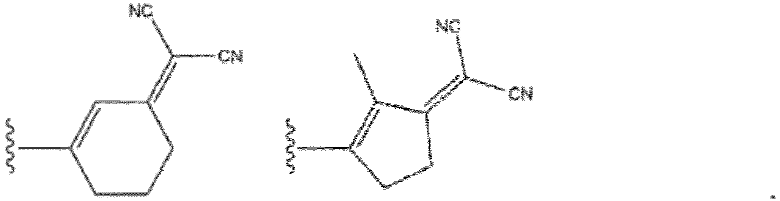

2. Composé selon la revendication 1, Y, Y', Z, Z' étant à chaque fois CH.

3. Composé selon l'une quelconque des revendications précédentes, au moins l'un des blocs conjugués D² présentant la formule générale

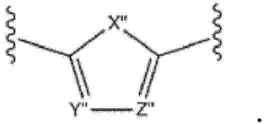

4. Composé selon l'une quelconque des revendications précédentes, au moins l'un des blocs conjugués D² présentant la formule générale 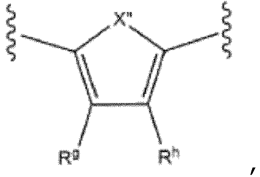
les radicaux R^{g} et R^{h} étant choisis indépendamment l'un de l'autre dans le groupe des radicaux suivants : H, F, C₁-C₅-alkyle, des atomes de C individuels pouvant être remplacés par des hétéroatomes, C₁-C₅-O-alkyle, C₁-C₅-S-alkyle, des atomes d'hydrogène des radicaux alkyle, O-alkyle et S-alkyle pouvant être substitués à chaque fois indépendamment les uns des autres par fluor,
les radicaux R^{g} et R^{h} pouvant être reliés l'un à l'autre sous forme d'une structure cyclique et un radical aryle pouvant être condensé à la structure cyclique.

5. Composé selon l'une quelconque des revendications précédentes, X" étant égal à S.

6. Composé selon la revendication 5 présentant la formule générale suivante :

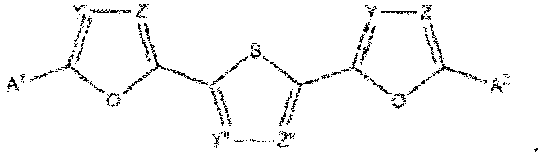

7. Composé selon l'une quelconque des revendications précédentes, au moins l'un des blocs conjugués D² présentant la formule générale 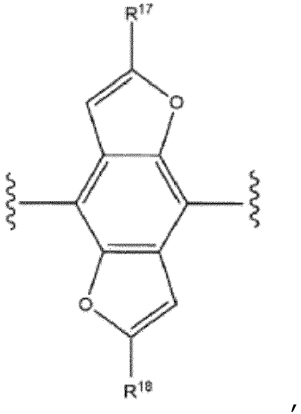 les radicaux R¹⁷ et R¹⁸ étant choisis indépendamment l'un de l'autre dans le groupe des radicaux suivants : H, F, C₁-C₅-alkyle, C₁-C₅-O-alkyle, C₁-C₅-S-alkyle, des atomes d'hydrogène des radicaux alkyle, O-alkyle et S-alkyle pouvant être remplacés à chaque fois indépendamment les uns des autres par des atomes de fluor.

8. Composé selon l'une quelconque des revendications précédentes, au moins l'un des blocs conjugués D² présentant la formule générale 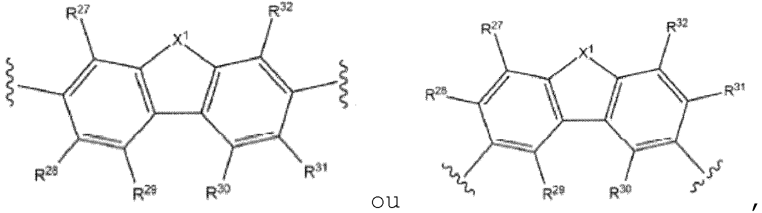
X¹ étant choisi parmi : NR^{l}, CR^{l}R^{m},
R^{l} et R^{m} étant choisis à chaque fois indépendamment l'un de l'autre dans le groupe des radicaux : H, C₁-C₅-alkyle à chaîne ouverte, C₅-C₁₂-aryle, C₅-C₁₂-hétéroaryle, des atomes d'hydrogène du C₁-C₅-alkyle à chaîne ouverte pouvant être remplacés au moins partiellement par des atomes de fluor et des atomes d'hydrogène du C₅-C₁₂-aryle et du C₅-C₁₂-hétéroaryle pouvant être au moins partiellement substitués, et
R²⁷ à R³² étant choisis indépendamment les uns des autres dans le groupe des radicaux suivants : H, F, C₁-C₅-alkyle, C₁-C₅-O-alkyle, C₁-C₅-S-alkyle, des atomes d'hydrogène des radicaux alkyle, O-alkyle et S-alkyle pouvant être remplacés à chaque fois indépendamment les uns des autres par des atomes de fluor.

9. Composé selon l'une quelconque des revendications précédentes, au moins l'un des blocs conjugués D² présentant la formule générale 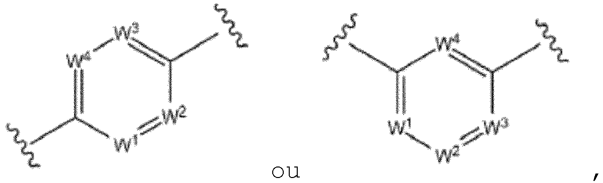
W¹ à W⁴ étant choisis indépendamment les uns des autres parmi : N, CRⁿ,
Rⁿ étant choisi dans le groupe des radicaux suivants : H, F, C₁-C₅-alkyle, C₁-C₅-O-alkyle, C₁-C₅-S-alkyle, des atomes d'hydrogène des radicaux alkyle, O-alkyle et S-alkyle pouvant être remplacés à chaque fois indépendamment les uns des autres par des atomes de fluor.

10. Composé selon l'une quelconque des revendications précédentes, au moins l'un des blocs conjugués D² présentant la formule générale 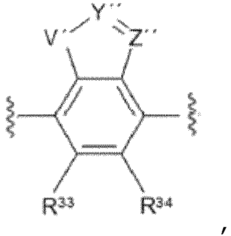
R³³ à R³⁴ étant choisis à chaque fois indépendamment l'un de l'autre dans le groupe des radicaux suivants : H, F, C₁-C₅-alkyle, C₁-C₅-O-alkyle, C₁-C₅-S-alkyle, des atomes d'hydrogène des radicaux alkyle, O-alkyle et S-alkyle pouvant être remplacés à chaque fois indépendamment les uns des autres par des atomes de fluor et
avec Y" choisi parmi : N et CR^{g} ;
avec Z" choisi parmi : N et CR^{h} ;
avec V' choisi parmi : O, S, Se et NR^{k} ;
R^{g} et R^{h} étant choisis parmi :
H, halogène, CN, NR^{d}R^{e}, R^{d} et R^{e} étant choisis à chaque fois indépendamment l'un de l'autre dans le groupe des radicaux : H, ainsi que C₁-C₂₀-alkyle cyclique ou à chaîne ouverte, des atomes de C individuels pouvant être remplacés par des hétéroatomes et des atomes d'hydrogène du radical alkyle pouvant être substitués, C₁-C₂₀-alkyle cyclique ou à chaîne ouverte, des atomes de C individuels pouvant être remplacés par des hétéroatomes, C₁-C₂₀-O-alkyle cyclique ou à chaîne ouverte, C₁-C₂₀-S-alkyle cyclique ou à chaîne ouverte, C₂-C₂₀-alcényle cyclique ou à chaîne ouverte, C₂-C₂₀-O-alcényle cyclique ou à chaîne ouverte, C₂-C₂₀-S-alcényle cyclique ou à chaîne ouverte, C-C₂₀-alcynyle cyclique ou à chaîne ouverte, aryle, hétéroaryle,
des atomes d'hydrogène des radicaux alkyle, O-alkyle, S-alkyle, alcényle, O-alcényle, S-alcényle, alcynyle, aryle et hétéroaryle pouvant être substitués à chaque fois indépendamment les uns des autres,
R^{k} étant choisi dans le groupe des radicaux : H, C₁-C₅-alkyle à chaîne ouverte, C₅-C₁₂-aryle, C₅-C₁₂-hétéroaryle, des atomes d'hydrogène du C₁-C₅-alkyle à chaîne ouverte pouvant être remplacés au moins partiellement par des atomes de fluor et des atomes d'hydrogène du C₅-C₁₂-aryle et du C₅-C₁₂-hétéroaryle pouvant être au moins partiellement substitués.

11. Composé selon l'une quelconque des revendications précédentes, les blocs conjugués D² étant choisis indépendamment les uns des autres dans le groupe des motifs structuraux suivants :

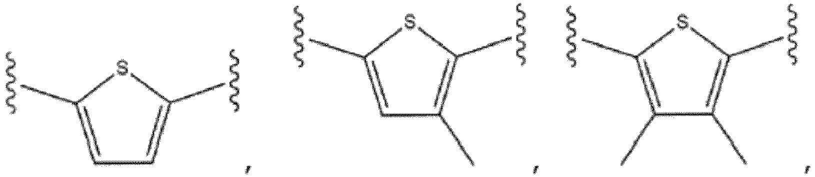

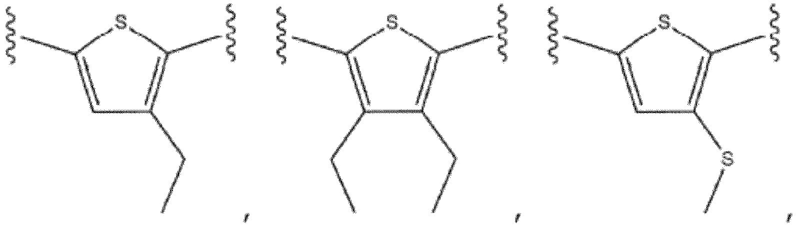

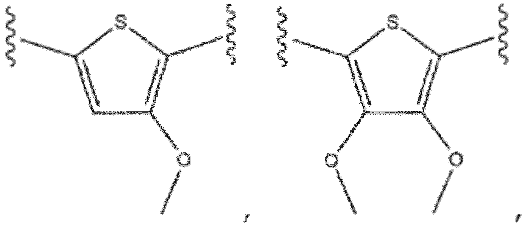

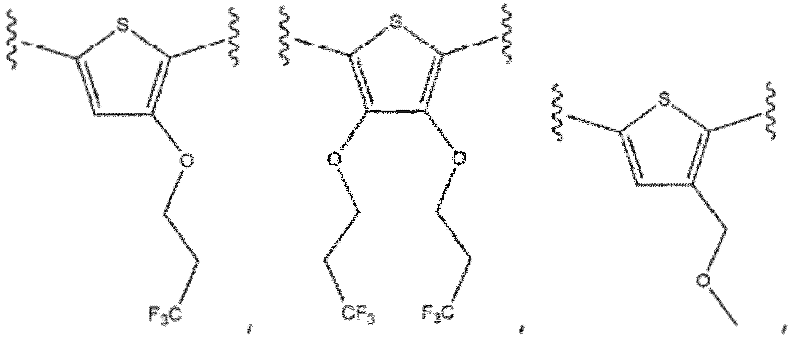

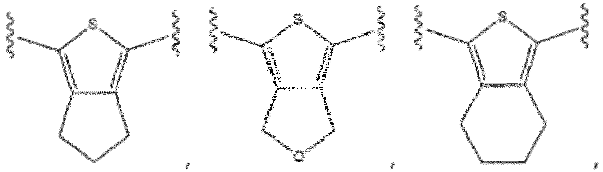

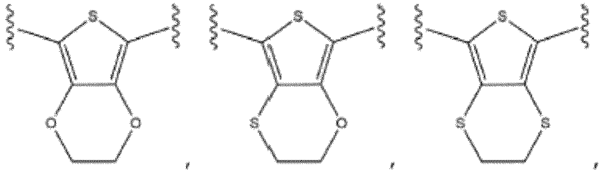

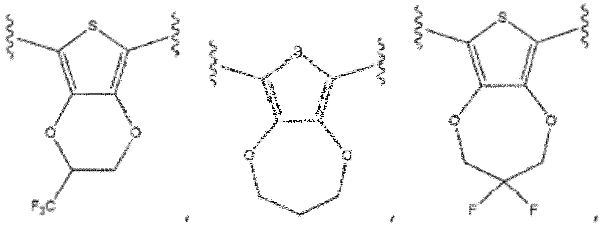

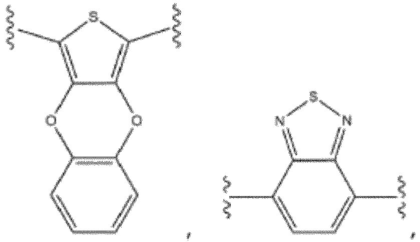

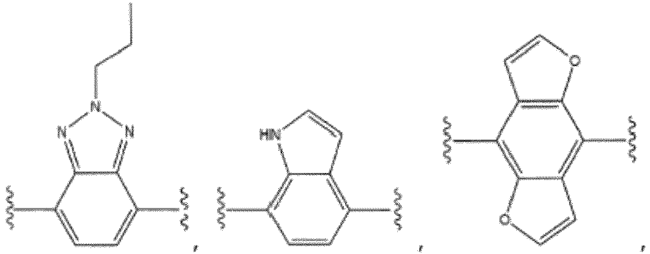

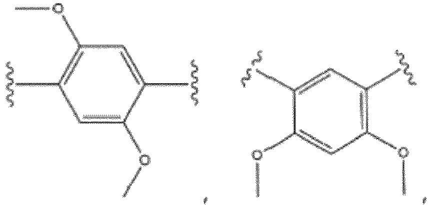

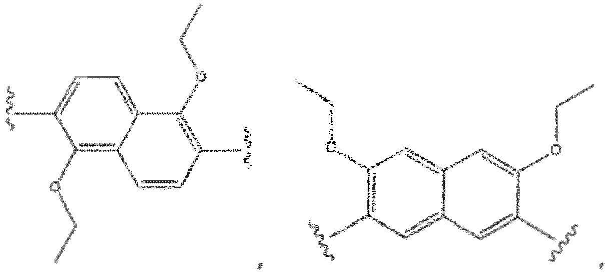

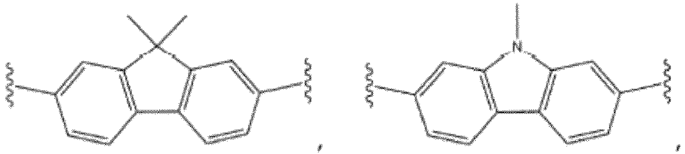

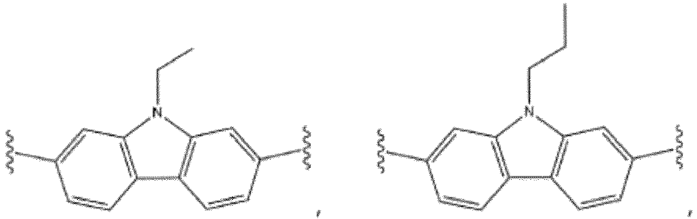

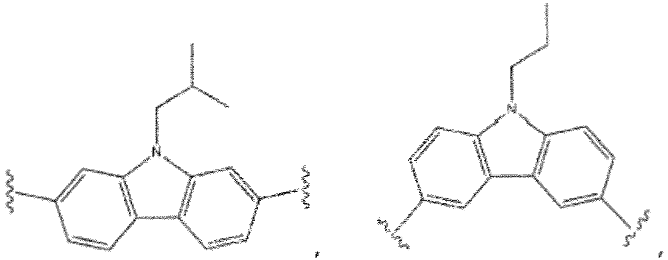

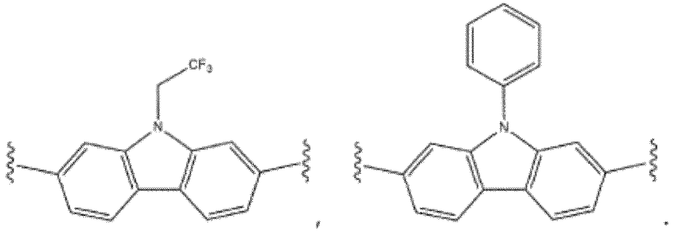

12. Utilisation d'un ou plusieurs composés selon l'une quelconque des revendications 1 à 11 dans un composant électronique organique photoactif, préférablement dans une cellule solaire organique.

13. Composant électronique organique photoactif, comprenant un composé selon l'une quelconque des revendications 1 à 11.

14. Composé pour la préparation d'un composé selon l'une quelconque des revendications 1 à 11, présentant la formule générale suivante 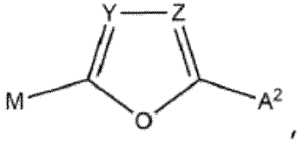 Y, Z et A² étant définis comme dans l'une quelconque des revendications précédentes et M étant choisi parmi l'un des groupes fonctionnels suivants :
-SnR*₃, -B(OR*)₂, -Zn-Hal*, -Mg-Hal*,
R* étant un C₁-C₁₀-alkyle et le groupe Hal* étant un halogène.
